# EUROPEAN PATENT SPECIFICATION

(11) **EP 1 802 606 B1**
(45) Date of publication and mention of the grant of the patent: **16.03.2011**
(21) Application number: 05814029.4
(22) Date of filing: 18.10.2005
(51) Int. Cl.: C07D 401/12

(54) **ARYLCARBOXAMIDES AND THEIR USE AS ANTI-TUMOR AGENTS**
ARYLCARBOXAMIDE UND IHRE VERWENDUNG ALS ANTITUMORMITTEL
ARYLCARBOXAMIDES ET LEUR UTILISATION COMME AGENTS ANTITUMORAUX

(30) Priority: 19.10.2004 US 620042 P
(43) Date of publication of application: 04.07.2007
(73) Proprietor: Compass Pharmaceuticals LLC, New York, NY 10003-3941 (US)
(72) Inventor: KANNE, David, Corte Madera, CA 94825 (US); WARD, John, Redwood City, California 94065 (US); LOOK, Gary, Santa Clara, CA 95051 (US); WANG, X., Michael, Livermore, CA 94550 (US); CHAN, Diva, San Francisco, CA 94122 (US); LEE, Tina, St. Louis, MO 63108 (US); JAMES, Donald, El Sobrante, CA 94803 (US); GLESS, Richard, Oakland, CA 94602 (US); SCHULLEK, John, San Jose, CA 95138 (US); VACIN, Charles, Oakland, CA 94619 (US)
(74) Representative: Zwicker, Jörk
(86) International application number: PCT/US2005/037618
(87) International publication number: WO 2006/044975

(56) References cited:
- EP-A- 0 574 808
- WO-A-2004/054977
- WO-A-2004/058762
- US-B1- 6 228 985
- LOOK, GARY C. ET AL: "The discovery of biaryl acids and amides exhibiting antibacterial activity against Gram-positive bacteria" BIOORGANIC & MEDICINAL CHEMISTRY LETTERS , 14(6), 1423-1426 CODEN: BMCLE8; ISSN: 0960-894X, 2004, XP002377847

## Description

### Cross Reference

This application claims priority to U.S. provisional patent application serial number 60/620,042 filed October 19, 2004.

### Background of the Invention

Approximately twenty percent of deaths from all causes in the United States are cancer-related. Although chemotherapy is a principal means of cancer treatment, the rate at which effective new drugs have become available for use in cancer chemotherapy has not increased (Horowitz et al., Journal of Clinical Oncology, Vol. 6, No. 2, pp. 308-314 (1988)). Despite many years of promising new therapies, cancer remains a major cause of morbidity and mortality (Bailar et al., N. Engl. J. Med. 336:1569-1574, 1997). Accordingly, there is a substantial need for new drugs that are effective in inhibiting the growth of tumors.

WO 2004 / 058 762 discloses a large library of over 1000 cyclic and heterocyclic compounds which are inhibitors of mitogen-activated protein-kinase activated protein kinase-2 (MK-2).

WO 2004 / 058 762 discloses a large library of over 1000 cyclic and heterocyclic compounds which are inhibitors of mitogen-activated protein-kinase activated protein kinase-2 (MK-2).

### Summary of the Invention

The present invention provides novel compounds and pharmaceutical compositions thereof, as well as methods for using the compounds and pharmaceutical compositions for treating tumors. Examples of specific tumor types that the compounds may be used to treat include, but are not limited to sarcomas, melanomas, neuroblastomas, carcinomas (including but not limited to lung, renal cell, ovarian, liver, bladder, and pancreatic carcinomas), and mesotheliomas. In one aspect, the present invention provides compounds according to the general formula I: 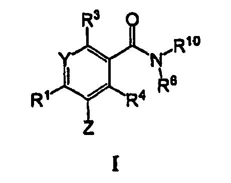 wherein
- Y: is C-R² or N;
- R¹: is selected from hydrogen, C₁-C₁₂ alkyl or halo;
- R², R³ and R⁴: are independently selected from hydrogen or halo;
- R⁶: is hydrogen, or
- R⁶: is C₁-C₁₂ alkyl optionally substituted with one or two groups selected from amino, monoalkylamino or dialkylamino;
- R¹⁰: is selected from aryl, heteroaryl, heteroarylalkyl, cycloalkyl, heterocycloalkyl or heterocycloalkylalkyl, each of which is optionally substituted at the ring portion and/or alkyl portion with one to five groups selected from C₁-C₁₂ alkyl, C₂-C₁₂ alkenyl, C₂-C₁₂ alkynyl, trifluoromethyl, C₁-C₁₂ alkoxy, hydroxyl, halo, amino, monoalkylamino, dialkylamino, aminoalkyl, monoalkylaminoalkyl, dialkylaminoalkyl, nitro, -CN, oxo, -C(O)OR, -NH-C(O)- R', -C(O)-NHR', -SR, -SO₂R, -CO₂R, or aryl, heteroaryl, cycloalkyl or heterocycloalkyl, each of which is optionally substituted with one to three groups selected from C₁-C₁₂ alkyl, C₂-C₁₂ alkenyl, C₂-C₁₂ alkynyl, trifluoromethyl, C₁-C₁₂ alkoxy, hydroxyl, halo, amino, monoalkylamino, dialkylamino, aminoalkyl, monoalkylaminoalkyl, dialkylaminoalkyl, nitro, -CN, oxo, -C(O)OR, -NH-C(O)-R', -C(O)-NHR', -SR, -SO₂R, -CO₂R, or
- R⁶ and R¹⁰: together with the nitrogen atom to which they are attached form a 5-7 membered heteroaryl ring optionally substituted with one to three groups independently selected from C₁-C₁₂ alkyl, C₂-C₁₂ alkenyl, C₂-C₁₂ alkynyl, trifluoromethyl, C₁-C₁₂ alkoxy, hydroxyl, halo, amino, monoalkylamino, dialkylamino, aminoalkyl, monoalkylaminoalkyl, dialkylaminoalkyl, nitro, -CN, oxo, -C(O)OR, -NH-C(O)-R', -C(O)-NHR', -SR, -SO₂R, -CO₂R;
- Z: is selected from 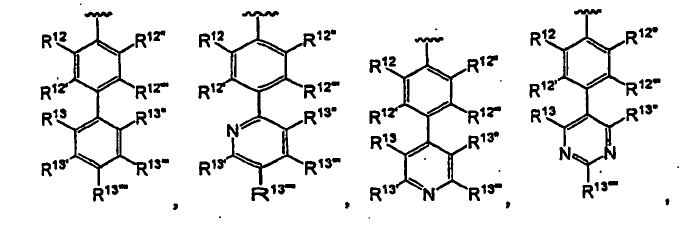 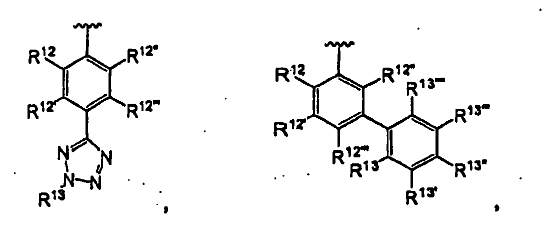 , or 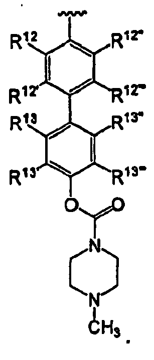
- R¹², R^{12'}, R^{12"} and R^{12'''}: are independently selected from hydrogen, C₁-C₁₂ alkyl, C₂-C₁₂ lkenyl, C₂-C₁₂ alkynyl, trifluoromethyl, C₁-C₁₂ alkoxy, hydroxyl, halo, amino, monoalkylamino, dialkylamino, aminoalkyl, monoalkylaminoalkyl, dialkylaminoalkyl, nitro, -CN, -C(O)OR, -NH-C(O)-R', -C(O)-NHR', -SR, -SO₂R, -CO₂R;
- R¹³, R^{13'}, R^{13"}, R^{13'"} and R^{13""}: are independently selected from hydrogen, C₁-C₁₂ alkyl, C₂-C₁₂ alkenyl, C₂-C₁₂ alkynyl, trifluoromethyl, C₁-C₁₂ alkoxy, hydroxyl, halo, amino, monoalkylamino, dialkylamino, aminoalkyl, monoalkylaminoalkyl, dialkylaminoalkyl, nitro, -CN, -C(O)OR, -NH-C(O)-R', -C(O)-NHR', -SR, -SO₂R, -CO₂R;
- X: is halo;
- R: is selected from hydrogen and C₁-C₁₂ alkyl; and
- R': is independently selected from hydrogen, C₁-C₁₂ alkyl, amino, monoalkylamino, dialkylamino, arylalkyl, heteroaryl, heteroarylalkyl, cycloalkyl, cycloalkylalkyl, heterocycloalkyl or heterocycloalkylalkyl.

In another aspect the present invention provides pharmaceutical compositions, comprising one or more compounds according to the invention, and a pharmaceutically acceptable carrier.

In another aspect, the present invention provides methods for treating a subject with a tumor, comprising administering to the subject an amount effective of a compound as specified in claim 41 or 43.

### Brief Description of the Figures

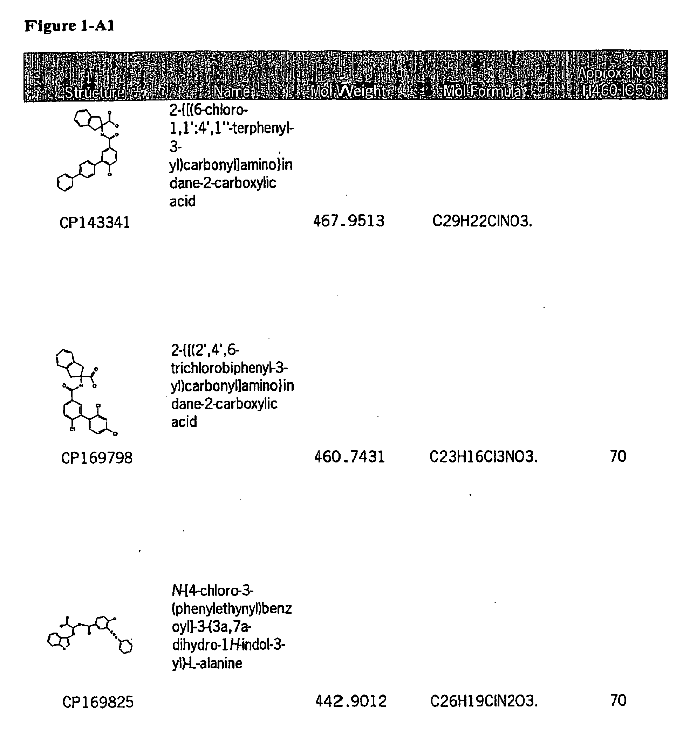

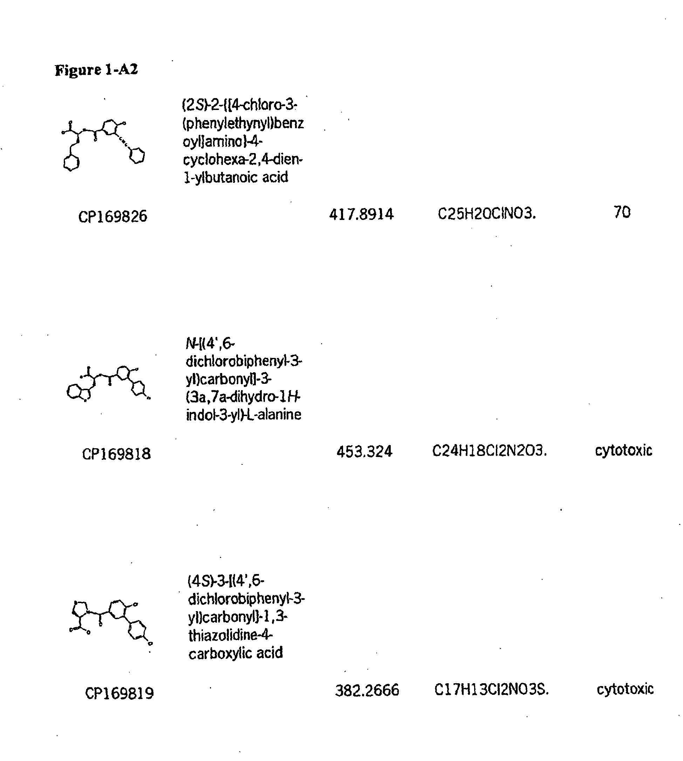

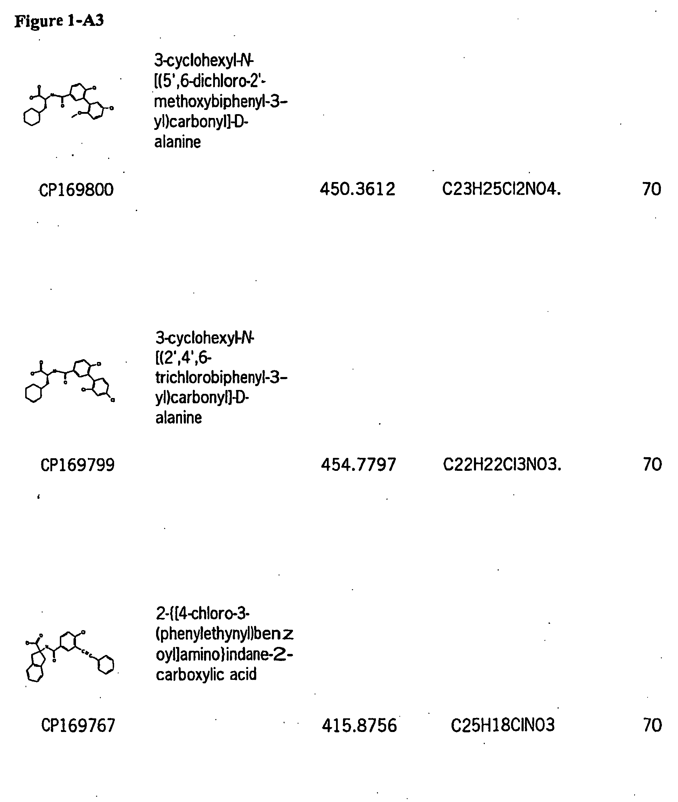

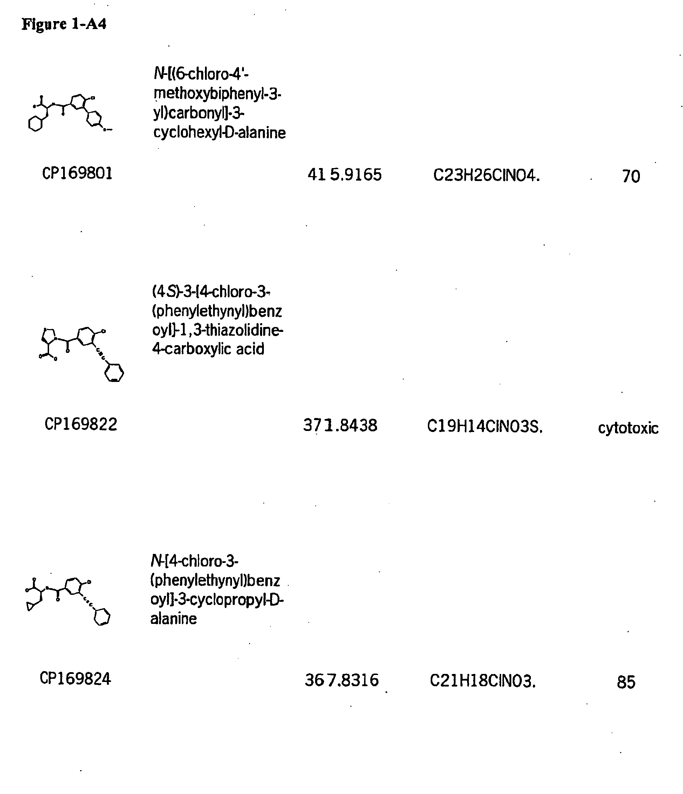

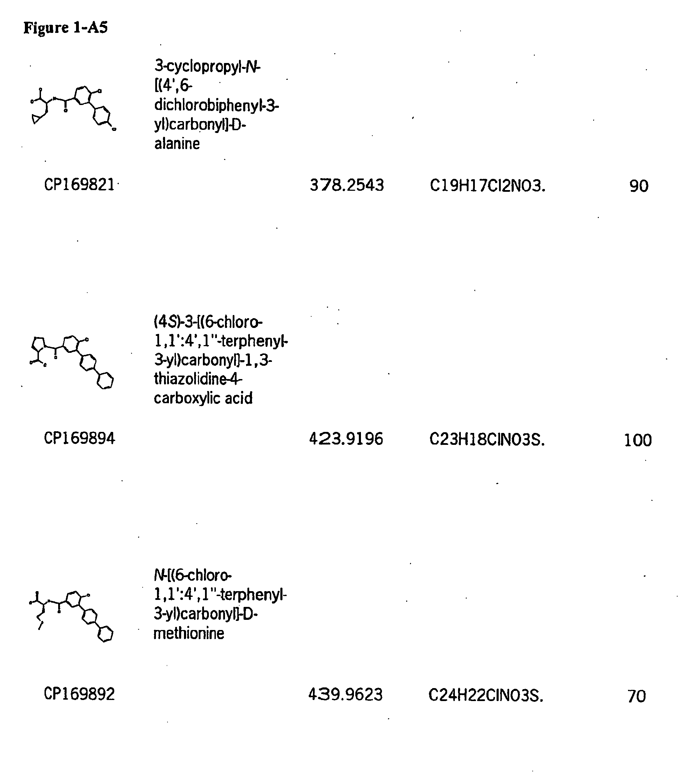

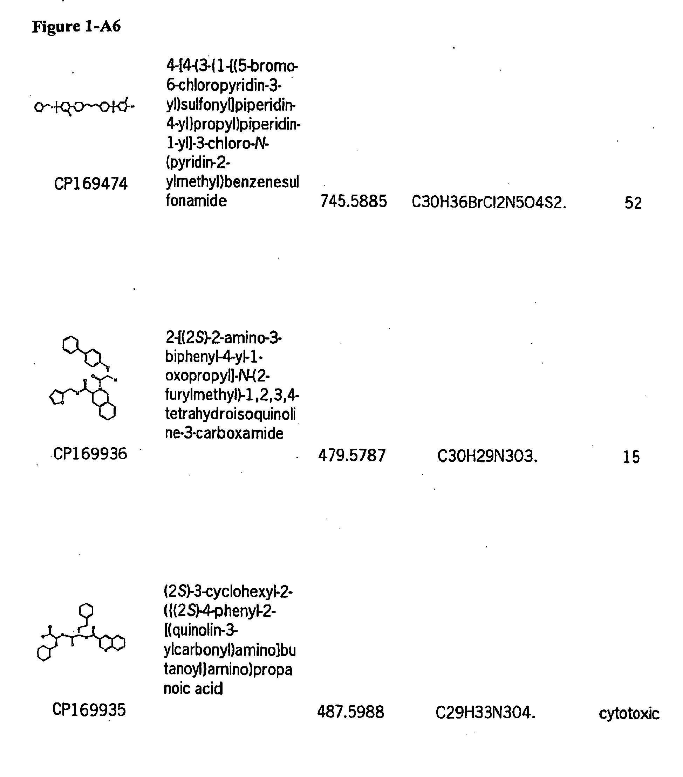

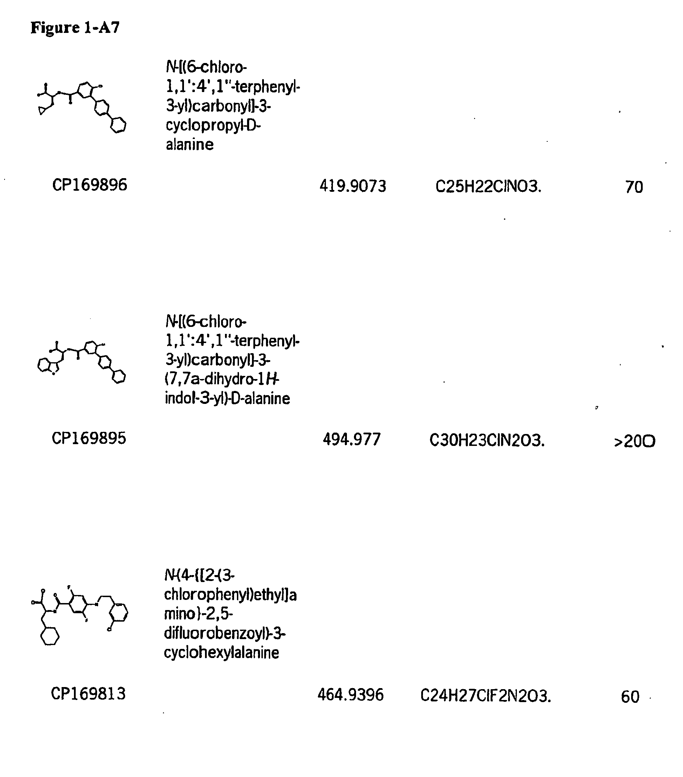

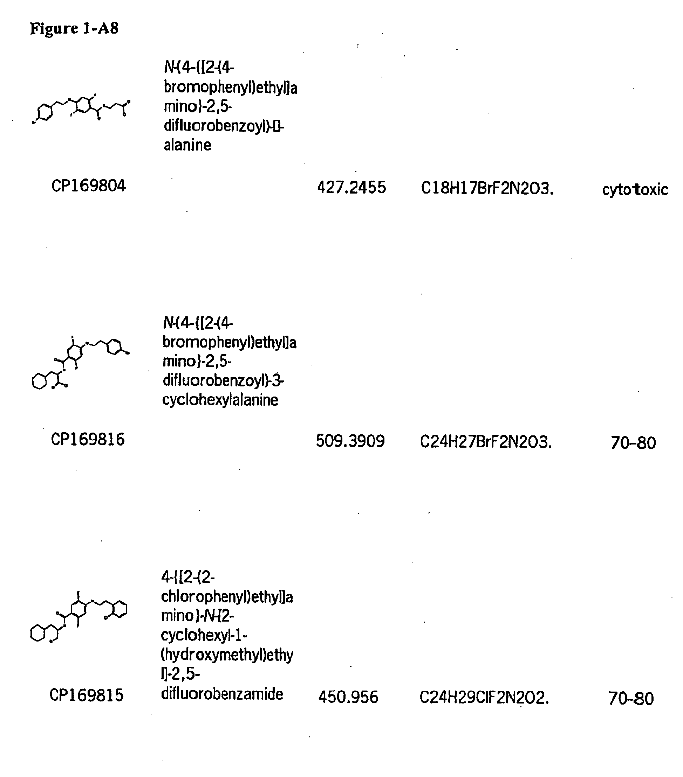

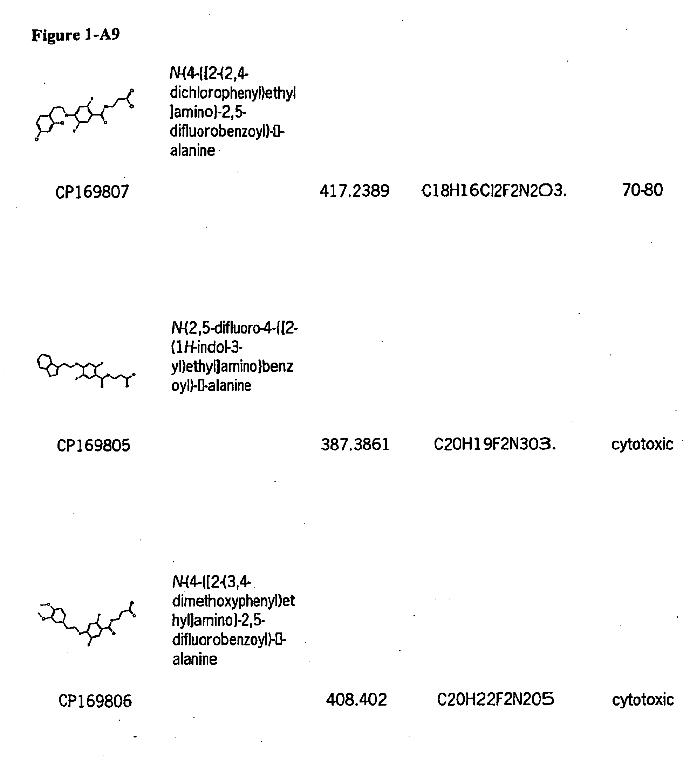

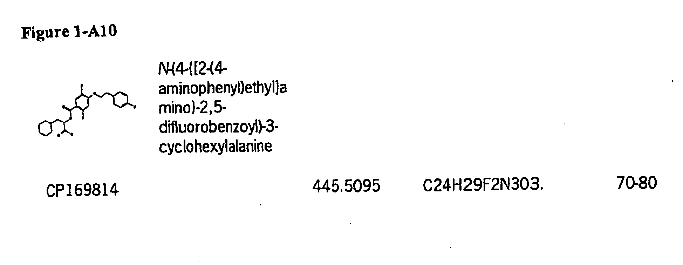

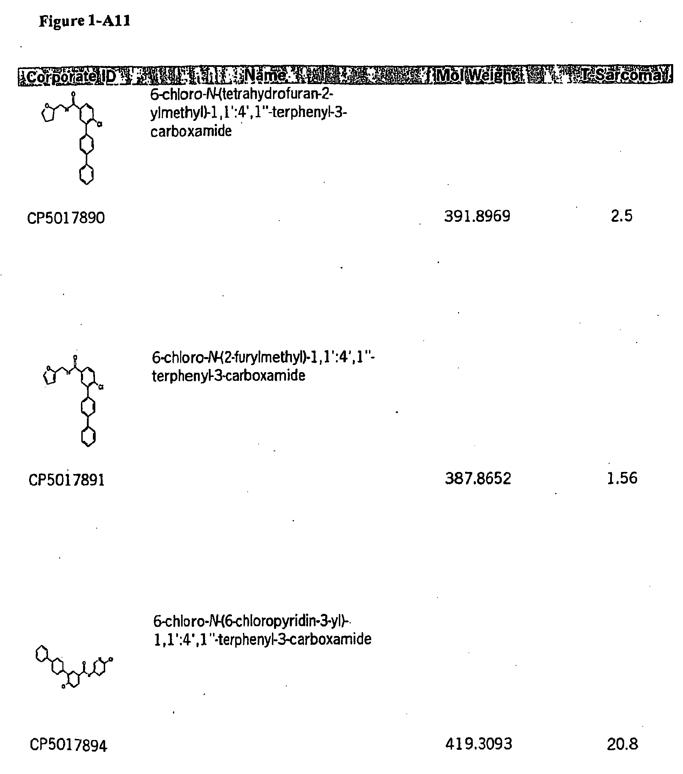

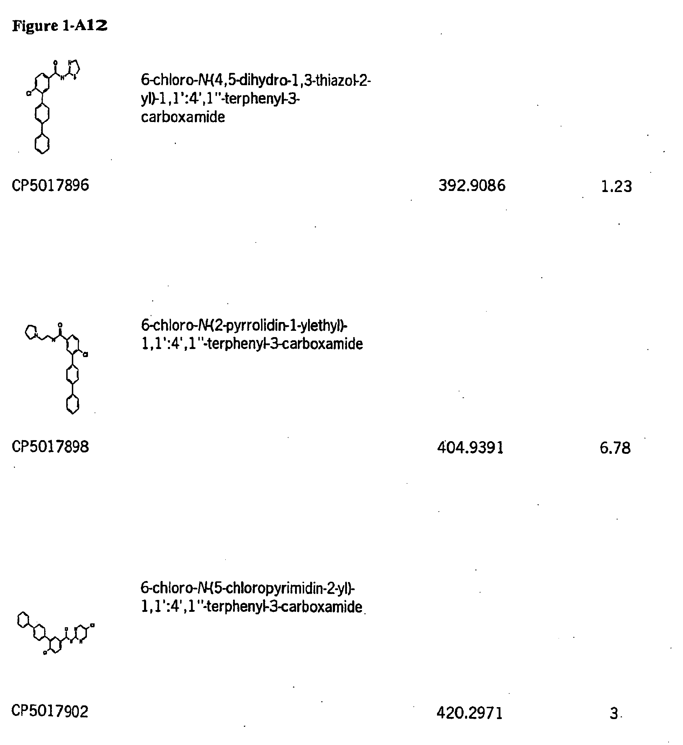

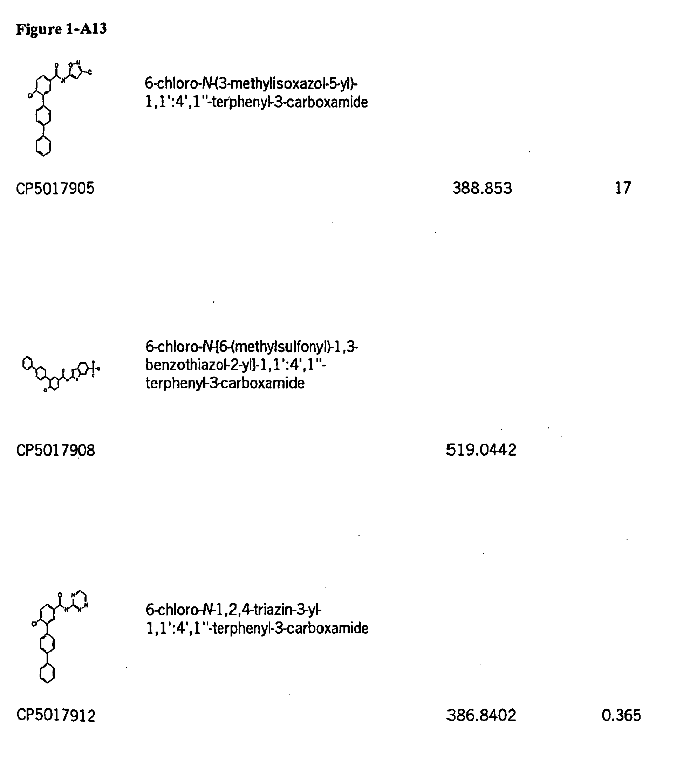

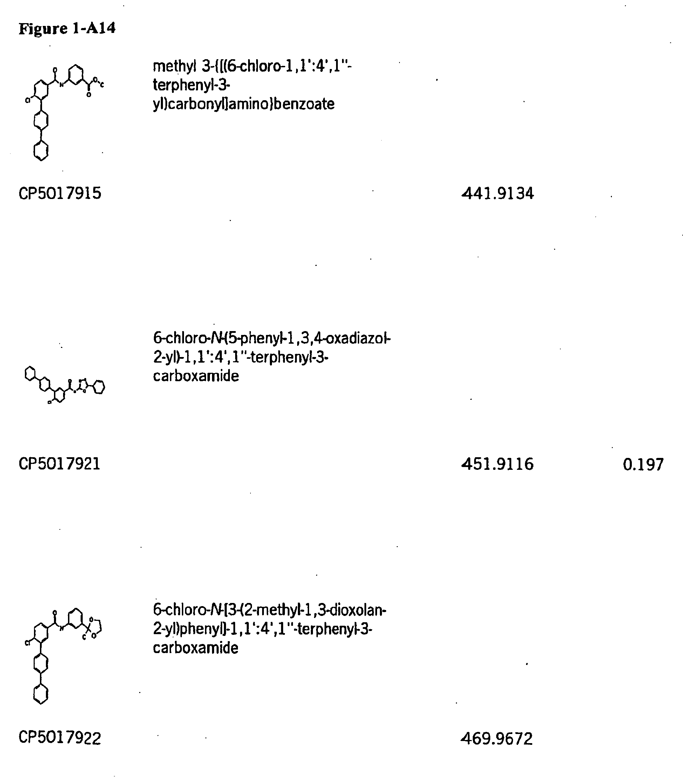

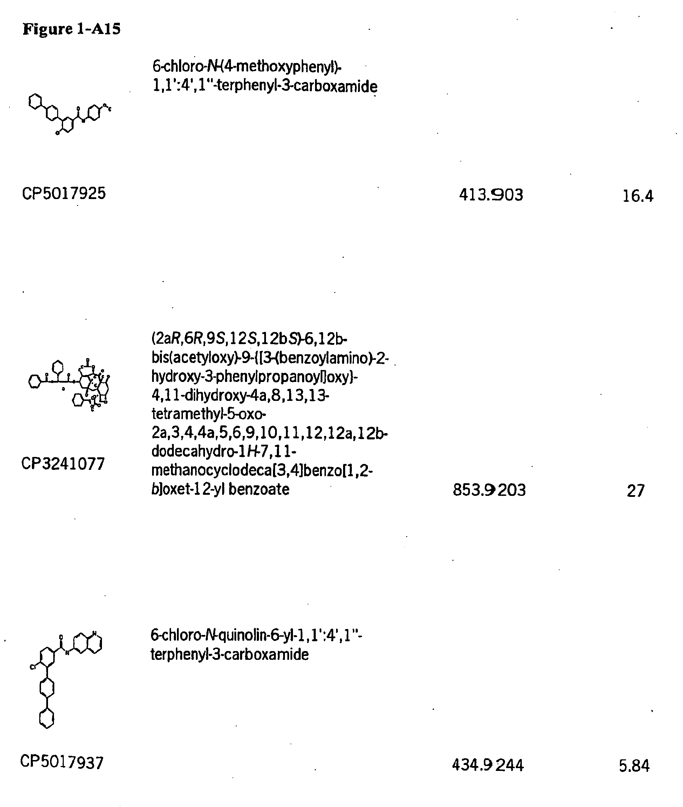

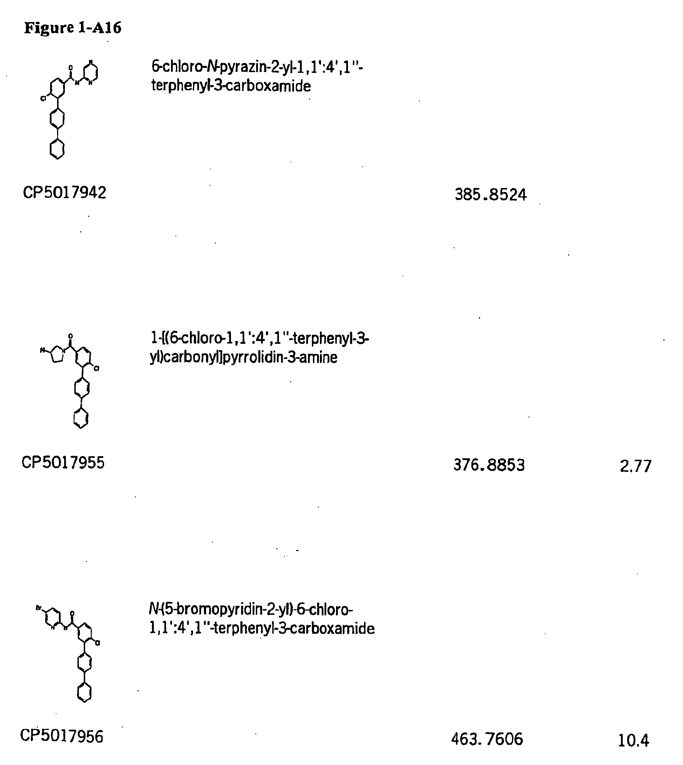

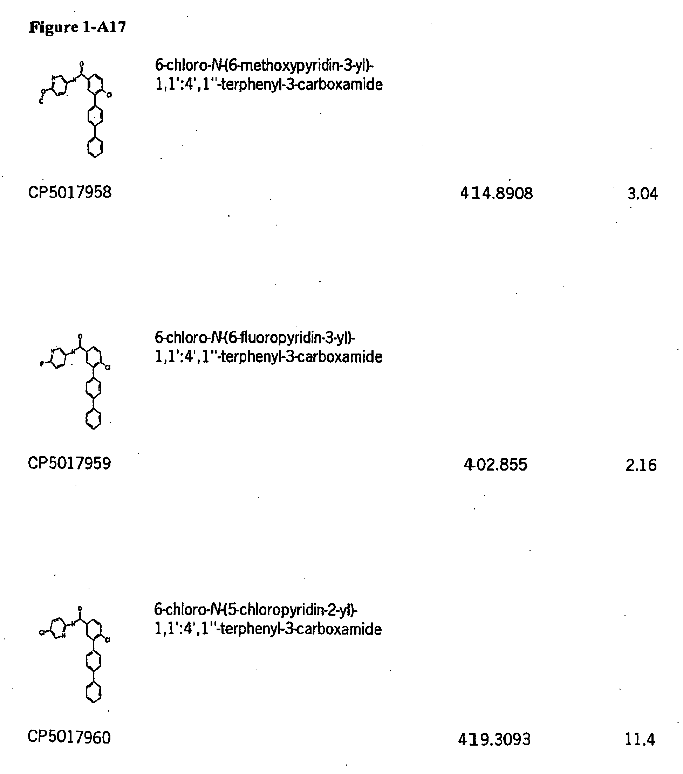

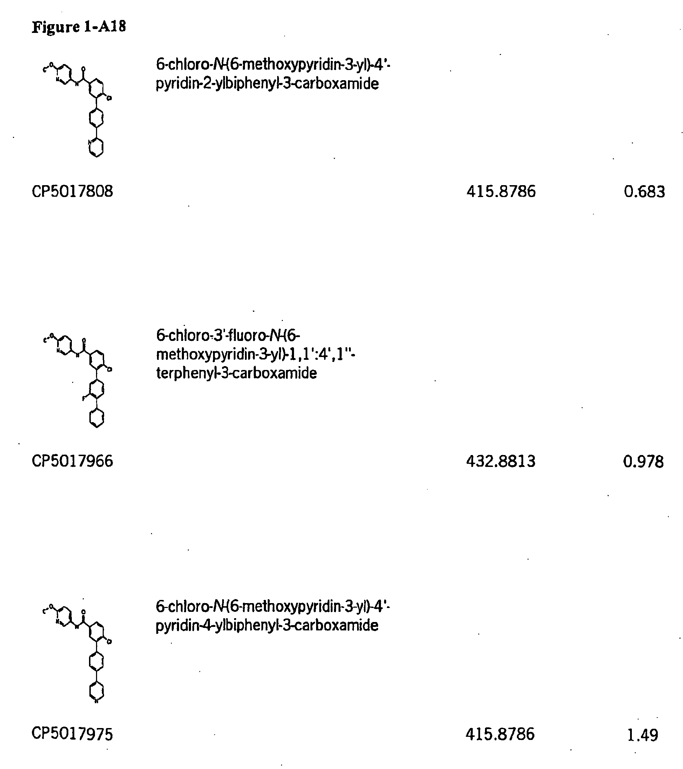

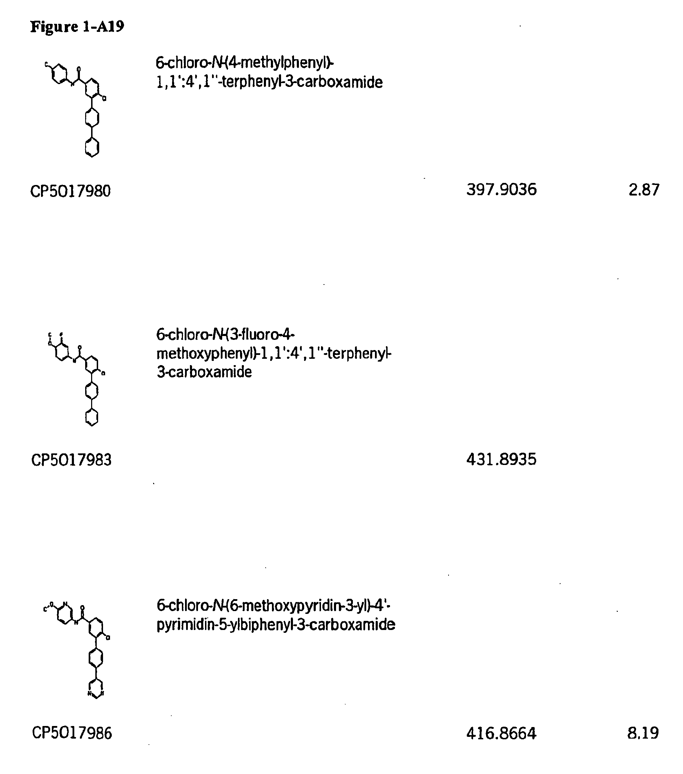

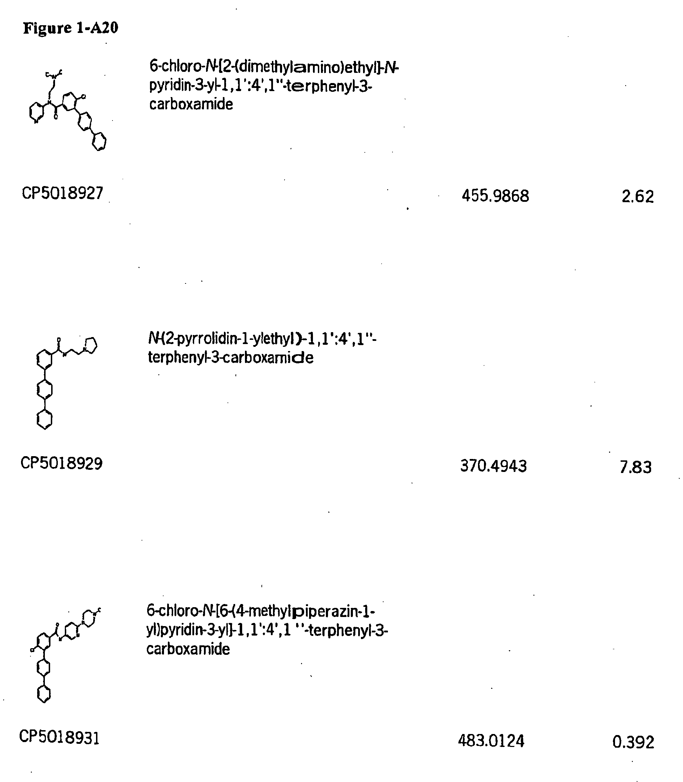

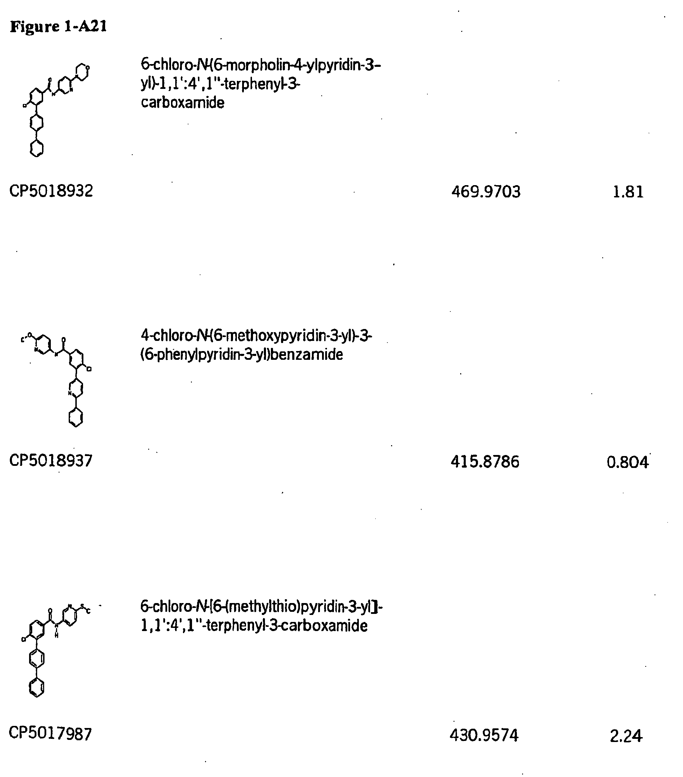

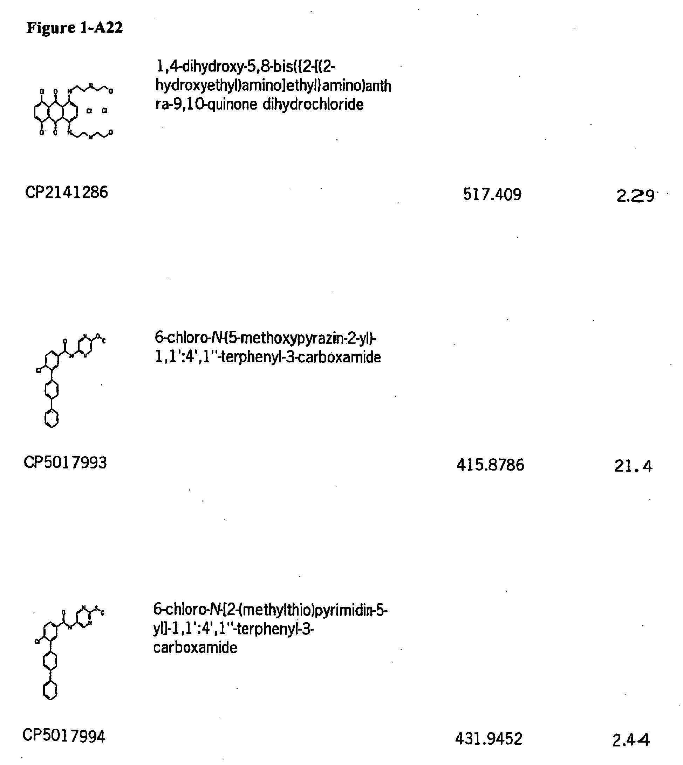

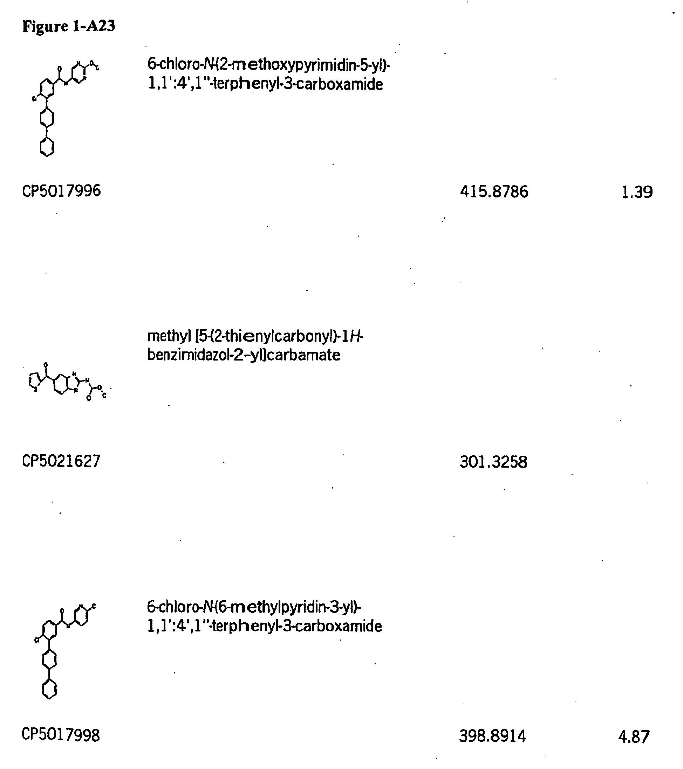

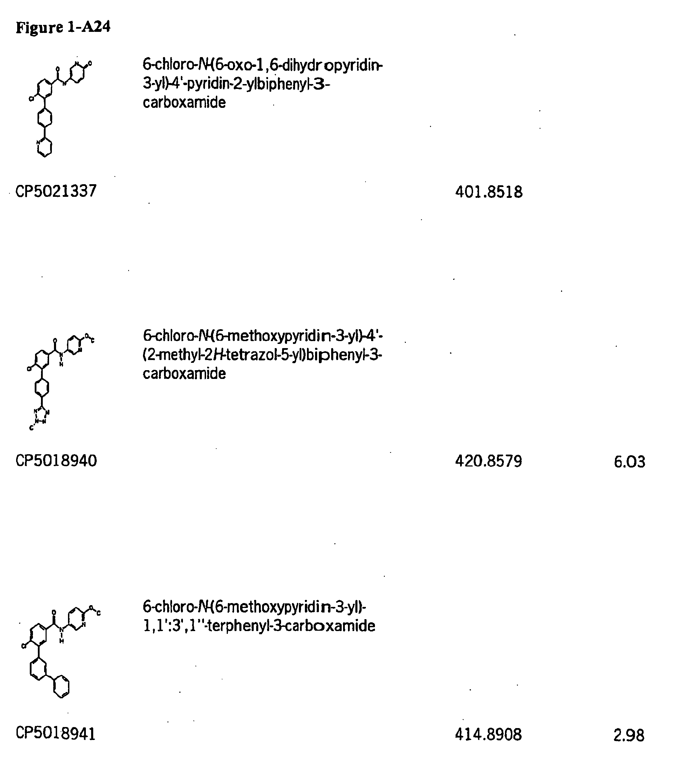

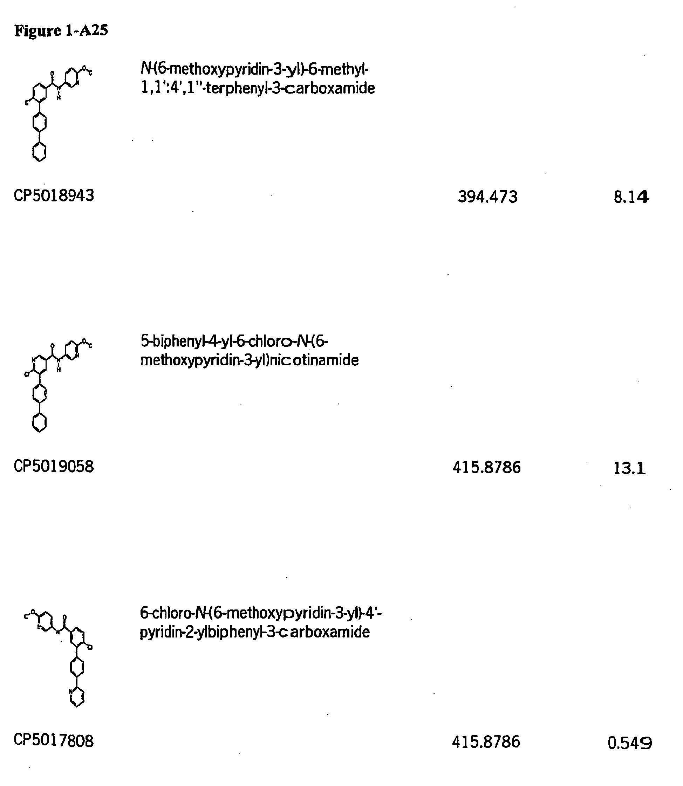

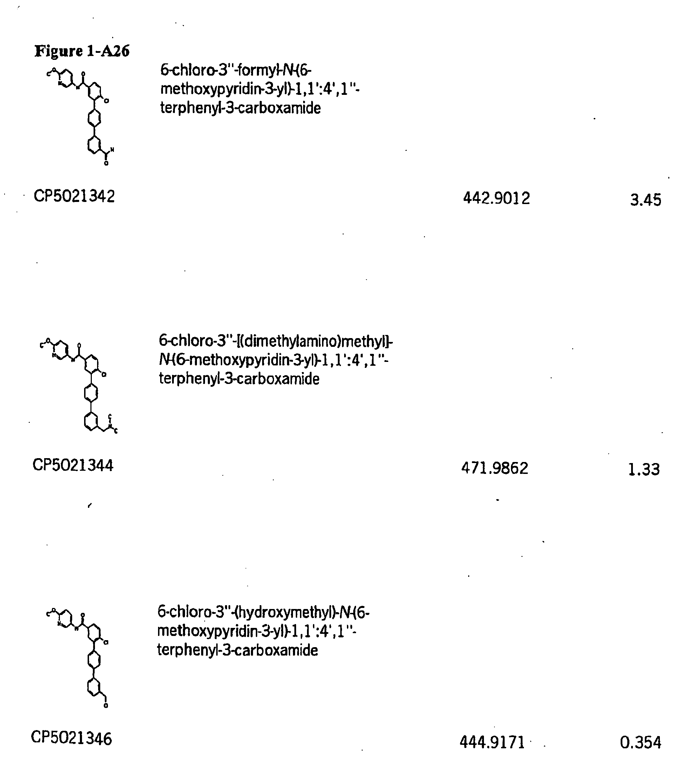

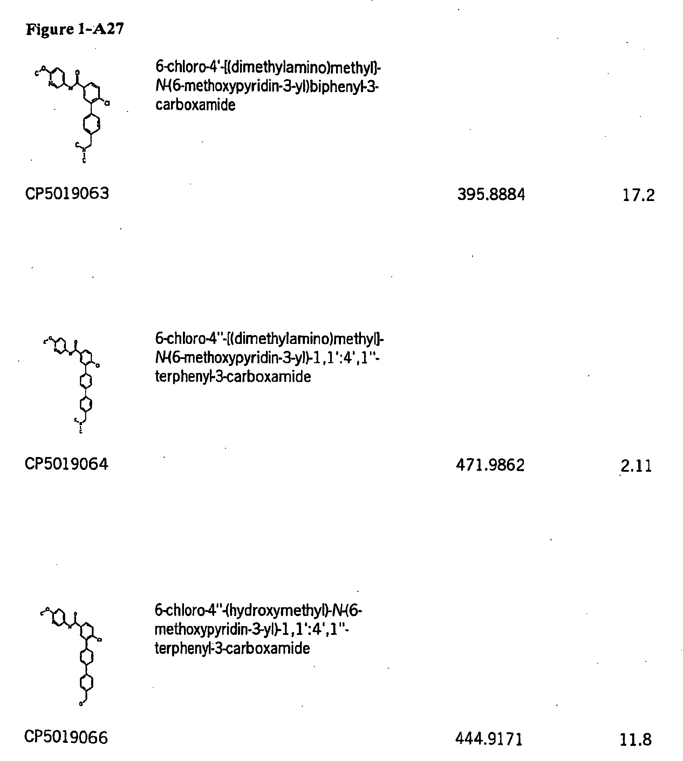

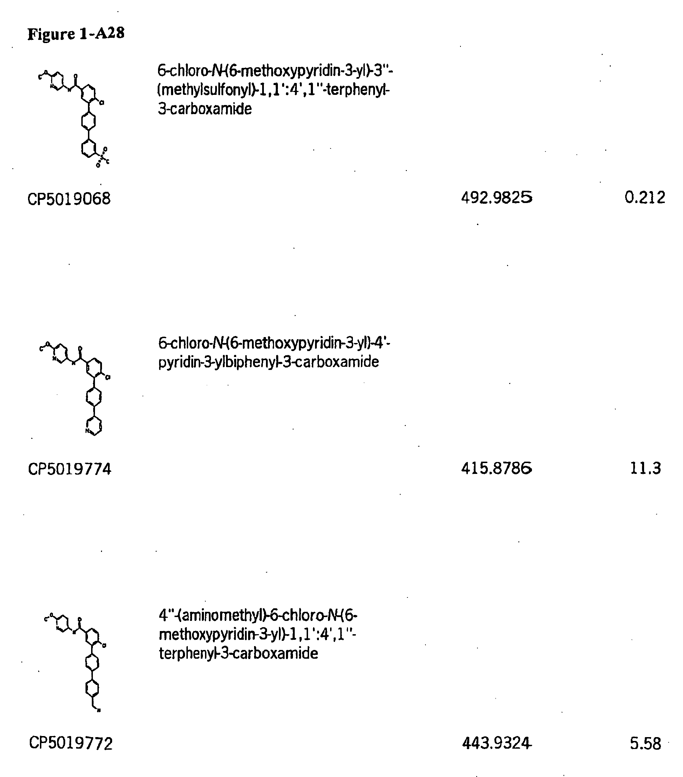

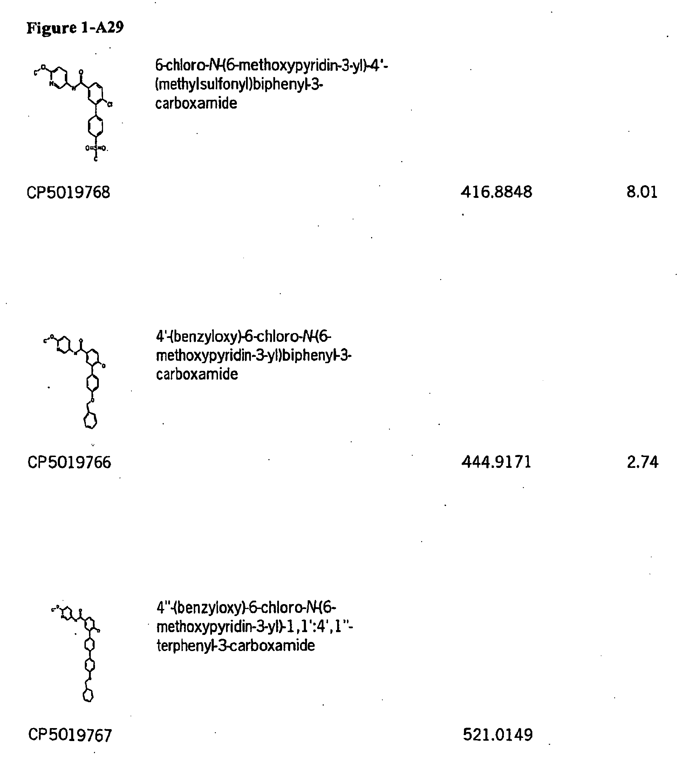

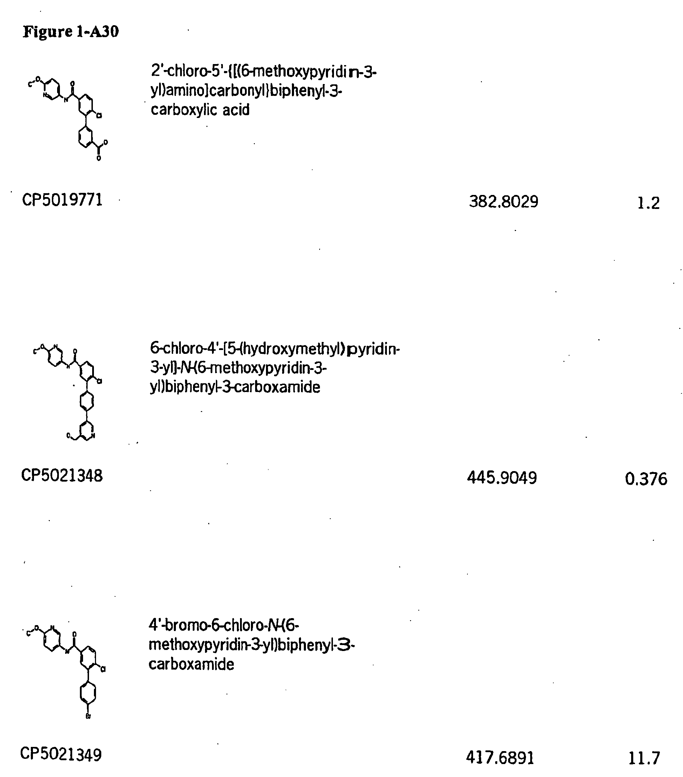

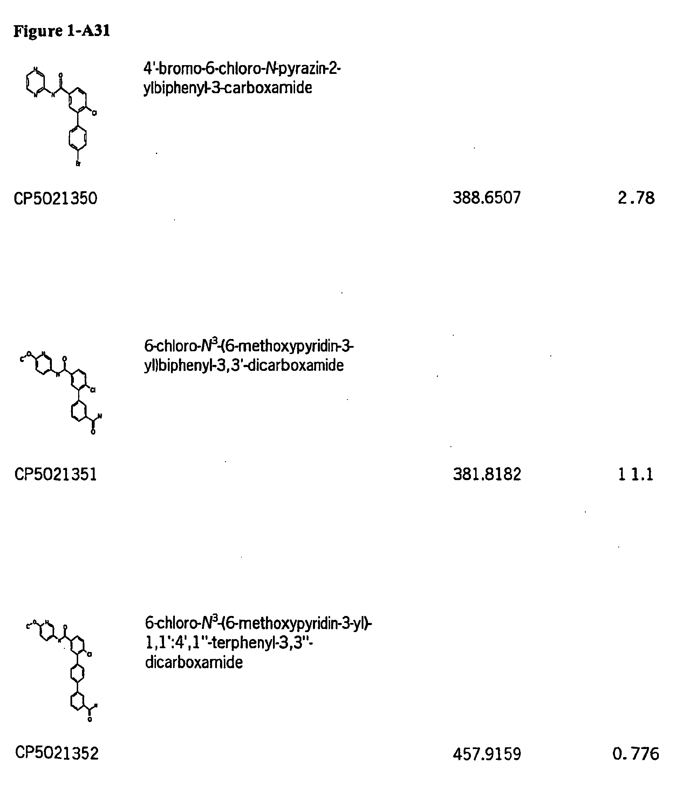

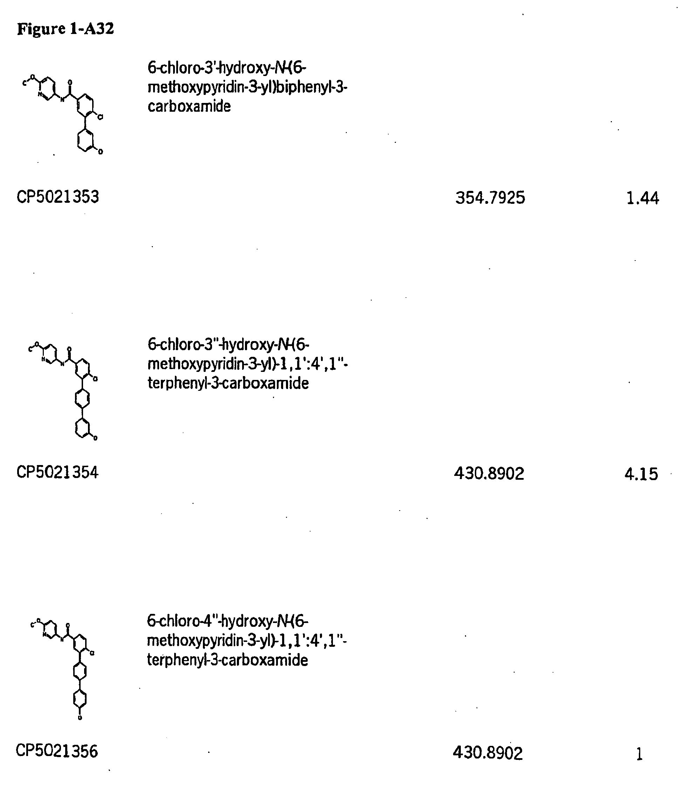

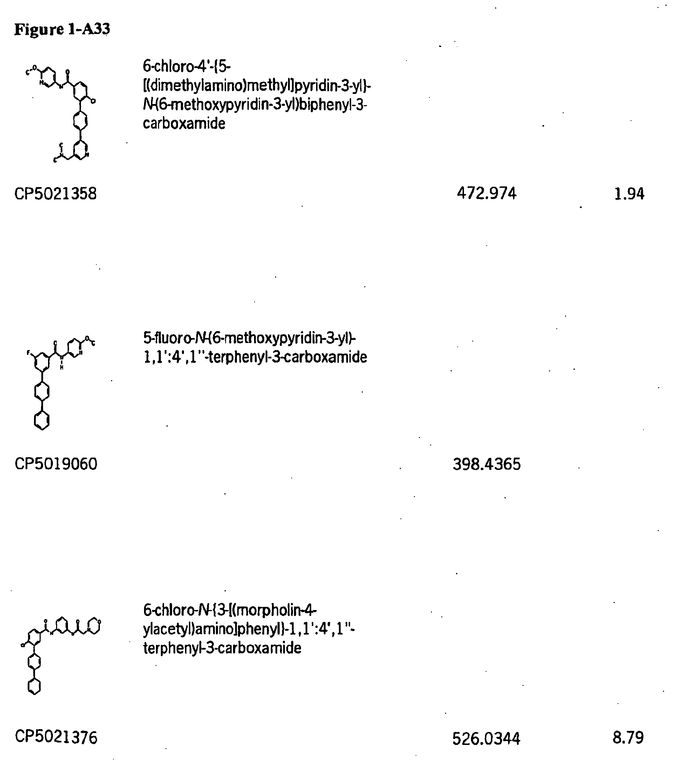

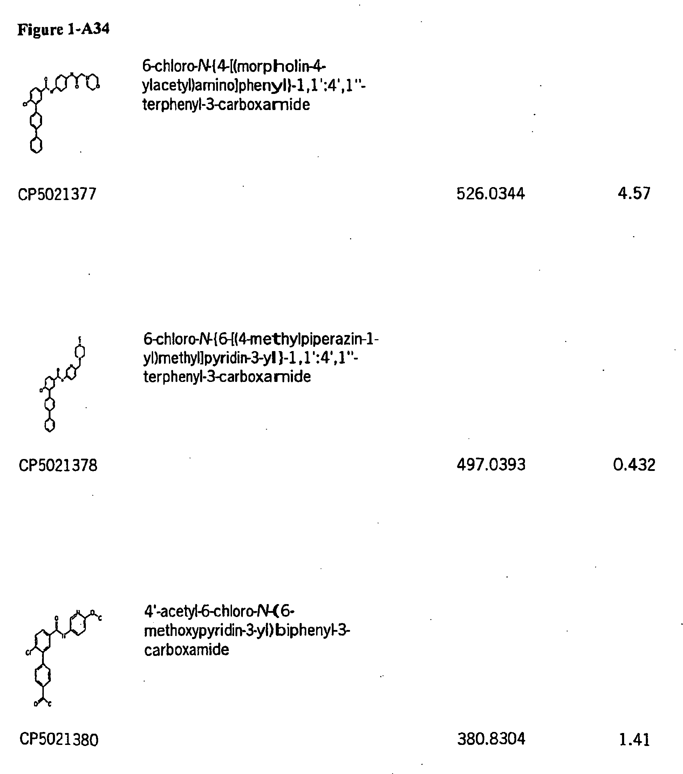

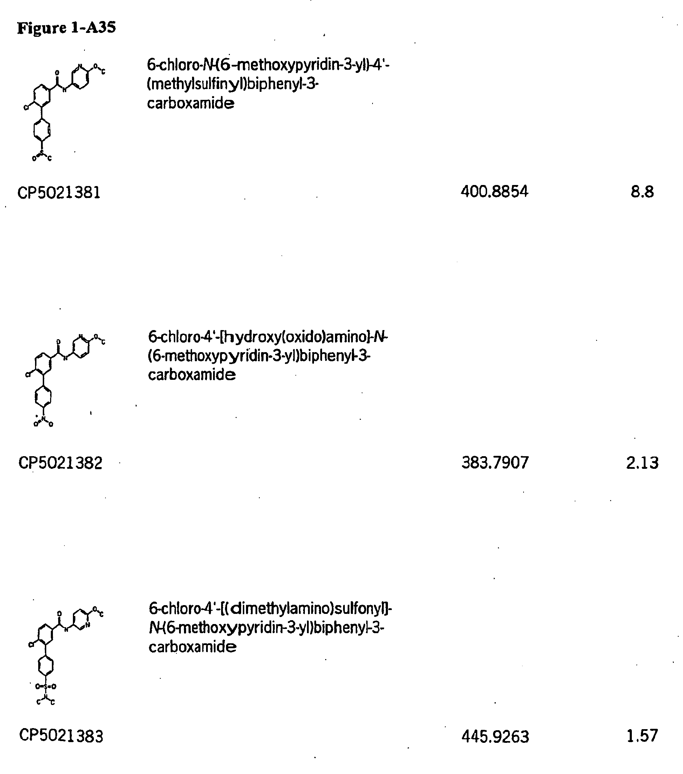

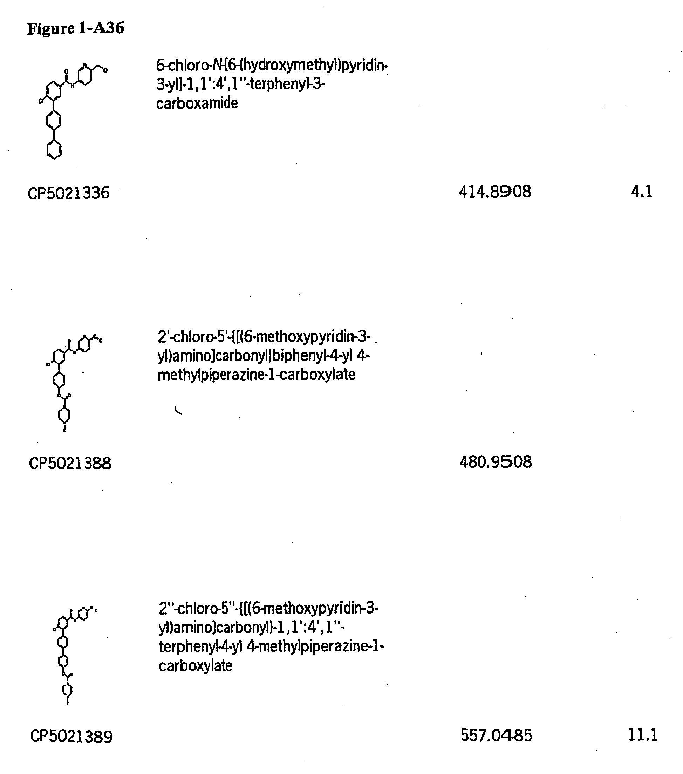

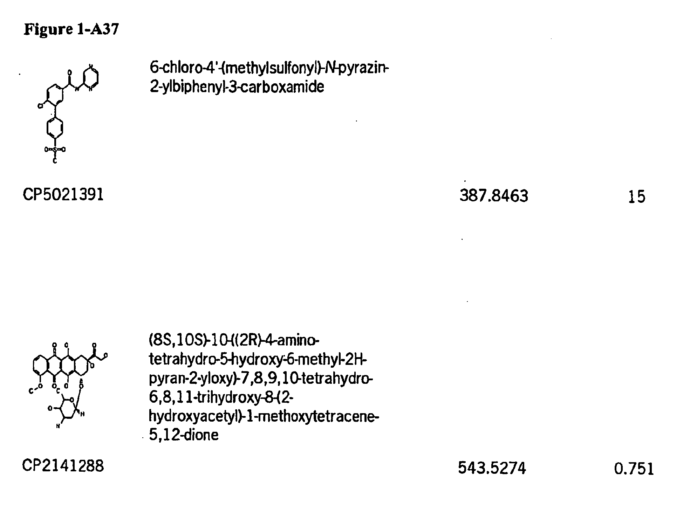

**Figure 1** is a table showing anti-tumor activity of representative compounds of the invention.

### Detailed Description of the Invention

In one aspect, the present invention provides novel compounds according the general formula I: 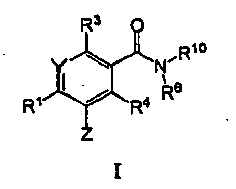 wherein
- Y: is C-R² or N;
- R¹: is selected from hydrogen, C₁-C₁₂ alkyl or halo;
- R², R³ and R⁴: are independently selected from hydrogen or halo;
- R⁶: is hydrogen or
- R⁶: is C₁-C₁₂ alkyl optionally substituted with one or two groups selected from amino, monoalkylamino or dialkylamino;
- R¹⁰: is selected from aryl, heteroaryl, heteroarylalkyl, cycloalkyl, heterocycloalkyl or heterocycloalkylalkyl, each of which is optionally substituted at the ring portion and/or alkyl portion with one to five groups selected from C₁-C₁₂ alkyl C₂-C₁₂ alkenyl, C₂-C₁₂ alkynyl, trifluoromethyl, C₁-C₁₂ alkoxy, hydroxyl, halo, amino, monoalkylamino, dialkylamino, aminoalkyl monoalkylaminoalkyl, dialkylaminoalkyl, nitro, -CN, oxo, -C(O)OR -NH-C(O)- R', -C(O)-NHR', -SR, -SO₂R, -CO₂R, or aryl, heteroaryl, cycloalkyl or heterocycloalkyl, each of which is optionally substituted with one to three groups selected from C₁-C₁₂ alkyl, C₂-C₁₂ alkenyl C₂-C₁₂ alkynyl, trifluoromethyl C₁-C₁₂ alkoxy, hydroxyl, halo, amino, monoalkylamino, dialkylamino, aminoalkyl, monoalkylaminoalkyl, dialkylaminoalkyl nitro, -CN, oxo, -C(O)OR, -NH-C(O)-R', -C(O)-NHR', -SR, -SO₂R, -CO₂R, or
- R⁶ and R¹⁰: together with the nitrogen atom to which they are attached form a 5-7 membered heteroaryl ring optionally substituted with one to three groups independently selected from C₁-C₁₂ alkyl, C₂-C₁₂ alkenyl, C₂-C₁₂ alkynyl, trifluoromethyl, C₁-C₁₂ alkoxy, hydroxyl, halo, amino, monoalkylamino, dialkylamino, aminoalkyl, monoalkylaminoalkyl, dialkylaminoalkyl, nitro, -CN, oxo, -C(O)OR, -NH-C(O)-R', -C(O)-NHR', -SR, -SO₂R, -CO₂R;
- Z: is selected from 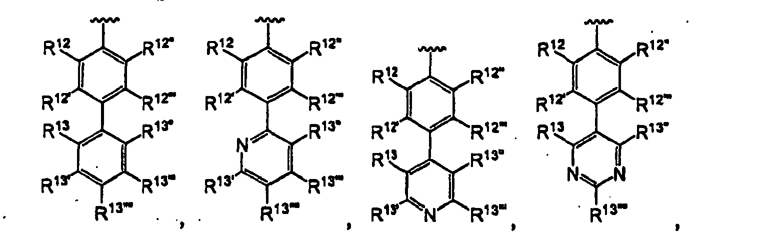 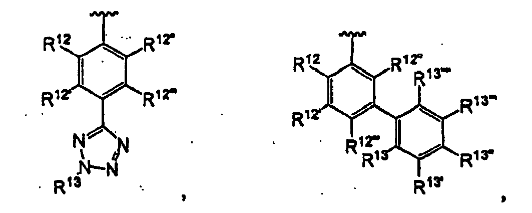 , or 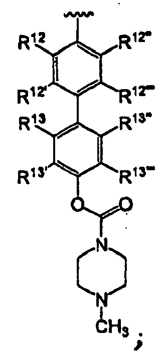
- R¹², R^{12'}, R^{12"} and R^{12'"}: are independently selected from hydrogen, C₁-C₁₂ alkyl, C₂-C₁₂ alkenyl, C₂-C₁₂ alkynyl, trifluoromethyl, C₁-C₁₂ alkoxy, hydroxyl, halo, amino, monoalkylamino, dialkylamino, aminoalkyl, monoalkylaminoalkyl, dialkylaminoalkyl, nitro, -CN, -C(O)OR, -NH-C(O)-R', -C(O)-NHR', -SR, -SO₂R, -CO₂R;
- R¹³, R^{13'}, R^{13"}, R^{13'''} and R^{13''''}: are independently selected from hydrogen, C₁-C₁₂ alkyl, C₂-C₁₂ alkenyl, C₂-C₁₂ alkynyl, trifluoromethyl, C₁-C₁₂ alkoxy, hydroxyl, halo, amino, monoalkylamino, dialkylamino, aminoalkyl, monoalkylaminoalkyl, dialkylaminoalkyl, nitro, -CN, -C(O)OR, -NH-C(O)-R', -C(O)-NHR" -SR, -SO₂R, -CO₂R;
- X: is halo;
- R: is selected from hydrogen and C₁-C₁₂ alkyl; and
- R': is independently selected from hydrogen, C₁-C₁₂ alkyl, amino, monoalkylamino, dialkylamino, arylalkyl, heteroaryl, heteroarylalkyl cycloalkyl, cycloalkylalkyl, heterocycloalkyl or heterocycloalkylalkyl; and pharmaceutically acceptable salts, esters or amides thereof.

The invention also relates to compounds of formula Ia: 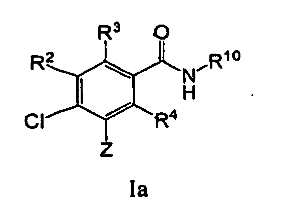 wherein R², R³, R⁴, R¹⁰ and Z are as defined above for formula I.

In an embodiment, the invention relates to compounds of formula Ia wherein R², R³ and R⁴ are hydrogen.

The invention also relates to compounds of formula Ib: 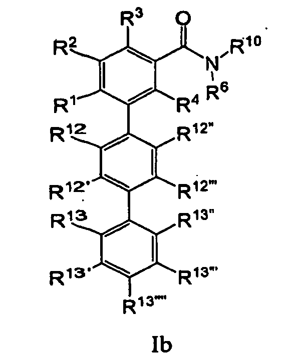 wherein, R¹, R², R³, R⁴, R⁶, R¹⁰, R¹², R^{12'}, R^{12"}, R^{12"'}, R¹³, R^{13'}, R^{13"}, R^{13"'}, and R^{13""} are as defined above for formula I.

In an embodiment, the invention relates to compounds of formula Ib wherein R¹ is hydrogen, chloro or methyl, and R², R³ and R⁴ are hydrogen.

In another embodiment, the invention relates to compounds of formula Ib wherein R^{12'} is hydrogen or halo, and R¹², R^{12"} and R^{12'''} are hydrogen.

In still another embodiment, the invention relates to compounds of formula Ib wherein R¹³, R^{13'} and R^{13"} are hydrogen.

The invention also relates to compounds of formula Ic: 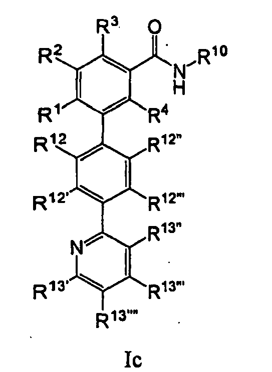 wherein R¹, R², R³, R⁴, R¹⁰, R¹², R^{12'}, R^{12"}, R^{12"'}, R^{13'}, R^{13"}, R^{13'"} and R^{13""} are as defined above for formula I.

In an embodiment, the invention relates to compounds of formula Ic wherein R¹ is chloro, and R², R³ and R⁴ are hydrogen.

In another embodiment, the invention relates to compounds of formula Ic wherein R¹², R^{12'}, R^{12"} and R^{12"'} are hydrogen.

In still another embodiment, the invention relates to compounds of formula Ic wherein R^{13'}, R^{13"}, R^{13'"} and R^{13""} are hydrogen.

The invention also relates to compounds of formula Id: 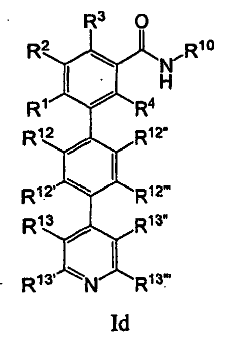 wherein R¹, R², R³, R⁴, R¹⁰, R¹², R^{12'}, R^{12"}, R^{12'''}, R¹³, R^{13'}, R^{13"} and R^{13'''} are as defined above for formula I.

In an embodiment, the invention relates to compounds of formula Id wherein R¹ is chloro, and R², R³ and R⁴ are hydrogen.

In another embodiment, the invention relates to compounds of formula Id wherein R¹², R^{12'}, R^{12"} and R^{12'''} are hydrogen.

In still another embodiment, the invention relates to compounds of formula Id wherein R¹³, R^{13'}, R^{13"} and R^{13"'} are hydrogen.

The invention also relates to compounds of formula Ie: 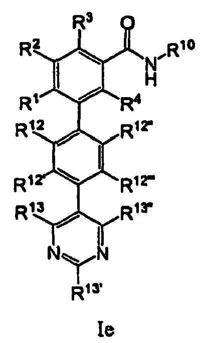 wherein R¹, R², R³, R⁴, R¹⁰, R¹³, R^{12'}, R^{12"}, R^{12'''}, R¹³, R^{13'} and R^{13"} are as defined above for formula I.

In an embodiment, the invention relates to compounds of formula Ie wherein R¹ is chloro, and R², R³ and R⁴ are hydrogen.

In another embodiment, the invention relates to compounds of formula Ie wherein R¹² R^{12'}, R^{12"} and R^{12'"} are hydrogen.

In still another embodiment, the invention relates to compounds of formula Ie wherein R¹³, R^{13'} and R^{13"} are hydrogen.

The invention also relates to compounds of formula Ii: 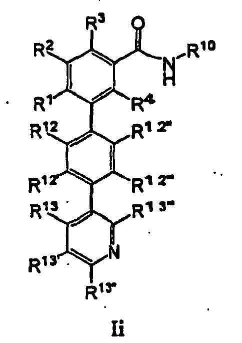 wherein R¹, R², R³, R⁴, R¹⁰, R¹², R^{12'}, R^{12"}, R^{12'''}, R¹³, R^{13'}, R^{13"} and R^{13'''} are as defined above for formula I.

In an embodiment, the invention relates to compounds of formula Ii wherein R¹ is chloro, and R², R³ and R⁴ are hydrogen.

In another embodiment, the invention relates to compounds of formula Ii wherein R¹², R^{12'}, R^{12"} and R^{12'''} are hydrogen.

In still another embodiment, the invention relates to compounds of formula Ii wherein R¹³, R^{13"} and R^{13'''} are hydrogen, and R^{13'} is selected from hydrogen, aminoalkyl, monoalkylaminoalkyl, dialkyaminoalkyl or C₁-C₁₂ alkoxy.

The invention also relates to compounds of formula Ik: 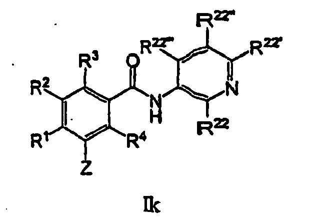 wherein R¹, R², R³, R⁴ and Z are as defined above for formula I and R²², R^{22'}, R^{2"} and R^{22'''} are independently selected from hydrogen, C₁-C₁₂ alkyl, C₂-C₁₂alkenyl, C₂-C₁₂ alkynyl, trifluoromethyl, C₁-C₁₂ alkoxy, hydroxyl, halo, amino, monoalkylamino, dialkylamino, aminoalkyl, monoalkylaminoalkyl, dialkylaminoalkyl, nitro, -CN, oxo, - C(O)OR, -NH-C(O)-R', -C(O)-NHR', -SR, -SO₂R, -CO₂R; or aryl, heteroaryl, cycloalkyl or heterocycloalkyl, each of which is optionally substituted with one to three groups selected from C₁-C₁₂ alkyl, C₂-C₁₂ alkenyl, C₂-C₁₂ alkynyl, trifluoromethyl, C₁-C₁₂ alkoxy, hydroxyl, halo, amino, monoalkylamino, dialkylamino, aminoalkyl, monoalkylaminoalkyl, dialkylaminoalkyl, nitro, -CN, oxo, -C(O)OR, -NH-C(O)-R', - C(O)-NHR', -SR, -SO₂R, -CO₂R.

In an embodiment, the invention relates to compounds of formula Ik wherein R¹ is chloro, and R², R³ and R⁴ are hydrogen.

In another embodiment, the invention relates to compounds of formula Ik wherein R²², R^{22'}, R^{22"} and R^{22"'} are selected from hydrogen, halo, C₁-C₁₂ alkyl, C₁-C₁₂ alkoxy or optionally substituted heterocyclyl.

The invention also relates to compounds of formula II: 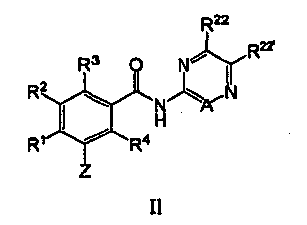 wherein A is -CH or N, and R¹, R², R³, R⁴ and Z are as defined above for formula I and R²² and R²² are independently selected from hydrogen, C₁-C₁₂ alkyl, C₂-C₁₂ alkenyl, C₂-C₁₂ alkynyl, trifluoromethyl, C₁-C₁₂ alkoxy, hydroxyl, halo, amino, monoalkylamino, dialkylamino, aminoalkyl, monoalkylaminoalkyl dialkylaminoalkyl, nitro, -CN, oxo, - C(O)OR -NH-C(O)-R', -C(O)-NHR', -SR, -SO₂R, -CO₂R; or aryl, heteroaryl, cycloalkyl or heterocycloalkyl, each of which is optionally substituted with one to three groups selected from C₁-C₁₂ alkyl, C₂-C₁₂ alkenyl, C₂-C₁₂ alkynyl, trifluoromethyl, C₁-C₁₂ alkoxy, hydroxyl, halo, amino, monoalkylamino, dialkylamino, aminoalkyl monoalkylaminoalkyl dialkylaminoalkyl, nitro, -CN, oxo, -C(O)OR, -NH-C(O)-R', - C(O)-NHR', -SR, -SO₂R, -CO₂R.

In an embodiment, the invention relates to compounds of formula II wherein R¹ is chloro, and R², R³ and R⁴ are hydrogen.

In another embodiment, the invention relates to compounds of formula II wherein R²² and R^{22'} are selected from hydrogen, halo, C₁-C₁₂ alkyl and C₁-C₁₂ alkoxy.

The invention also relates to compounds of formula Im: 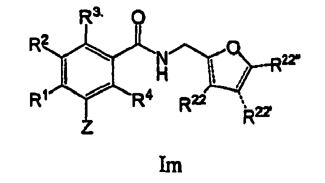 wherein the --- bonds are both present or both absent, and R¹, R², R³, R⁴ and Z are as defined above for formula I and R²², R22' and R^{22"} are independently selected from hydrogen, C₁-C₁₂ alkyl, C₂-C₁₂ alkenyl, C₂-C₁₂ alkynyl, trifluoromethyl, C₁-C₁₂ alkoxy, hydroxyl, halo, amino, monoalkylamino, dialkylamino, aminoalkyl, monoalkylaminoalkyl, dialkylaminoalkyl, nitro, -CN, oxo, -C(O)OP, -NH-C(O)-R', - C(O)-NHR', -SR, -SO₂R, -CO₂R; or aryl, heteroaryl, cycloalkyl or heterocycloalkyl, each of which is optionally substituted with one to three groups selected from C₁-C₁₂ alkyl, C₂-C₁₂ alkenyl, C₂-C₁₂ alkynyl, trifluoromethyl, C₁-C₁₂ alkoxy, hydroxyl, halo, amino, monoalkylamino, dialkylamino, aminoalkyl, monoalkylaminoalkyl, dialkylaminoalkyl, nitro, -CN, oxo, -C(O)OR, -NH-C(O)-R', -C(O)-NHR', -SR, -SO₂R, -CO₂R.

In an embodiment, the invention relates to compounds of formula Im wherein R¹ is chloro, and R², R³ and R⁴ are hydrogen.

In another embodiment, the invention relates to compounds of formula Im wherein R²² and R^{22'} are selected from hydrogen, halo, C₁-C₁₂ alkyl and C₁-C₁₂ alkoxy.

The invention also relates to compounds of formula In: 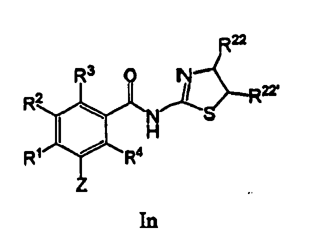 wherein A is -CH or N, and R¹, R², R³, R⁴ and Z are as defined above for formula I and R²² and R^{22'} are independently selected from hydrogen, C₁-C₁₂ alkyl, C₂-C₁₂ alkenyl, C₂-C₁₂ alkynyl, trifluoromethyl, C₁-C₁₂ alkoxy, hydroxyl, halo, amino, monoalkylamino, dialkylamino, aminoalkyl, monoalkylaminoalkyl, dialkylaminoalkyl, nitro, -CN, oxo, -C(O)OR, -NH-C(O)-R', -C(O)-NHR', -SR, -SO₂R, -CO₂R; or aryl, heteroaryl, cycloalkyl or heterocycloalkyl, each of which is optionally substituted with one to three groups selected from C₁-C₁₂ alkyl, C₂-C₁₂ alkenyl, C₂-C₁₂ alkynyl, trifluoromethyl, C₁-C₁₂ alkoxy, hydroxyl, halo, amino, monoalkylamino, dialkylamino, aminoalkyl, monoalkylaminoalkyl, dialkylaminoalkyl, nitro, -CN, oxo, -C(O)OR, -NH-C(O)-R', -C(O)-NHR', -SR, -SO₂R, -CO₂R.

In an embodiment, the invention relates to compounds of formula In wherein R¹ is chloro, and R², R³ and R⁴ are hydrogen.

In another embodiment, the invention relates to compounds of formula In wherein R²² and R^{22'} are selected from hydrogen, halo, C₁-C₁₂ alkyl and C₁-C₁₂ alkoxy.

The invention also relates to compounds of formula Io: 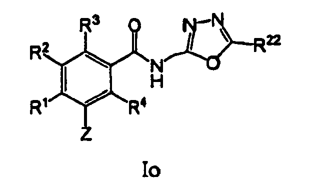 wherein A is -CH or N, and R¹, R², R³, R⁴ and Z are as defined above for formula I and R²² is as independently selected from hydrogen, C₁-C₁₂ alkyl, C₂-C₁₂ alkenyl, C₂-C₁₂ alkynyl, trifluoromethyl, C₁-C₁₂ alkoxy, hydroxyl, halo, amino, monoalkylamino, dialkylamino, aminoalkyl, monoalkylaminoalkyl, dialkylaminoalkyl, nitro, -CN, oxo, -C(O)OR, -NH-C(O)-R', -C(O)-NHR', -SR, -SO₂R, -CO₂R; or aryl, heteroaryl, cycloalkyl or heterocycloalkyl, each of which is optionally substituted with one to three groups selected from C₁-C₁₂ alkyl, C₂-C₁₂ alkenyl, C₂-C₁₂ alkynyl, trifluoromethyl, C₁-C₁₂ alkoxy, hydroxyl, halo, amino, monoalkylamino, dialkylamino, aminoalkyl, monoalkylaminoalkyl, dialkylaminoalkyl, nitro, -CN, oxo, -C(O)OR, -NH-C(O)-R', -C(O)-NHR'; -SR, -SO₂R, -CO₂R.

In an embodiment, the invention relates to compounds of formula Io wherein R¹ is chloro, and R², R³ and R⁴ are hydrogen.

In another embodiment, the invention relates to compounds of formula Io wherein R²² and R^{22'} are selected from hydrogen, halo, C₁-C₁₂ alkyl, C₁-C₁₂ alkoxy and optionally substituted aryl.

A family of specific compounds of particular interest within formula I consists of compounds and pharmaceutically-acceptable salts thereof as follows (all compounds are named via the structure naming plug-in to either ChemDraw Ultra 6.0 or 8.0, or ACDLabs version 6.0, all versions using IUPAC rules):
6-chloro-*N*-(tetrahydrofuran-2-ylmethyl)-1,1':4',1"-terphenyl-3-carboxamide;
6-chloro-*N*-(2-furylmethyl)-1,1':4',1"-terphenyl-3-carboxamide;
6-chloro-*N*-(6-cbloropyridin-3-yl)-1,1':4',1"-terphenyl-3-carboxamide;
6-chloro-*N*-(4,5-dihydro-1,3-thiazol-2-yl)-1,1':4',1"-terphenyl-3-carboxamide;
6-chloro-*N*-(2-pyrrolidin-1-ylethyl)-1,1':4',1"-terphenyl-3-carboxamide;
6-chloro-*N*-(5-chloropyrimidin-2-yl)-1,1':4',1"-terphenyl-3-carboxamide;
6-chloro-*N-*(3-methylisoxazol-5-yl)-1,1':4',1"-terphenyl-3-carboxamide;
6-chloro-*N*-[6-(methylsulfonyl)-1,3-benzothiazol-2-yl]-1,1':4',1"-terphenyl-3-carboxamide;
6-chloro-*N-*1,2,4-triazin-3-yl-1,1':4',1"-terphenyl-3-carboxamide;
methyl 3-{[(6-chloro-1,1':4',1"-terphenyl-3-yl)carbonyl]amino}benzoate;
6-chloro-*N*-(5-phenyl-1,3,4-oxadiazol-2-yl)-1,1':4',1"-terphenyl-3-carboxamide;
6-chloro-*N*-[3-(2-methyl-1,3-dioxolan-2-yl)pbenyl]-1,1':4',1"-terphenyl-3-carboxamide;
6-chloro-*N*-(4-methoxyphenyl)-1,1':4',1"-terphenyl-1-3-carboxamide;
6-chloro-*N*-quinolin-6-yl-1,1':4',1"-terphenyl-3-carboxamide;
6-chloro-*N*-pyrazin-2-yl-1,1':4',1"-terphenyl-3-carboxamide;
*N*-(5-bromopyridin-2-yl)-6-chloro-1,1':4',1"-terphenyl-3-carboxamide;
6-chloro-*N*-(6-methoxypyridin-3-yl)-1,1':4',1"-terphenyl-3-carboxamide;
6-chloro-*N*-(6-fluoropyridin-3-yl)-1,1':4',1"-terphenyl-3-carboxamide
6-chloro-*N*-(5-chloropyridin-2-yl)-1,1':4',1"-terphenyl-3-carboxamide;
6-chloro-*N*-(6-methoxypyridin-3-yl)-4'-pyridin-2-ylbiphenyl-3-carboxamide
6-chloro-3'-fluoro-*N*-(6-methoxypyridin-3-yl)-1,1':4',1"-terphenyl-3-carboxamide;
6-chloro-*N*-(6-methoxypyridin-3-yl)-4'-pyridin-4-ylbiphenyl-3-carboxamide;
6-chloro-*N*-(4-methylphenyl)-1,1':4',1"-terphenyl-3-caxboxamide;
6-chloro-*N*-(3-fluom-4-methoxyphenyl)-1,1':4',1"-terphenyl-3-emboxamide;
6-chloro-*N*-(6-methoxypyridin-3-yl)-4'-pyrimidin-5-ylbiphenyl-3-carboxamide;
6-chloro-*N*-[2-(dimethylamino)ethyl]-*N*-pyridin-3-yl-1,1':4',1"-terphenyl-3-carboxamide;
*N*-(2-pyrrolidin-1-ylethyl)-1,1':4,1"-terphenyl-3-carboxamide;
6-chloro-*N*-[6-(4-methylpiperazin-1-yl)pyridin-3-yl]-1,1':4',1"-terphenyl-3-carboxamide;
6-chloro-*N*-(6-morpholin-4-ylpyridin-3-yl)-1,1':4',1"-terphenyl-3-carboxamide;
4-chloro-*N*-(6-methoxypyridin-3-yl)-3-(6-phenylpyridin-3-yl)benzamide;
6-chloro-*N*-[6-(methylthio)pyridin-3-yl]-1,1':4',1"-terphenyl-3-carboxamide;
6-chloro-*N*-(5-methoxypyrazin-2-yl)-1,1':4',1"-terphenyl-3-carboxamide;
6-chloro-*N*-[2-(methylthio)pyrimidin-5-yl]-1,1':4',1"-terphenyl-3-carboxamide
6-chloro-*N*-(2-methoxypyrimidin-5-yl)-1,1':4',1"-terphenyl-3-carboxamide;
6-chloro-*N*(6-methylpyridin-3-yl)-1,1':4',1"-terphenyt-3-carboxamide;
6-chloro-*N*-(6-oxo-1,6-dihydropyridin-3-yl)-4'-pyridin-2-ylbiphenyl-3-carboxamide;
6-chloro-*N*-(6-methoxypyridin-3-yl)-4'-(2-methyl-2*H-*tetrazol-5-yl)biphenyl-3-carboxamide; 6-chloro-*N*-(6-methoxypyridin-3-yl)-1,1':3',1"-terphenyl-3-carboxamide;
*N*-(6-methoxypyridin-3-yl)-6-methyl-1,1':4',1"-terphenyl-3-carboxamide;
5-biphenyl-4-yl.6-chloro-*N*-(6-methoxypyridin-3-yl)micotinamide;
6-chloro-*N*-(6-methoxypyridin-3-yl)-4'-pyridin-2-ylbiphenyl-3-carboxamide;
6-chloro-3"-formyl-*N*-(6-methoxypyridin-3-yl).1,1':4',1"-terphenyl-3-carboxamide;
6-chloro-3"-[(dimethylamino)methyl]-*N*-(6-methoxypyridin-3-yl)-1,1':4',1"-terphenyl-3-carboxamide;
6-chloro-3"-(hydroxymethyl)-*N*-(6-methoxypyridin-3-yl)-1,1':4',1"-terphenyl-3-carboxamide;
6-chloro-4"-[(dimethylamino)methyl]-*N*-(6-methoxypyridin-3-yl)-1,1':4',1"-terphenyl-3-carboxamide;
6-chloro-4"-(hydroxymethyl)-*N*-(6-methoxypyridin-3-yl)-1,1':4',1"-terphenyl-3-carboxamide;
6-chloro-*N*-(6-methoxypyridin-3-yl)-3"-(methylsulfonyl)-1,1':4',1"-terphenyl-3-carboxamide;
6-chloro-*N*-(6-methoxypyridin-3-yl)-4'-pyridin-3-ylbiphenyl-3-carboxamide;
4"-(aminomethyl)-6-chloro-*N*-(6-methoxypyridin-3-yl)-1,1':4',1"-terphenyl-3-carboxamide;
6-chloro4'-[5-(hydroxymethyl)pyridin-3-yl]-*N*-(6-methoxypyridin-3-yl)biphenyl-3-carboxamide;
6-chloro-*N*³-(6-methoxypyridin-3-yl)-1,1':4',1"-terphenyl-3,3"-dicarboxamide;
6-chloro-3"-hydroxy-*N*-(6-methoxypyridin-3 -yl)-1,1':4',1"-terphenyl-3-carboxamide;
6-chloro-4"-hydroxy-*N*-(6-methoxypyridin-3 -yl)-1,1':4',1"-terphenyl-3-carboxamide;
6-chloro-4'-{5-[(dimethylamino)methyl]pyridin-3-yl}-*N*-(6-methoxypyridin-3-yl)biphenyl-3-carboxamide;
5-fluoro-*N*-(6-methoxypyridin-3-yl)-1,1':4',1"-terphenyl-3-carboxamide;
6-chloro-*N*-(3-[(morpholin-4-ylacetyl)amino]phenyl)-1,1':4',1"-terphenyl-3-carboxamide;
6-chloro-*N*-{4-[(morpholin-4-ylacetyl)amino]phenyl}-1,1':4',1"-terphenyl-3-carboxamide;
6-chloro-*N*-{6-[(4-methylpiperazin-1-yl)methyl]pyridin-3-yl}-1,1':4',1"-terphenyl-3-carboxamide;
6-chloro-*N*-[6-(hydroxymethyl)pyridin-3-yl]1,1':4',1"-terphenyl-3-carboxamide;
2"-chloro-5"-{[(6-methoxypyridin-3-yl)amino]carbonyl}-1,1':4',1"-terphenyl-4-yl methytpiperazine-1-carboxylate;
2-{[(6-chloro-1,1':4',1"-terphenyl-3-yl)carbonyl]amino}indane-2-carboxylic acid;
(4*S*)-3-[(6-chloro-1,1':4',1"-terphenyl-3-yl)carbonyl]-1,3-thiazolidine-4-carboxylic acid;
*N*-[(6-chloro-1,1':4',1"-terphenyl-3-yl)carbonyl]-D-methionine; and
*N*-[(6-chloro-1,1':4',1"-terphenyl-3-yl)carbonyl]-3-(7,7a-dihydro)-1*H-*indol-3-yl)-D-alanine.

The invention also relates to a pharmaceutical composition comprising a compound according to the invention and a pharmaceutically acceptable carrier.

A further aspect of the invention is the use of
a compound of the formula: 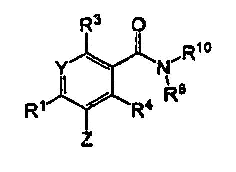 or a pharmaceutically acceptable salt, ester or amide thereof,
wherein
- Y: is C-R² or N;
- R¹: is selected from hydrogen, C₁-C₁₂ alkyl or halo;
- R², R³ and R⁴: are independently selected from hydrogen or halo;
- R⁶: is hydrogen, or
- R⁶: is C₁-C₁₂ alkyl optionally substituted with one or two groups selected from amino, monoalkylamino or dialkylamino;
- R¹⁰: is selected from aryl, arylalkyl, heteroaryl, heteroarylalkyl, cycloalkyl, cycloalkylalkyl, heterocycloalkyl or heterocycloalkylalkyl, each of which is optionally substituted at the ring portion and/or alkyl portion with one to five groups selected from C₁-C₁₂ alkyl, C₂- C₁₂ alkenyl, C₂-C₁₂ alkynyl, trifluororrxethyl, C₁-C₁₂ alkoxy, hydroxyl, halo, amino, monoalkylamino, dialkylamino, aminoalkyl, monoalkylaminoalkyl, dialkylaminoalkyl, nitro, -CN, oxo, -C(O)OR, -NH-C(O)-R', -C(O)-NHR', -SR, -SO₂R, -CO₂R, or aryl, heteroaryl, cycloalkyl or heterocycloalkyl, each of which is optionally substituted with one to three groups selected from C₁-C₁₂ alkyl, C₂-C₁₂ alkenyl, C₂-C₁₂ alkynyl, trifluoromethyl, C₁-C₁₂ alkoxy, hydroxyl, halo, amino, monoalkylamino, dialkylamino, aminoalkyl, monoalkylaminoalkyl, dialkylaminoalkyl, nitro, -CN, oxo, -C(O)OR, -NH-C(O)-R', -C(O)-NHR', -SR, -SO₂R, -CO₂R, or
- R⁶ and R¹⁰: together with the nitrogen atom to which they are attached form a 5-7 membered heteroaryl or heterocyclic ring optionally substituted with one to three groups independently selected from C₁-C₁₂ alkyl, C₂-C₁₂ alkenyl, C₁-C₁₂ alkynyl, trifluoromethyl, C₁-C₁₂ alkoxy, hydroxyl, halo, amino, monoalkylamino, dialkylamino, aminoalkyl, monoalkylaminoalkyl, dialkylaminoalkyl, nitro, -CN, oxo, -C(O)OR, -NH- C(O)-R', -C(O)-NHR', -SR, -SO₂R, -CO₂R;
- Z: is selected from 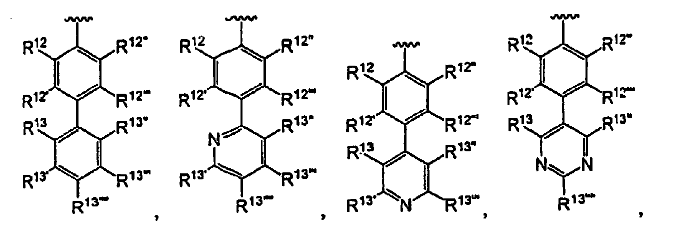 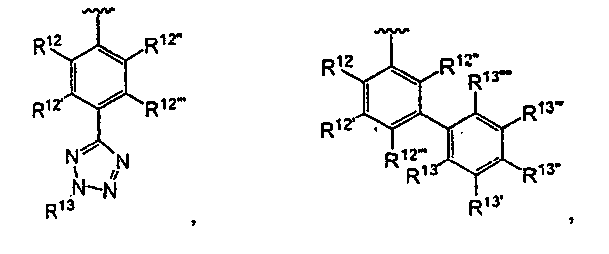 , or 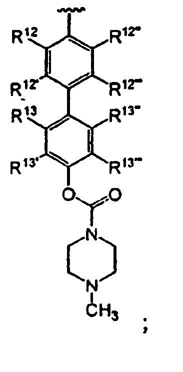
- R¹² R^{12'}, R^{12"} and R^{12'"}: are independently selected from hydrogen, C₁-C₁₂ alkyl, C₂- C₁₂ alkenyl, C₂-C₁₂ alkynyl, trifluoromethyl, C₁-C₁₂ alkoxy, hydroxyl, halo, amino, monoalkylamino, dialkylamino, aminoalkyl, monoalkylaminoalkyl, dialkylaminoalkyl, nitro, -CN, -C(O)OR, -NH-C(O)-R', -C(O)-NHR', -SR, - SO₂R, -CO₂R;
- R¹³ R^{13'}, R^{13"}, R^{13"'} and R^{13""}: are independently selected from hydrogen, C₁-C₁₂ alkyl, C₂-C₁₂ alkenyl, C₂-C₁₂ alkynyl, trifluoromethyl, C₁-C₁₂ alkoxy, hydroxyl, halo, amino, monoalkylamino, dialkylamino, aminoalkyl, monoalkylaminoalkyl, dialkylaminoalkyl, nitro, -CN, -C(O)OR, -NH-C(O)R', -C(O)-NHR', -SR, -SO₂R, -CO₂R;
- X: is halo;
- R: is selected from hydrogen and C₁-C₁₂ alkyl; and
- R': is independently selected from hydrogen, C₁-C₁₂ alkyl, amino, monoalkylamino, dialkylamino, arylalkyl, heteroaryl, heteroarylalkyl, cycloalkyl, cycloalkylalkyl, heterocycloalkyl or heterocycloalkylalkyl;
for the preparation of a pharmaceutical composition for treating a tumor.

In a preferred embodiment of the use according to the invention, the tumor is selected from the group consisting of a sarcoma, a melanoma, a neuroblastoma, a carcinoma and a mesothelioma.

The invention also provides a compound of the formula: 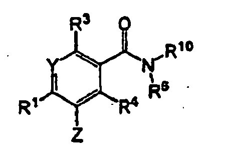 or a pharmaceutically acceptable salt, ester or amide thereof,
wherein
- Y: is C-R² or N;
- R¹: is selected from hydrogen, C₁-C₁₂ alkyl or halo;
- R², R³ and R⁴: are independently selected from hydrogen or halo;
- R⁶: is hydrogen, or
- R⁶: is C₁-C₁₂ alkyl optionally substituted with one or two groups selected from amino, monoalkylamino or dialkylamino;
- R¹⁰: is selected from aryl, arylalkyl, heteroaryl, heteroarylalkyl, cycloalkyl, cycloalkylalkyl, heterocycloalkyl or heterocycloalkylalkyl, each of which is optionally substituted at the ring portion and/or alkyl portion with one to five groups selected from C₁-C₁₂ alkyl, C₂-C₁₂ alkenyl, C₂-C₁₂ alkynyl, trifluororrxethyl, C₁-C₁₂ alkoxy, hydroxyl, halo, amino, monoalkylamino, dialkylamino, aminoalkyl, monoalkylaminoalkyl, dialkylaminoalkyl, nitro, - CN, oxo, -C(O)OR, -NH-C(O)-R', -C(O)-NHR', -SR, -SO₂R, -CO₂R, or aryl, heteroaryl, cycloalkyl or heterocycloalkyl, each of which is optionally substituted with one to three groups selected from C₁-C₁₂ alkyl, C₂-C₁₂ alkenyl, C₂-C₁₂ alkynyl, trifluoromethyl, C₁-C₁₂ alkoxy, hydroxyl, halo, amino, monoalkylamino, dialkylamino, aminoalkyl, monoalkylaminoalkyl, dialkylaminoalkyl, nitro, -CN, oxo, -C(O)OR, -NH-C(O)-R', -C(O)-NHR', -SR, -SO₂R, -CO₂R, or
- R⁶ and R¹⁰: together with the nitrogen atom to which they are attached form a 5-7 membered heteroaryl or heterocyclic ring optionally substituted with one to three groups independently selected from C₁-C₁₂ alkyl, C₂-C₁₂ alkenyl, C₂-C₁₂ alkynyl, trifluoromethyl, C₁-C₁₂ alkoxy, hydroxyl, halo, amino, monoalkylamino, dialkylamino, aminoalkyl, monoalkylaminoalkyl, dialkylaminoalkyl, nitro, -CN, oxo, -C(O)OR, -NH-C(O)-R', -C(O)-NHR', -SR, -SO₂R, -CO₂R;
- Z: is selected from 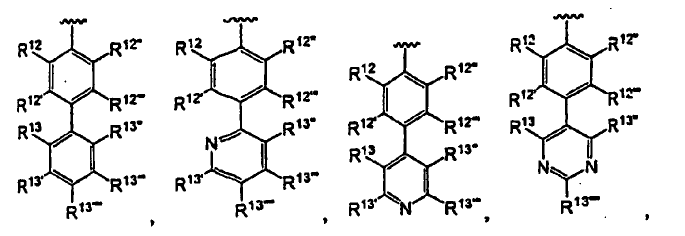 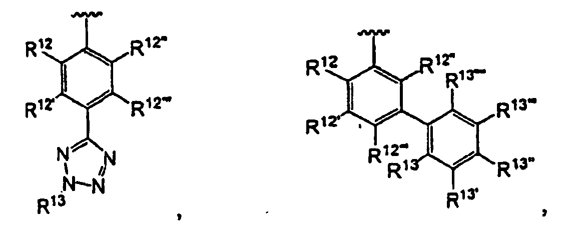 , or 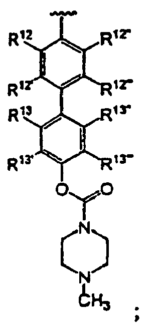
- R¹², R^{12'}, R^{12"} and R^{12'"}: are independently selected from hydrogen, C₁-C₁₂ alkyl, C₂- C₁₂ alkenyl, C₂-C₁₂ alkynyl, trifluoromethyl, C₁-C₁₂ alkoxy, hydroxyl, halo, amino, monoalkylamino, dialkylamino, aminoalkyl, monoalkylaminoalkyl, dialkylaminoalkyl, nitro, -CN, -C(O)OR, -NH-C(O)-R', -C(O)-NHR', -SR, - SO₂R, -CO₂R,
- R¹³, R^{13'}, R^{13"}, R^{13"'} and R^{13""}: are independently selected from hydrogen, C₁-C₁₂ alkyl, C₂-C₁₂ alkynyl, C₂-C₁₂ alkynyl, trifluoromethyl, C₂-C₁₂ alkoxy, hydroxyl, halo, amino, monoalkylamino, dialkylamino, aminoalkyl, monoalkylaminoalkyl, dialkylaminoalkyl, nitro, -CN, -C(O)OR, -NH-C(O)-R', -C(O)-NHR', -SR, -SO₂R, -CO₂R;
- X: is halo;
- R: is selected from hydrogen and C₁-C₁₂ alkyl; and
- R': is independently selected from hydrogen, C₁-C₁₂ alkyl, amino, monoalkylamino, dialkylamino, arylalkyl, heteroaryl, heteroarylalkyl, cycloalkyl, cycloalkylalkyl, heterocycloalkyl or heterocycloalkylalkyl;
for use in the treatment of a tumor, preferably a tumor selected from the group consisting of a sarcoma, a melanoma, a neuroblastoma, a carcinoma and a mesothelioma.

By "alkyl" and slower alkyl" in the present invention, either alone or within other terms such as "alkylamino,", is meant straight or branched chain alkyl groups having 1-12 carbon atoms, such as, methyl, ethyl, propyl, isopropyl, n-butyl, sec-butyl, tert-butyl, pentyl, 2-pentyl, isopentyl, neopentyl, hexyl, 2-hexyl, 3-hexyl, and 3-methylpentyl. It is understood that in cases where an alkyl chain of a substituent (e.g. of an alkyl, alkoxy or alkenyl group) is within a distinct range, it will be so indicated in the second "C" as, for example, "C₁-C₆" indicates a maximum of 6 carbons. The alkyl groups herein may be substituted in one or more substitutable positions with various groups. For example, such alkyl groups may be optionally substituted with C₁-C₆ alkyl, C₁-C₆ alkoxy, halogen, hydroxy, cyano, nitro, amino, mono(C₁-C₆)alkylamino, di(C₁-C₆)alkylamino, C₂-C₆alkenyl, C₂-C₆alkynyl, C₁-C₆ haloalkyl, C₁-C₆ haloalkoxy, amino(C₁-C₆)alkyl, mono(C₁-C₆)alkylamino(C₁-C₆)alkyl, di(C₁-C₆)alkylamino(C₁-C₆)alkyl or =O.

By "alkoxy" and "lower alkoxy" in the present invention is meant straight or branched chain alkyl groups having 1-12 carbon atoms, attached through at least one divalent oxygen atom, such as, for example, methoxy, ethoxy, propoxy, isopropoxy, n-butoxy, sec-butoxy, tert-butoxy, pentoxy, isopentoxy, neopentoxy, hexoxy, and 3-methylpentoxy. The alkoxy groups herein may be substituted in one or more substitutable positions with various groups. For example, such alkoxy groups may be optionally substituted with C₁-C₆ alkyl, C₁-C₆ alkoxy, halogen, hydroxy, cyano, nitro, amino, mono(C₁-C₆)alkylamino, di(C₁-C₆)alkylamino, C₂-C₆alkenyl, C₂-C₆alkynyl, C₁-C₆ haloalkyl, C₁-C₆ haloalkoxy, amino(C₁-C₆)alkyl, mono(C₁-C₆)alkylamino(C₁-C₆)alkyl, di(C₁-C₆)atkylamino(C₁-C₆)alkyl or =O.

The term "alkenyl" or "lower alkyenyl" embraces linear or branched radicals having at least one carbon-carbon double bond of two to twelve atoms. More preferred alkenyl radicals are those radicals having two to about four carbon atoms. Examples of alkenyl radicals include ethenyl, 2-propenyl, alkyl, butenyl and 4-methylbutenyl. The terms "alkenyl" and "lower alkenyl", embrace radicals having "cis" and "trans" orientations, or alternatively, "E" and "Z" orientations. The alkenyl groups herein may be alkenyl groups may be optionally substituted with C₁-C₆ alkyl, C₁-C₆ alkoxy, halogen, hydroxy, cyano, nitro, amino, mono(C₁-C₆)alkylamino, di(C₁-C₆)alkylamino, C₂-C₆alkenyl, C₂-C₆alkynyl, C₁-C₆ haloalkyl, C₁-C₆ haloalkoxy, amino(C₁-C₆)alkyl, mono(C₁-C₆)alkylamino(C₁-C₆)alkyl, di(C₁-C₆)alkylamino(C₁-C₆)alkyl or =O.

The term "alkynyl" embraces linear or branched radicals having at least one carbon-carbon triple bond of two to twelve carbon atoms. More preferred alkynyl radicals are those radicals having two to about four carbon atoms. Examples of alkynyl radicals include ethynyl, 2-propynyl, and 4-methylbutynyl. The alkynyl groups herein may be substituted in one or more substitutable positions with various groups. For example, such alkynyl groups may be optionally substituted with C₁-C₆ alkyl, C₁-C₆ alkoxy, halogen, hydroxy, cyano, nitro, amino, mono(C₁-C₆)alkylamino, di(C₁-C₆)alkylamino, C₂-C₆alkenyl, C₂-C₆alkynyl, C₁-C₆ haloalkyl, C₁-C₆ haloalkoxy, amino(C₁-C₆)alkyl, mono(C₁-C₆)alkylamino(C₁-C₆)alkyl, di(C₁-C₆)alkylamino(C₁-C₆)alkyl or =O.

The term "halo" or "halogen" means halogens such as fluorine, chlorine, bromine or iodine atoms.

By "aryl" is meant an aromatic carbocyclic group having a single ring (e.g., phenyl), multiple rings (e.g., biphenyl), or multiple condensed rings in which at least one is aromatic, (e.g., 1,2,3,4-tetrahydronaphthyl, naphthyl), wherein such rings may be attached together in a pendent manner or may be fused. The term "aryl" embraces aromatic radicals such as phenyl, naphthyl, tetrahydronaphthyl, indane and biphenyl. More preferred aryl is phenyl. The aryl groups herein may be substituted in one or more substitutable positions with various groups. For example, such aryl groups may be optionally substituted with C₁-C₆ alkyl, C₁-C₆ alkoxy, halogen, hydroxy, cyano, nitro, amino, mono(C₁-C₆)alkylamino, di(C₁-C₆)alkylamino, C₂-C₆alkenyl, C₂-C₆alkynyl, C₁-C₆ haloalkyl, C₁-C₆ haloalkoxy, amino(C₁-C₆)alkyl, mono(C₁-C₆)alkylamino(C₁-C₆)alkyl, di(C₁-C₆)alkylamino(C₁-C₆)alkyl or =O.

By "heteroaryl" is meant a single ring, multiple rings, or multiple condensed rings in which at least one is aromatic, wherein such rings may be attached together in a pendent manner or may be fused. The ring systems contain of from between 9-15 atoms containing at least one and up to four heteroatoms selected from nitrogen, oxygen, or sulfur. Examples include, but are not limited to, pyrrolyl, pyrrolinyl, imidazolyl, pyrazolyl, 2-pyridinyl, 3-pyridinyl, 4-pyridinyl, pyrimidinyl, pyrazinyl and pyridazinyl. The heteroaryl groups herein may be substituted in one or more substitutable positions with various groups. For example, such heteroaryl groups may be optionally substituted with C₁-C₆ alkyl, C₁-C₆ alkoxy, halogen, hydroxy, cyano, nitro, amino, mono(C₁-C₆)alkylamino, di(C₁-C₆)alkylamino, C₂-C₆alkenyl, C₂-C₆alkynyl, C₁-C₆ haloalkyl, C₁-C₆ haloalkoxy, amino(C₁-C₆)alkyl, mono(C₁-C₆)alkylamino(C₁-C₆)alkyl, di(C₁-C₆)alkylamino(C₁-C₆)alkyl or =O.

As used herein, the term "cycloalkyl" refers to saturated carbocyclic radicals having three to twelve carbon atoms. The cycloalkyl can be monocyclic, or a polycyclic fused or spiro system, and can optionally contain a double bond. Examples of such radicals include cyclopropyl, cyclobutyl, cyclopentyl, cyclohexyl and cycloheptyl. The cycloalkyl groups herein are unsubstituted or, as specified, substituted in one or more substitutable positions with various groups. For example, such cycloalkyl groups may be optionally substituted with C₁-C₆ alkyl, C₁-C₆ alkoxy, halogen, hydroxy, cyano, nitro, amino, oxo, mono(C₁-C₆)alkylamino, di(C₁-C₆)alkylamino, C₂-C₆alkenyl, C₂-C₆alkynyl, C₁-C₆ haloalkyl, C₁-C₆ haloalkoxy, amino(C₁-C₆)alkyl, mono(C₁-C₆)alkylamino(C₁-C₆)alkyl or di(C₁-C₆)alkylamino(C₁-C₆)alkyl.

By "heterocycle" or "heterocycloalkyl" is meant one or more carbocyclic ring systems which includes fused and spiro ring systems of 9-15 atoms containing at least one and up to four heteroatoms selected from nitrogen, oxygen, or sulfur. The heterocycle may optionally contain a double bond. Examples of heterocycles of the present invention include morpholinyl, thiomorpholinyl, thiomorpholinyl S-oxide, thiomorpholinyl S,S-dioxide, piperazinyl, homopiperazinyl, pyrrolidinyl, pyrrolinyl, tetrahydropyranyl, piperidinyl, tetrahydrofuranyl, tetrahydrothienyl, homopiperidinyl, homomorpholinyl, homothiomorpholinyl, homothiomorpholinyl S,S-dioxide, oxazolidinonyl, dihydropyrazolyl, dihydropyrrolyl, dihydropyrazinyl, dihydropyridinyl, dihydropyrimidinyl, dihydrofuryl, dihydropyranyl, tetrahydrothienyl S-oxide, tetrahydrothienyl S,S-dioxide and homothiomorpholinyl S-oxide. The heterocycle groups herein may be substituted in one or more substitutable positions with various groups. For example, such heterocycle groups may be optionally substituted with C₁-C₆ alkyl, C₁-C₆ alkoxy, halogen, hydroxy, cyano, nitro, amino, mono(C₁-C₆)alkylamino, di(C₁-C₆)alkylamino, C₂-C₆alkenyl, C₂-C₆alkynyl, C₁-C₆ haloalkyl, C₁-C₆ haloalkoxy, amino(C₁-C₆)alkyl, mono(C₁-C₆)alkylamino(C₁-C₆)alkyl, di(C₁-C₆)alkylamino(C₁-C₆)alkyl or =O.

Any term that includes two radicals such as, for example, "arylalkyl", denotes the first radical, or aryl as in the example, attached to the concluding radical, or alkyl as in the example. The concluding radical is attached to the substituent in question.

The compounds of the invention can be synthesized by procedures known in the art. A representative example is depicted below in Scheme 1.

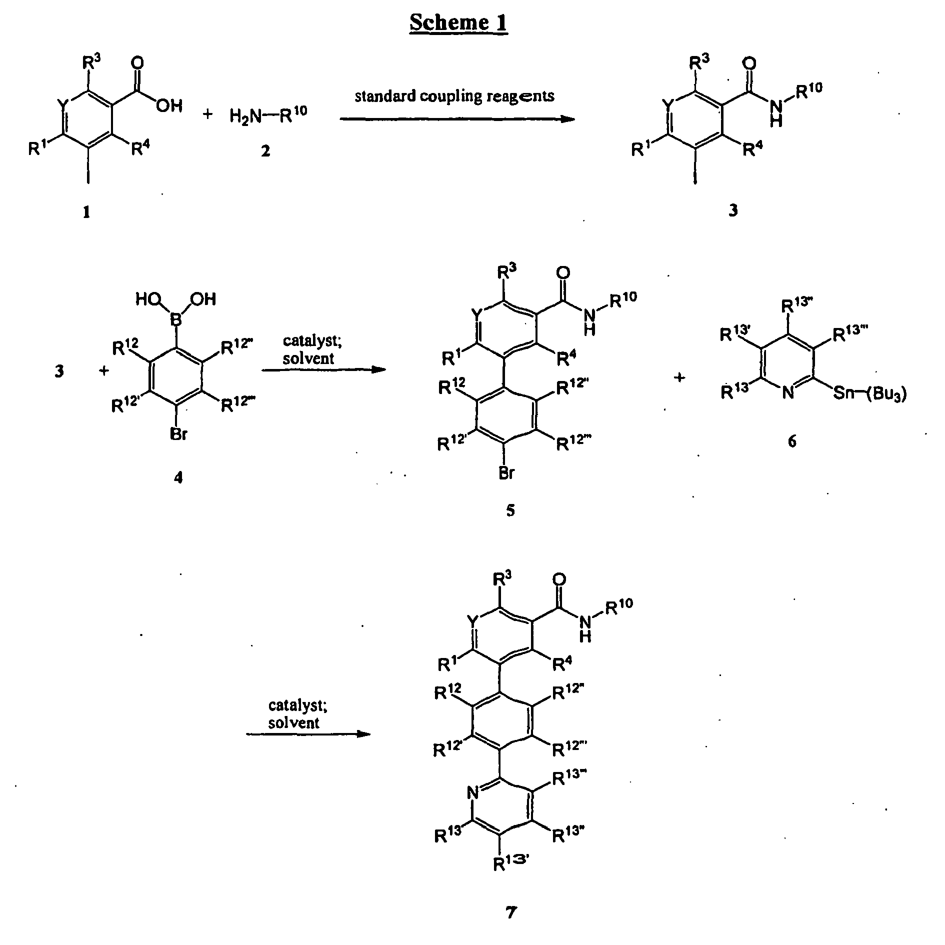

According to scheme 1, the amide **3** can be prepared by coupling the acid **1** with the amine **2** under standard coupling conditions, such as, for example, (3-dimethylaminopropyl)-ethyl-carbodiimide-HCl salt (EDAC) and 1-hydroxybenzotriazole (HOBT) and optionally in the presence of a base such as diisopropylethylamine in an appropriate solvent. Next, the amide **3** can be coupled to a 4-halo-aryl- or heteroaryl-boronic acid **4** with a catalyst, including but not limited to, tetrakis(triphenylphosphine)palladium, optionally in the presence of base, for example, potassium carbonate, in a solvent such as dioxane or tetrahydrofuran (THF). The resulting 4-halo-bicyclic-4-benzoic acid **5** can subsequently be coupled to a tributylstannylaryl or heteroaryl **6** under similar, but not necessarily the exact, conditions described above in the previous step, to afford the tricyclic compound **7.**

Compounds of the present invention can possess, in general, one or more asymmetric carbon atoms and are thus capable of existing in the form of optical isomers as well as in the form of racemic or non-racemic mixtures thereof. Unless otherwise indicated, the compounds of the present invention, as depicted or named, may exist as the racemate, a single enantiomer, or any uneven (i.e. non 50/50) mixture of enantiomers. The optical isomers can be obtained by resolution of the racemic mixtures according to conventional processes, *e.g*., by formation of diastereoisomeric salts, by treatment with an optically active acid or base. Examples of appropriate acids are tartaric, diacetyltartaric, dibenzoyltartaric, ditoluoyltartaric, and camphorsulfonic acid and then separation of the mixture of diastereoisomers by crystallization followed by liberation of the optically active bases from these salts. A different process for separation of optical isomers involves the use of a chiral chromatography column, such as, for example, a CHIRAL-AGP column, optimally chosen to maximize the separation of the enantiomers. Still another available method involves synthesis of covalent diastereoisomeric molecules by reacting compounds of the invention with an optically pure acid in an activated form or an optically pure isocyanate. The synthesized diastereoisomers can be separated by conventional means such as chromatography, distillation, crystallization or sublimation, and then hydrolyzed to deliver the enantiomerically pure compound. The optically active compounds of the invention can likewise be obtained by using optically active starting materials. These isomers may be in the form of a free acid, a free base, an ester or a salt.

The compounds of the invention include pharmaceutically acceptable salts, esters, amides, and prodrugs therof, including but not limited to carboxylate salts, amino acid addition salts, esters, amides, and prodrugs of the compounds of the present invention which are, within the scope of sound medical judgment, suitable for use in contact with the tissues of patients without undue toxicity, irritation, allergic response, and the like, commensurate with a reasonable benefit/risk ratio, and effective for their intended use, as well as the zwitterionic forms, where possible, of the compounds of the invention. The term "salts" refers to the relatively non-toxic, inorganic and organic acid addition salts of compounds of the present invention. These salts can be prepared in situ during the final isolation and purification of the compounds or by separately reacting the purified compound in its free base form with a suitable organic or inorganic acid and isolating the salt thus formed. Representative salts include the hydrobromide, hydrochloride, sulfate, bisulfate, nitrate, acetate, oxalate, valerate, oleate, palmitate, stearate, laurate, borate, benzoate, lactate, phosphate, tosylate, citrate, maleate, fumarate, succinate, tartrate, naphthylate mesylate, glucoheptonate, lactobionate, and laurylsulphonate salts, and the like. These may include cations based on the alkali and alkaline earth metals, such as sodium, lithium, potassium, calcium, magnesium, and the like, as well as non-toxic ammonium, quaternary ammonium, and amine cations including, but not limited to ammonium, tetramethylammonium, tetraethylammonium, methylamine, dimethylamine, trimethylamine, triethylamine, ethylamine, and the like. (See, for example, Berge S.M. et al., "Pharmaceutical Salts," J. Pharm. Sci., 1977;66:1-19 which is incorporated herein by reference.)

Examples of pharmaceutically acceptable, non-toxic esters of the compounds of this invention include C₁-C₆ alkyl esters, wherein the alkyl group is a straight or branched, substituted or unsubstituted, C₅-C₇ cycloalkyl esters, as well as arylalkyl esters such as benzyl and triphenylmethyl. C₁-C₄ alkyl esters are preferred, such as methyl, ethyl, 2,2,2-trichloroethyl, and tert-butyl. Esters of the compounds of the present invention may be prepared according to conventional methods.

Examples of pharmaceutically acceptable, non-toxic amides of the compounds of this invention include amides derived from ammonia, primary C₁-C₆ alkyl amines and secondary C₁-C₆ dialkyl amines, wherein the alkyl groups are straight or branched. In the case of secondary amines, the amine may also be in the form of a 5- or 6-membered heterocycle containing one nitrogen atom. Amides derived from ammonia, C₁-C₃ alkyl primary amines and C₁-C₂ dialkyl secondary amines are preferred. Amides of the compounds of the invention may be prepared according to conventional methods.
The term "prodrug" refers to compounds that are rapidly transformed in vivo to yield the parent compound of the above formulae, for example, by hydrolysis in blood. A thorough discussion of prodrugs is provided in T. Higuchi and V. Stella, "Pro-drugs as Novel Delivery Systems," Vol. 14 of the A.C.S. Symposium Series, and in Bioreversible Carriers in Drug Design, ed. Edward B. Roche, American Pharmaceutical Association and Pergamon Press, 1987, both of which are hereby incorporated by reference.

These compounds can be administered individually or in combination, usually in the form of a pharmaceutical composition. Such compositions are prepared in a manner well known in the pharmaceutical art and comprise at least one active compound. Accordingly, a further aspect of the present invention includes pharmaceutical compositions comprising as one or more compounds of the invention disclosed above, associated with a pharmaceutically acceptable carrier. For administration, the compounds are ordinarily combined with one or more adjuvants appropriate for the indicated route of administration. The compounds may be admixed with lactose, sucrose, starch powder, cellulose esters of alkanoic acids, stearic acid, talc, magnesium stearate, magnesium oxide, sodium and calcium salts of phosphoric and sulfuric acids, acacia, gelatin, sodium alginate, polyvinylpyrrolidine, and/or polyvinyl alcohol, and tableted or encapsulated for conventional administration. Alternatively, the compounds of this invention may be dissolved in saline, water, polyethylene glycol, propylene glycol, carboxymethyl cellulose colloidal solutions, ethanol, corn oil, peanut oil, cottonseed oil, sesame oil, tragacanth gum, and/or various buffers. Other adjuvants and modes of administration are well known in the pharmaceutical art. The carrier or diluent may include time delay material, such as glyceryl monostearate or glyceryl distearate alone or with a wax, or other materials well known in the art.

In another aspect, the present invention provides methods for treating a subject with a tumor, comprising administering to the subject an amount effective of a compound according to formula I and formulas Ia-Io.

Non-limiting examples of specific tumor types that the compounds may be used to treat include, but are not limited to sarcomas, melanomas, neuroblastomas, carcinomas (including but not limited to lung, renal cell, ovarian, liver, bladder, and pancreatic carcinomas), and mesotheliomas

As used herein, the term "amount effective" means a dosage sufficient to produce a desired result. The desired result can be subjective or objective improvement in the recipient of the dosage; a decrease in tumor size, time to progression of disease, and/or survival; inhibiting an increase in tumor size; reducing or preventing metastases; and/or limiting or preventing recurrence of the tumor in a subject that has previously had a tumor.

In one embodiment, the methods of the invention can be used in combination with surgery on the subject, wherein surgery includes primary surgery for removing one or more tumors, secondary cytoreductive surgery, and palliative secondary surgery.

In a further embodiment, the methods of the invention further comprise treating the subject with chemotherapy and/or radiation therapy. One benefit of such a method if that use of the compounds permits a reduction in the chemotherapy and/or radiation dosage necessary to inhibit tumor growth and/or metastasis. As used herein, "radiotherapy" includes but is not limited to the use of radio-labeled compounds targeting tumor cells. Any reduction in chemotherapeutic or radiation dosage benefits the patient by resulting in fewer and decreased side effects relative to standard chemotherapy and/or radiation therapy treatment. In this embodiment, the one or more compounds may be administered prior to, at the time of, or shortly after a given round of treatment with chemotherapeutic and/or radiation therapy. In a preferred embodiment, the one or more compounds is administered prior to or simultaneously with a given round of chemotherapy and/or radiation therapy. In a most preferred embodiment, the one or more compounds is administered prior to or simultaneously with each round of chemotherapy and/or radiation therapy. The exact timing of compound administration will be determined by an attending physician based on a number of factors, but the compound is generally administered between 24 hours before a given round of chemotherapy and/or radiation therapy and simultaneously with a given round of chemotherapy and/or radiation therapy.

The methods of the invention are appropriate for use with chemotherapy using one or more cytotoxic agent (ie: chemotherapeutic), including, but not limited to, cyclophosphamide, taxol, 5-fluorouracil, adriamycin, cisplatinum, methotrexate, cytosine arabinoside, mitomycin C, prednisone, vindesine, carbaplatinum, and vincristine. The cytotoxic agent can also be an antiviral compound which is capable of destroying proliferating cells. For a general discussion of cytotoxic agents used in chemotherapy, see Sathe, M. et al., Cancer Chemotherapeutic Agents: Handbook of Clinical Data (1978), hereby incorporated by reference. When administered as a combination, the therapeutic agents can be formulated as separate compositions that are given at the same time or different times, or the therapeutic agents can be given as a single composition.

The methods of the invention are also particularly suitable for those patients in need of repeated or high doses of chemotherapy and/or radiation therapy.

The actual compound dosage range for administration is based on a variety of factors, including the age, weight, sex, medical condition of the individual, the severity of the condition, and the route of administration. Thus, the dosage regimen may vary widely, but can be determined by a physician using standard methods. An effective amount of the one or more compounds that can be employed ranges generally between 0.01 µg/kg body weight and 10 mg/kg body weight, preferably ranging between 0.05 µg/kg and 5 mg/kg body weight, more preferably between 1 µg /kg and 5 mg/kg body weight, and even more preferably between about 10 µg /kg and 5 mg/kg body weight.

The compounds may be made up in a solid form (including granules, powders or suppositories) or in a liquid form (e.g., solutions, suspensions, or emulsions). The compounds of the invention may be applied in a variety of solutions and may be subjected to conventional pharmaceutical operations such as sterilization and/or may contain conventional adjuvants, such as preservatives, stabilizers, wetting agents, emulsifiers, buffers etc. The compounds of the invention may be administered by any suitable route, including orally, parentally, by inhalation or rectally in dosage unit formulations containing conventional pharmaceutically acceptable carriers, adjuvants, and vehicles, including liposomes. The term parenteral as used herein includes, subcutaneous, intravenous, intraarterial, intramuscular, intrasternal, intratendinous, intraspinal, intracranial, intrathoracic, infusion techniques, intracavity, or intraperitoneally.

In yet further aspects, the invention provides an article of manufacture comprising packaging material and the above pharmaceutical compositions.

The instant invention may be embodied in other forms or carried out in other ways without departing from the spirit or essential characteristics thereof. The present disclosure and enumerated examples are therefore to be considered as in all respects illustrative and not restrictive, and all equivalency are intended to be embraced therein. One of ordinary skill in the art would be able to recognize equivalent embodiments of the instant invention, and be able to practice such embodiments using the teaching of the instant disclosure and only routine experimentation.

### Examples

### Example 1: Synthetic

### 4-Chloro-3-iodo-N-(6-methoxy-pyridin-3-yl)-benzamide (3a)

To a solution of 4-chloro-3-iodobenzoic acid (20g, 70.8mmol), EDC•HCl (15g 78.2mmol) and HOBt (11g, 78mmol) in 500mL of dry dichloromethane was added DIEA (27mL, 156mmol). The mixture was stirred for 15 minutes at which time 5-amino-2-methoxy pyridine (10g, 79mmol) was added. The reaction stirred at room temperature for 12 hours. The reaction was washed with 600mL of water. The DCM layer was separated, and the product precipitated out of solution. Pure product **3a** was obtained upon filtration and dried in a vacuum oven at 50°C to yield 21g (78%) of a white solid.

### 4-Bromo-6-chloro-biphenyl-3-carboxylic acid (6-methoxy-pyridine-3-yl)-amide (5a)

To a solution of 4-chloro-3-iodo-N-(6-methoxy-pyridin-3-yl)-benzamide (3a) (10g, 25.77mmol) and 4-bromophenylboronic acid (10.35g 51.5mmol) in 200mL dry dioxane was added Pd₂(PPh₃)₄ (5g, 15mol%) and potassium carbonate (8g, 58mmol). The solution was heated to 50°C for 12 hours, reaction completion was determined by TLC and LC/MS. The solution was cooled to room temperature and reduced to dryness. After chromatography eluting with (4-1) to (2-1) heptanes/ethyl acetate pure product as a white solid **(5a),** 7.3g (70%) was obtained.

### 6-Chloro-4-pyridine-2-yl-biphenyl-3-carboxylic acid (6-methoxy-pyridin-3-yl)-amide (7a)

To a solution of 4-bromo-6-chloro-biphenyl-3-carboxylic acid (6-methoxy-pyridine-3-yl)-amide (7.3g 17.5mmol) and 2-tributylstannylpyridine (7g, 19mmol) in 100mL dry dioxane was added Pd₂(PPh₃)₄ (5g, 20mol%). The solution was heated to 80°C for 12 hours, the reaction was monitored by TLC and LC/MS; After 6 hours, another equivalent of 2-tributylstannylpyridine and ~10mol% Pd₂(PPh₃)₄ were added. Upon completion, the reaction was cooled to room temperature and reduced to a dark solid. The product was purified via chromatography eluting with (4-1) to (2-1) heptanes/ethyl acetate to afford target 7a (4g, 60%) as a white solid.
¹H NMR (400 MHz, DMSO-*d₆*) ζ ppm 3.82 (s, 3 H) 6.82 (d, *J=*8.98 Hz, 1 H) 7.33 - 7.40 (m, 1 H) 7.63 (d, *J* =8.40 Hz, 2 H) 7.74 (d, *J* =8.40 Hz, 1 H) 7.83 - 7.92 (m, 2 H) 7.95 - 8.05 (m, 3 H) 8.08 (d, *J=*2.15 Hz, 1 H) 8.21 (d, *J*=8.59 Hz, 2 H) 8.50 (d, *J=*2.54 Hz, 1 H) 8.66 - 8.71 (m, 1 H) 10.38 (s, 1 H).

### Example 2

### Initial discovery

Bi-phenyl compounds according to the present invention were initially identified based on potential anti-microbial activity. A representative compound was subsequently tested and found to possess gyrase-inhibitory activity. Such activity suggested that the compound might also possess topoisomerase inhibitory activity.

In order to verify whether these compounds might have anti-topo activity, CP5017808 inhibition of topoisomerase II was assayed by monitoring the appearance of 2.5 kilobase DNA from eukaryotic topoII decatenation of kinetoplast DNA via agarose gel electrophoresis. All reagents were purchased from Topogen, Inc, Port Orange, FL, unless otherwise noted. Reactions contained 1 uL of titrated CP5017808 in DMSO, 0.1 ug of kinetoplast DNA, 2 units of eukaryotic topoisomerase II, and IX topoII reaction buffer (50mM Tris-HCl pH 8, 120mM KCl, 10mM MgCl2, 0.5mM each of dithiothreitol, ATP and 30ug BSA/mL), in a final volume of 25uL. Reactions were incubated in a 37° C water bath for 15 minutes and terminated with 5uL of stop buffer (5% sarkosyl, 0.025% bromophenol blue, 50% glycerol). 25uL of the reaction products were analyzed on a 1% agarose gel. Electrophoresis analysis of the reaction products were performed using standard agarose gel electrophoresis units (Biorad, Hercules, CA). Gels were run at 100 volts, allowing the bromophenol blue dye front to migrate 75% down the gel. Following electrophoresis, gels were stained with 0.5ug ethidium bromide/mL for 30 minutes and then photographed using a Gel Doc 2000 imaging system (Biorad, Hercules, CA). The resulting data confirmed that CP5017808 possessed anti-topoisomerase activity, suggesting it and other compounds according to the present invention may be cytotoxic to tumor cells, and thus useful for treating tumors. Further studies demonstrated that CP5017808 inhibits microtubule polymerization, further suggesting its potential use as an anti-tumor agent.

### Example 3: Anti-tumor screening

Raji cells (lymphoblastoid) or MCF-7 (breast carcinoma) cells were seeded at 5,000 to 10,000 cells per will, incubated for 3 days at 37°C with compounds according to the invention. Alamar Blue (Accumed International, Westlake OH) was then added to the cells at 1/10 the volume of the well, and the cells were further incubated at 37°C for various times. Alamar Blue dye measures cellular re-dox reactions (ie: cellular mitochrondrial respiration) whereby a spectral shift occurs upon reduction of the dye. (Excitation 530 nm; emission 590 nm). IC50 determinations were made from these readings.

Subsequently, a large number of candidate compounds were analyzed for anti-tumor activity in a cell line assay versus NCI-H460 (lung carcinoma cells).

For screening of anti-tumor activity versus primary human sarcoma tumor cells, excess tissue specimens obtained from freshly at the time of surgery were sent for pathological testing. For diagnosis and grading of tissue samples (ie: prior to processing), hematoxylin and eosin stained tissue sections were examined by a pathologist. If the diagnosis and grading of the tissue concurred with the determination made by the surgical pathologist that provided the tissue, then the tissue was used in the screen. If there was no agreement, then two additional pathologists served as referees. If no consensus was reached, then the tissue was discarded. The remaining tissue was used to prepare cell suspensions. The tissue was initially treated enzymatically via standard methods until only undigested material remained. The digested cell suspension was filtered through one or more screens of between 40 micron and 100 micron porosity. The resulting cell suspension was further purified via isokinetic density centrifugation.

Additional normal cells were removed from the cell suspension by negative immunoselection with a combination of monoclonal antibodies linked to magnetic beads (Dynal) that were used according to the manufacturers' instructions. The remaining cells were placed into appropriate medium, frozen down in 1.0 mL aliquots, and stored until use.

After tissue processing, the relative purity of the resulting cell suspension was determined by cytological examination after pap staining. Only those cell preparations greater than 80% tumor cells were used for testing of candidate compounds. If there was any doubt about the percentage of tumor cells in the cell preparation, additional pathologists served as referees to make a determination.

Cell preparations that passed histological and cytological examination for diagnosis, grading, and cell purity were thawed at 37°C and resuspended in tissue culture medium designed to maintain the cells during the incubation period. The live and dead cells were counted and the cells were diluted in culture medium to 1.0 x 10³ live tumor cells/test well.

The cells were added to microtiter plates and incubated at 37°C overnight with 10 µM of the candidate compounds that were added at 1/10th the volume of the cell suspension. Alamar Blue (Accumed International, Westlake OH) was then added to the cells at 1/10 the volume of the well, and the cells were further incubated at 37°C for various times. Alamar Blue dye measures cellular re-dox reactions (ie: cellular respiration) whereby a spectral shift occurs upon reduction of the dye. (Excitation 530 nm; emission 590 nm)

The kinetics of cellular re-dox reactions were subsequently measured at various times, for example at 3 hours, 3 days, and 5 days post-dye addition. These measurements, in comparison with control cells (untreated with compound) and media controls (test wells without cells) provide the IC₅₀ determinations.

Compounds according to the present invention with activity against at least one tumor type tested are presented in Figure 1. IC₅₀ values are reported for the designated tumor type, according to the methods disclosed above. The IC₅₀ values are in micromolar concentrations and the acronyms used in the Tables are as follows:
T = Tumor
Cytotoxic: The compound showed activity at one, or more concentrations, but an IC50 was not determined; these compounds are considered "active."

These data clearly show that the compounds of the invention can be used as an anti-tumor agent against a variety of tumor types.

### Example 4

The *in vivo* anti-tumor activity of CP5017808 was evaluated against A375 human melanoma xenografts and ovarian carcinoma. The data presented below show that CP5017808 possesses significant anticancer activity -

CP5017808 was dissolved with heat and sonication in a 1:1 mixture of absolute ethanol and CREMAPHOR™ equivalent to 20% of the final desired volume, and then brought to a final volume with 40% hydroxypropyl-β-cyclodextin in water. CP5017808 produced a fine suspension with a pH of 6.2.

Female NCr nu/nu mice (Charles River Laboratory) were used for the study.They were 5-6 weeks old on Day 1 of the experiment. The animals were fed Rodent Diet 5010 (LabDiet™) and water *ad libitum.* The mice were housed in Thoren™ microisolator caging with Bed-O'Cobs™ bedding. All treatments, body weight determinations, and tumor measurements were carried out in laminar down flow cabinets. The environment was controlled to a temperature range of 70-78°F and a humidity range of 30-70%. Test animals were implanted subcutaneously on day 0 with 30 to 60mg tumor fragments using a 12-gauge trocar needle. All animals were observed for clinical signs at least once daily. Animals with tumors in excess of 1g or with ulcerated tumors were euthanized, as were those found in obvious distress or in a moribund condition.

Treatments began when the tumors were approximately 100 mg. In the present study, treatment began on Day 12 when the mean estimated tumor mass for all groups in the experiment was 116 mg (range, 111-121mg). All animals weighed >18g at the initiation of therapy. Mean group body weights at first treatment were well-matched (range, 21-22.5ag). All animals were dosed according to individual body weight on the day of treatment (0.2 ml/20g).

Body weights and tumor measurements were recorded three times weekly. Tumor burden (mg) was estimated from caliper measurements by the formula for the volume of a prolate ellipsoid assuming unit density as: Tumor burden (mg) = (L x W2)/2, where L and W are the respective orthogonal tumor length and width measurements (mm). The primary endpoints used to evaluate efficacy were study day 26 T/C values, complete and partial tumor responses, tumor growth delay, and the number of tumor-free survivors at the end of the study. T/C values were calculated by dividing the treated median tumor mass by the median control tumor mass and multiplying by 100. The USA National Cancer Institute (NCI) considers a compound which produces a %T/C value of 42 of less is active. Complete response (CR) is defined as a decrease in tumor mass to an undetectable size (<50mg), and a partial response (PR) is defined as a ≥ 50% decrease in tumor mass from that at first treatment. PRs are exclusive of CRs, as are Tumor-Free Survivors (TFS). Tumor Growth Delay (T-C) was also used to quantify efficacy. Tumor growth delay for this experiment was expressed as a T-C value, where T and C are the median times in days required for the treatment and control group tumors, respectively, to grow to a selected evaluation size, 750mg.

All animals were observed for clinical signs at least once daily. Animals were weighed on each day of treatment and 3x/week thereafter. Treatment related weight loss in excess of 15% is generally considered unacceptably toxic. As used in this example, a dose is described as tolerated if treatment related weight loss (during and two weeks after treatment) was <15% and lethality during this period in the absence of potentially lethal tumor burdens was <10%. Upon death or euthanasia, all animals were necropsied to provide a general assessment of potential cause of death and perhaps target organs for toxicity. The presence or absence of metastases was also noted.

The time for individual tumors to reach evaluation size, time to fold growth, and tumor volume doubling times were analyzed by a single factor ANOVA. Frankly, toxic dose levels were excluded from this analysis. If the ANOVA analysis indicated no significant differences, no additional analyses were performed. If the ANOVA analysis indicated significant differences between the study groups, the data ware then analyzed to determine if the data within each treatment group were drawn from a normal distribution using the Kolmogrov-Smimov test for normality. Finally, a least significant difference analysis was applied to determine exactly which treatment groups were significantly different from the control group. The "within mean sum of squares" term and degrees of freedom from the single factor ANOVA were used as the common variance and degrees of freedom, respectively, in this analysis: Statistical significance was determined using built-in Microsoft Excel data analysis tools and R (R Project for Statistical Computing).

### Results

### Tumor Growth/General Observations/Controls

The mean estimated tumor burden for all groups in the experiment on the first day of treatment was 116mg and all of the groups in the experiment were well-matched (range, 111-121mg). All animals weighed >18 grams at the initiation of therapy. Mean group body weights at first treatment were also well-matched (range, 21-22.5g). 750mg was chosen as the evaluation size for the experiment. The median control tumor reached evaluation size on Day 25, and the tumor volume doubling time for the control group was 4.6 days. Control animals experienced 0.5g mean weight loss during the treatment regimen. There were no spontaneous regressions in the control group. Based on historical data for this model, the biology of the control group was judged to be within the normal range. The median mouse in the control group surpassed a mass of 1-gram size on study day 26, the day used for %T/C calculations. Treatment with 30mg/kg of docetaxel, the positive control compound for this experiment, produced a %T/C value of 32% on study day 26 and produced a 10 day tumor growth delay.. This was consistent with expected activity.

CP5017808 was toxic at 100mg/kg; producing 100% treatment related deaths. Six out of the 8 mice were found dead within four treatments, with common necropsy findings of mottled liver and red intestines. The remaining two mice died several days later with similar necropsy observations. CP5017808 was tolerated at 60 and 36mg/kg, producing 13 and 0% treatment related deaths, respectively. The 60mg/kg group experienced one death, which occurred on study day 27. This mouse was too decomposed to necropsy. Minimal weight loss, 6 and 3.8%, respectively was observed in these dosage groups, both maximal on study day 15. The weight loss induced by the 60mg/kg treatment was recovered within 13 days and the weight loss related to the 36mg/kg treatment was recovered within 3.6 days. All animals were necropsied as they left the study due to tumor burden with no remarkable findings to report.

### Efficacy

CP5017808 was significantly active at 60mg/kg producing a %T/C value of 34% on study day 26, which is well below the NCl-designated threshold for meaningful anti-tumor activity of 42% (ie: 42% or lower represents meaningful activity). This treatment produced a tumor growth delay of 6.1 days. CP7808 was less active at 36mg/kg producing a %T/C value of 66%, and a tumor growth delay of 4.7 days. Neither value was statistically significant compared to the control group. Neither dose level produced complete or partial regressions or tumor free survivors.

### Example 5

Under experimental conditions similar to those in Example 4, CP5017808 was active at 60mg/kg against A2780 human ovarian adenocarcinoma xenografts. A positive control (Taxol®) was included in the experiment to ensure the responsiveness of the tumor model to taxanes.

Test animals were implanted subcutaneously on day 0 with 30 to 60mg A2780 tumor fragments. The primary endpoints used to evaluate efficacy were day 19 T/C values, complete and partial tumor responses, and the number of tumor-free survivors at the end of the study. Tumor mass on day 19 was analyzed by a single factor ANOVA.

The mean estimated tumor burden for all groups in the experiment on the first day of treatment was 100mg and all of the groups in the experiment were well-matched (range 90-108mg). All animals weighed >16.9 grams at the initiation of therapy. Mean group body weights at first treatment were also well-matched (range, 19.6-22.1g). 750mg was chosen as the evaluation size for the experiment. The median control tumor reached evaluation size on Day 17, and the tumor volume doubling time for the control group was 2.4 days (Table 1). Vehicle only (negative control) treated animals experienced a 1.1g mean weight loss during the treatment regimen. There were no spontaneous regressions in the control group. Based on historical data for this model, the biology of the control group was judged to be within the normal range. The median mouse in the control group surpassed a mass of 1-gram size on Day 19, which was used for all %T/C calculations. The positive control compound for this experiment, Taxol® at 15mg/kg, produced a %T/C value of 15 on the last day of the study (Day 19). This is consistent with expected activity.

CP5017808 was tolerated at all dose levels (60, 36, and 21.6mg/kg) tested. Treatment at the highest dose tested, 60mg/kg, produced an 11.2% weight loss and one death (17%). Although 17% mortality is usually considered to reflect unacceptable toxicity, the unusual behavior of this animal compared to its group mates, prompted the study leader to include this dose level in the analyses for anticancer activity. CP5017808 at 36 and 21.6mg/kg produced 16.7% and 8.3% weight losses, respectively and no treatment-related deaths. All of the animals in these dose groups had large red livers and full gall bladders at necropsy on day 20. Other organs appeared normal and no drug deposits were present in the peritoneal cavity.

CP5017808 was active at the highest dose tested, 60mg/kg, producing a day 19 T/C value of 29%. Treatment at 36 and 21.6mg/kg was ineffective producing day 19 T/C values of 81 and 94% respectively. There were no complete or partial regressions and no tumor free survivors at any dose level.

## Claims

1. A compound of the formula: 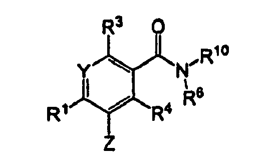 or a pharmaceutically acceptable salt, ester or amide thereof, wherein
Y is C-R² or N;
R¹ is selected from hydrogen, C₁-C₁₂ alkyl or halo;
R², R³ and R⁴ are independently selected from hydrogen or halo;
R⁶ is hydrogen, or
R⁶ is C₁-C₁₂ alkyl optionally substituted with one or two groups selected from amino, monoalkylamino or dialkylamino;
R¹⁰ is selected from aryl, heteroaryl, heteroarylalkyl, cycloalkyl, heterocycloalkyl or heterocycloalkylalkyl, each of which is optionally substituted at the ring portion and/or alkyl portion with one to five groups selected from C₁-C₁₂ alkyl, C₂-C₁₂ alkenyl, C₂-C₁₂ alkynyl, trifluoromethyl, C₁-C₁₂ alkoxy, hydroxyl, halo, amino, monoalkylamino, dialkylamino, aminoalkyl, monoalkylaminoalkyl, dialkylaminoalkyl, nitro, -CN, oxo, -C(O)OR, -NH-C(O)-R', -C(O)- NHR', -SR, -SO₂R, -CO₂R, or aryl, heteroaryl, cycloalkyl or heterocycloalkyl, each of which is optionally substituted with one to three groups selected from C₁-C₁₂ alkyl, C₂-C₁₂ alkenyl, C₂-C₁₂ alkynyl, trifluoromethyl, C₁-C₁₂ alkoxy, hydroxyl, halo, amino, monoalkylamino, dialkylamino, aminoalkyl, monoalkylaminoalkyl, dialkylaminoalkyl, nitro, -CN, oxo, -C(O)OR, -NH-C(O)-R', -C(O)-NHR', -SR, -SO₂R, -CO₂R, or
R⁶ and R¹⁰ together with the nitrogen atom to which they are attached form a 5-7 membered heteroaryl ring optionally substituted with one to three groups independently selected from C₁-C₁₂ alkyl, C₂-C₁₂ alkenyl, C₂-C₁₂ alkynyl, trifluoromethyl, C₁-C₁₂ alkoxy, hydroxyl, halo, amino, monoalkylamino, dialkylamino, aminoalkyl, monoalkylaminoalkyl, dialkylaminoalkyl, nitro, -CN, oxo, -C(O)OR, - NH-C(O)-R', -C(O)-NHR', -SR, -SO₂R, -CO₂R;
Z is selected from 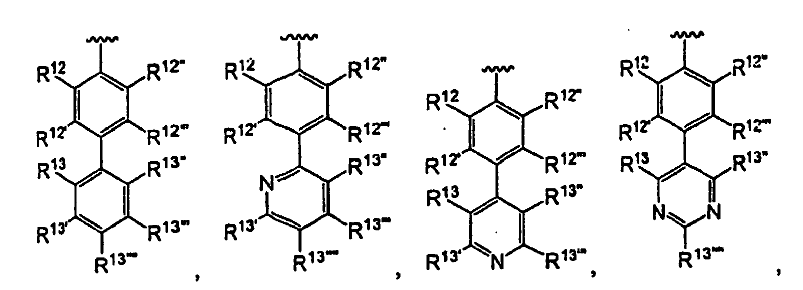 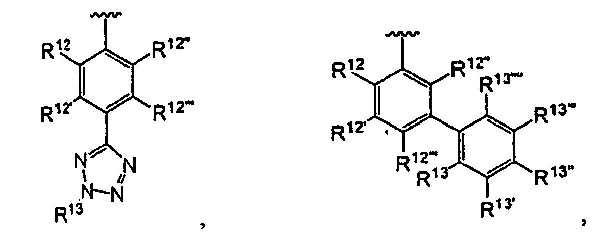 , or 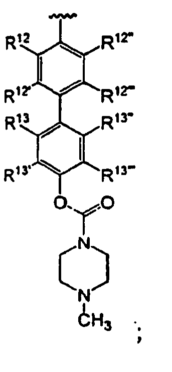
R¹², R^{12'}, R^{12"} and R^{12'''} are independently selected from hydrogen, C₁-C₁₂ alkyl, C₂-C₁₂ alkenyl, C₂-C₁₂ alkynyl, trifluoromethyl, C₁-C₁₂ alkoxy, hydroxyl, halo, amino, monoalkylamino, dialkylamino, aminoalkyl, monoalkylaminoalkyl, dialkylaminoalkyl, nitro, -CN, -C(O)OR, -NH- C(O)-R', -C(O)-NHR', -SR, -SO₂R, -CO₂R;
R¹³, R^{13'}, R^{13"}, R^{13"'} and R^{13''''} are independently selected from hydrogen, C₁-C₁₂ alkyl, C₂-C₁₂ alkenyl, C₂-C₁₂ alkynyl, trifluoromethyl, C₁-C₁₂ alkoxy, hydroxyl, halo, amino, monoalkylamino, dialkylamino, aminoalkyl, monoalkylaminoalkyl, dialkylaminoalkyl, nitro, -CN, - C(O)OR, -NH-C(O)-R', -C(O)-NHR', -SR, -SO₂R, -CO₂R;
X is halo;
R is selected from hydrogen and C₁-C₁₂ alkyl; and
R' is independently selected from hydrogen, C₁-C₁₂ alkyl, amino, monoalkylamino, dialkylamino, arylalkyl, heteroaryl, heteroarylalkyl, cycloalkyl, cycloalkylalkyl, heterocycloalkyl or heterocycloalkylalkyl.

2. A compound according to claim 1 having the formula:

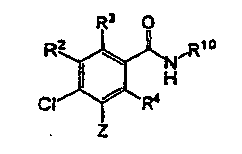

3. A compound according to claim 2 wherein
R², R³ and R⁴ are hydrogen.

4. A compound according to claim 1 having the formula

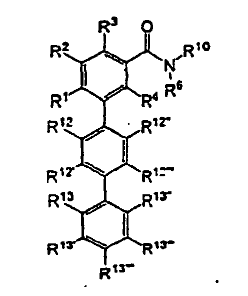

5. A compound according to claim 4 wherein
R¹ is hydrogen, chloro or methyl, and
R², R³ and R⁴ are hydrogen.

6. A compound according to claim 4 wherein
R^{12'} is hydrogen or halo, and
R¹², R^{12'} and R^{12'''} are hydrogen.

7. A compound according to claim 4 wherein
R¹³, R^{13'} and R^{13"} are hydrogen.

8. A compound according to claim 1 having the formula:

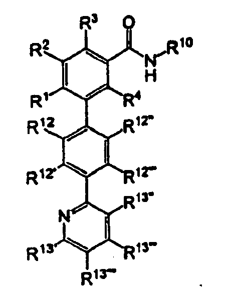

9. A compound according to claim 8 wherein R¹ is chloro, and R², R³ and R⁴ are hydrogen.

10. A compound according to claim 8 wherein R¹², R^{12'}, R^{12"} and ^{R12'''} are hydrogen.

11. A compound according to claim 8 wherein R^{13'}, R^{13"}, R^{13'''} and R^{13''''} are hydrogen.

12. A compound according to claim 1 having the formula:

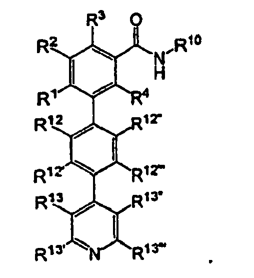

13. A compound according to claim 12 wherein R¹ is chloro, and R², R³ and R⁴ are hydrogen.

14. A compound according to claim 12 wherein R¹², R^{12'}, R^{12"} and R^{12'"} are hydrogen.

15. A compound according to claim 12 wherein R13, R^{13'}, R^{13"} and R^{13'''} are hydrogen.

16. A compound according to claim 1 having the formula:

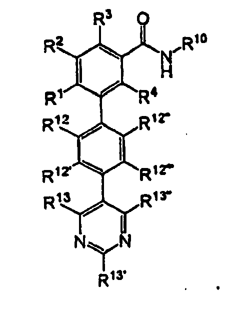

17. A compound according to claim 16 wherein R¹ is chloro, and R², R³ and R⁴ are hydrogen.

18. A compound according to claim 16 wherein R¹², R^{12'}, R^{12"} and R^{12'''} are hydrogen.

19. A compound according to claim 16 wherein R¹³, R^{13'} and R^{13"} are hydrogen.

20. A compound according to claim 1 having the formula:

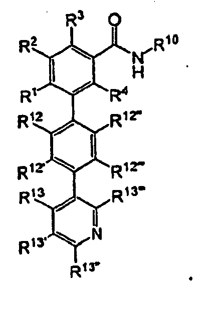

21. A compound according to claim 20 wherein R¹ is chloro, and R², R³ and R⁴ are hydrogen.

22. A compound according to claim 20 wherein R¹², R^{12'}, R^{12"} and R^{12"'} are hydrogen.

23. A compound according to claim 20 wherein R¹³, R^{13"} and R^{13'"} are hydrogen, and R^{13'} is selected from hydrogen, aminoalkyl, monoalkylaminoalkyl, dialkyaminoalkyl or C₁-C₁₂ alkoxy.

24. A compound according to claim 1 having the formula: 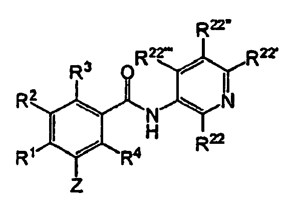 wherein
R²², R^{22'}, R^{22"} and R^{22"'} are independently selected from hydrogen, C₁-C₁₂ alkyl, C₂-C₁₂ alkenyl, C₂-C₁₂ alkynyl, trifluoromethyl, C₁-C₁₂ alkoxy, hydroxyl, halo, amino, monoalkylamino, dialkylamino, aminoalkyl, monoalkylaminoalkyl, dialkylaminoalkyl, nitro, -CN, oxo, -C(O)OR, -NH-C(O)-R', -C(O)-NHR', -SR, -SO₂R, -CO₂R; or aryl, heteroaryl, cycloalkyl or heterocycloalkyl, each of which is optionally substituted with one to three groups selected from C₁-C₁₂ alkyl, C₂-C₁₂ alkenyl, C₂-C₁₂ alkynyl, trifluoromethyl, C₁-C₁₂ alkoxy, hydroxyl, halo, amino, monoalkylamino, dialkylamino, aminoalkyl, monoalkylaminoalkyl, dialkylaminoalkyl, nitro, -CN, oxo, -C(O)OR, -NH-C(O)-R', -C(O)-NHR', -SR, -SO₂R, -CO₂R.

25. A compound according to claim 24 wherein R¹ is chloro, and R², R³ and R⁴ are hydrogen.

26. A compound according to claim 24 wherein R²², R^{22'}, R^{22"} and R^{22'"} are selected from hydrogen, halo, C₁-C₁₂ alkyl, C₁-C₁₂ alkoxy or optionally substituted heterocyclyl.

27. A compound according to claim 1 having the formula: 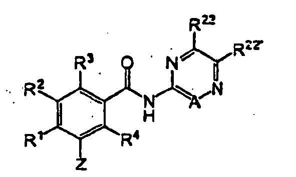 wherein
A is -CH or N; and
R²² and R^{22'} are independently selected from hydrogen, C₁-C₁₂ alkyl, C₂-C₁₂ alkenyl, C₂-C₁₂ alkynyl, trifluoromethyl, C₁-C₁₂ alkoxy, hydroxyl, halo, amino, monoalkylamino, dialkylamino, aminoalkyl, monoalkylaminoalkyl, dialkylaminoalkyl, nitro, -CN, oxo, -C(O)OR, -NH-C(O)-R', -C(O)-NHR', -SR, -SO₂R, -CO₂R; or aryl, heteroaryl, cycloalkyl or heterocycloalkyl, each of which is optionally substituted with one to three groups selected from C₁-C₁₂ alkyl, C₂-C₁₂ alkenyl, C₂-C₁₂ alkynyl, trifluoromethyl, C₁-C₁₂ alkoxy, hydroxyl, halo, amino, monoalkylamino, dialkylamino, aminoalkyl, monoalkylaminoalkyl, dialkylaminoalkyl, nitro, -CN, oxo, -C(O)OR, -NH-C(O)-R', -C(O)-NHR', -SR, -SO₂R, -CO₂R.

28. A compound according to claim 27 wherein R¹ is chloro, and R², R³ and R⁴ are hydrogen.

29. A compound according to claim 27 wherein R²² and R^{22'} are selected from hydrogen, halo, C₁-C₁₂ alkyl and C₁-C₁₂ alkoxy.

30. A compound according to claim 1 having the formula: 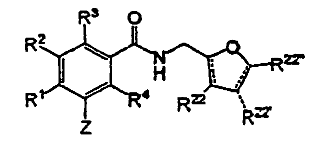 wherein the ---- bonds are both present or both absent, and R¹, R², R³, R⁴ and Z are as defined in claim 1, and
R²², R^{22'} and R^{22"} are independently selected from hydrogen, C₁-C₁₂ alkyl, C₂-C₁₂ alkenyl, C₂-C₁₂ alkynyl, trifluoromethyl, C₁-C₁₂ alkoxy, hydroxyl, halo, amino, monoalkylamino, dialkylamino, aminoalkyl, monoalkylaminoalkyl, dialkylaminoalkyl, nitro, -CN, oxo, -C(O)OR, - NH-C(O)-R', -C(O)-NHR', -SR, -SO₂R, -CO₂R; or aryl, heteroaryl, cycloalkyl or heterocycloalkyl, each of which is optionally substituted with one to three groups selected from C₁-C₁₂ alkyl, C₂- C₁₂ alkenyl, C₂-C₁₂ alkynyl, trifluoromethyl, C₁-C₁₂ alkoxy, hydroxyl, halo, amino, monoalkylamino, dialkylamino, aminoalkyl, monoalkylaminoalkyl, dialkylaminoalkyl, nitro, -CN, oxo, -C(O)OR, - NH-C(O)-R', -C(O)-NHR', -SR, -SO₂R, -CO₂R.

31. A compound according to claim 30 wherein R¹ is chloro, and R², R³ and R⁴ are hydrogen.

32. A compound according to claim 30 wherein R²² and R^{22'} are selected from hydrogen, halo, C₁-C₁₂ alkyl and C₁-C₁₂ alkoxy.

33. A compound according to claim 1 having the formula: 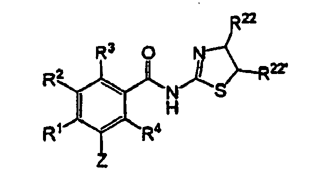 wherein
A is -CH or N; and
R²² and R^{22'} are independently selected from hydrogen, C₁-C₁₂ alkyl, C₂-C₁₂ alkenyl, C₂-C₁₂ alkynyl, trifluoromethyl, CC₁-C₁₂ alkoxy, hydroxyl, halo, amino, monoalkylamino, dialkylamino, aminoalkyl, monoalkylaminoalkyl, dialkylaminoalkyl, nitro, -CN, oxo, -C(O)OR, - NH-C(O)-R', -C(O)-NHR', -SR, -SO₂R, -CO₂R; or aryl, heteroaryl, cycloalkyl or heterocycloalkyl, each of which is optionally substituted with one to three groups selected from C₁-C₁₂ alkyl, C₂- C₁₂ alkenyl, C₁-C₁₂ alkynyl, trifluoromethyl, C₁-C₁₂ alkoxy, hydroxyl, halo, amino, monoalkylamino, dialkylamino, aminoalkyl, monoalkylaminoalkyl, dialkylaminoalkyl, nitro, -CN, oxo, -C(O)OR, -NH-C(O)-R', -C(O)-NHR', -SR, -SO₂R, -CO₂R.

34. A compound according to claim 33 wherein R¹ is chloro, and R², R³ and R⁴ are hydrogen.

35. A compound according to claim 33 wherein R²² and R^{22'} are selected from hydrogen, halo, C₁-C₁₂ alkyl and C₁-C₁₂ alkoxy.

36. A compound according to claim 1 having the formula: 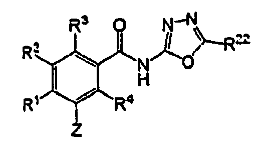 wherein
A is -CH or N; and
R²² is as independently selected from hydrogen, C₁-C₁₂ alkyl, C₂-C₁₂ alkenyl, C₂-C₁₂ alkynyl, trifluoromethyl, C₁-C₁₂ alkoxy, hydroxyl, halo, amino, monoalkylamino, dialkylamino, aminoalkyl, monoalkylaminoalkyl, dialkylaminoalkyl, nitro, -CN, oxo, -C(O)OR, - NH-C(O)-R', -C(O)-NHR', -SR, -SO₂R, -CO₂R; or aryl, heteroaryl, cycloalkyl or heterocycloalkyl, each of which is optionally substituted with one to three groups selected from C₁-C₁₂ alkyl, C₂- C₁₂ alkenyl, C₂-C₁₂ alkynyl, trifluoromethyl, C₁-C₁₂ alkoxy, hydroxyl, hallo, amino, monoalkylamino, dialkylamino, aminoalkyl, monoalkylaminoalkyl, dialkylaminoalkyl, nitro, -CN, oxo, -C(O)OR, - NH-C(O)-R', -C(O)-NHR', -SR, -SO₂R, -CO₂R.

37. A compound according to claim 36 wherein R¹ is chloro, and R², R³ and R4 are hydrogen.

38. A compound according to claim 36 wherein R²² and R^{22'} are selected from hydrogen, halo, C₁-C₁₂ alkyl, C₁-C₁₂ alkoxy and optionally substituted aryl.

39. A compound according to claim 1 selected from:
6-chloro-*N-*(tetrahydrofuran-2-ylmethyl)-1,1':4',1"-terphenyl-3-carboxamide;
6-chloro-*N-*(2-furylmethyl)-1,1':4',1"-terphenyl-3-carboxamide;
6-chloro-*N-*(6-chloropyridk-3-yl)-1,1':4',1"-terphenyl-3-carboxanide;
6-chloro-*N-*(4,5-dihydro-1,3-thiazol-2-yl)-1,1':4',1"-terphenyl-3. carboxamide;
6-chloro-*N*-(2-pyrrolidin-1-ylethyl)-1,1':4',1"-terphenyl-3-carboxamide;
6-chloro-*N-*(5-chloropyrimidm-2-yl)-1,1':4',1"-terphenyl-3-carboxamide;
6-chloro-*N-*(3-methylisoxazol-5-yl)-1,1':4',1".terphenyl-3-carboxamide;
6-chloro-*N-*[6-(methylsulfonyl)-1,3-benzothiazol-2-yl]-1,1':4',1"-terphenyl-3-carboxamide;
6-chloro-*N-*1,2,4-triazin-3-yl-1,1':4',1"-terphenyl-3-carboxamide;
methyl 3-{[(6-chloro-1,1':4',1"-terphenyl-3-yl)carbonyl]amino}benzoate;
6-chloro-*N-*(5-phenyl-1,3,4-oxadiazol-2-yl)-1,1':4',1"-terphenyl-3-carboxiamide;
6-chloro-*N-*{3-(2-methyl-1,3-dioxolan-2-yl)phenyl]-1,1':4',1-terphenyl-3-carboxamide;
6-chloro-*N-*(4-methoxyphenyl)-1,1':4',1"-terphenyl-3-carboxamide;
6-chloro-*N-*quinolin-6-yl-1,1':4',1"-terphenyl-3-carboxamide;
6-chloro-*N-*pyrazin-2-yl-1,1':4',1"-terphenyl-3-carboxamide;
*N-*(5-bromopyridin-2-yl)-6-chloro-1,1':4',1"-terphenyl-3-carboxamide;
6-chloro-*N-*(6-methoxypyridm-3-yl)-1,1':4',1"-terphenyl-3-carboxamide;
6-chloro-*N-*(6-fluoropyridin-3-yl)-1,1':4',1"-terphenyl-3-carboxamide;
6-chloro-*N-*(5-chloropyridin-2-yl)-1,1':4',1"-terphenyl-3-carboxamide;
6-chloro-*N-*(6-methoxypyridin-3-yl)-4'-pyridin-2-ylbiphenyl-3-carboxamide;
6-chloro-3'-fluoro-*N-*(6-methoxypyridin-3-yl)-1,1':4',1"-terphenyl-3-carboxamide;
6-chloro-*N-*(6-methoxypyridin-3-yl)-4'-pyridin-4-ylbiphenyl-3-carboxamide;
6-chloro-*N-*(4-methylphenyl)-1,1':4',1"-terphenyl-3-carboxamide;
6-chloro-*N-*(3-fluoro-4-methoxyphenyl)-1,1':4',1"-terphenyl-3-carboxamide;
6-chloro-*N-*(6-methoxypyridin-3-yl)-4'-pyrimidin-5-ylbiphenyl-3-carboxamide;
6-chloro-N-[2-(dimethylamino)ethyll-*N-*pyridin-3-yl-1,1':4',1"-terphenyl-3-carboxamide;
*N-*(2-pyrrolidin-1-ylethyl)-1,1':4',1"-terphenyl-3-carboxamide;
6-chloro-*N-*[6-(4-methylpiperazin-1-yl)pyridin-3-yl]-1,1':4',1"-terphenyl-3-carboxamide;
6-chloro-*N-*(6-morpholin-4-ylpyridin-3-yl)-1,1':4',1"-terphenyt-3-carboxamide;
4-chloro-*N-*(6-methoxypyridin-3-yl)-3-(6-phenylpyridin-3-yl)benzamide;
6-chloro-*N-*[6-(methylthio)pyridin-3-yl]-1,1':4',1"-terphenyl-3-carboxamide;
6-chloro-*N-*(5-methoxypyrazin-2-yl)-1,1':4',1"-terphenyl-3-carboxamide;
6-chloro-*N-*[2-(methylthio)pyrimidin-5-yl]-1,1':4',1"-terphenyl-3-carboxamide ;
6-chloro-*N-*(2-methoxypyrimidin-5-yl)-1,1':4',1"-terphenyl-3-carboxamide;
6-chloro-*N-*(6-methylpyridin-3-yl)-1,1':4',1"-terphenyl-3-carboxamide;
6-chloro-*N-*(6-oxo-1,6-dihydropyridin-3-yl)-4'-pyridin-2-ylbiphenyl-3-carboxamide;
6-chloro-*N-*(6-methoxypyridin-3-yl)-4'-(2-methyl-2*H-*tetrazol-5-yl)biphenyl-3-carboxamide;
6-chloro-*N-*(6-methoxypyridin-3-yl)-1,1':3',1"-terphenyl-3-carboxamide;
*N-*(6-methoxypyridin-3-yl)-6-methyl-1,1':4',1"-terphenyl-3-carboxaimde;
5-biphenyl-4-yl-6-chloro-*N-*(6.methoxypyridin-3-yl)nicotinamide;
6-chloro-*N-*(6-methoxypyridin-3-yl)-4'-pyridin-2-ylbiphenyl-3-carboxamide;
6-chloro-3"-formyl-*N-*(6-methoxypyridin-3-yl)-1,1':4',1"-terphenyl-3-carboxamide;
6-chloro-3"-[(dimethylamino)methyl]-*N*-(6-methoxypyridin-3-yl)-1,1':4',1"-terphenyl-3-carboxamide;
6-chloro-3"-(hydroxymethyl)-*N-*(6-methoxypyridin-3-yl)-1,1':4',1"-terphenyl-3-carboxamide;
6-chloro-4"-[(dimethylamino)methyl]-*N*-(6-methoxypyridin-3-yl)-1,1':4',1"-terphenyl-3-carboxamide;
6-chloro-4"-(hydroxymethyl)-*N-*(6-methoxypyridin-3-yl)-1,1':4',1"-terphenyl-3-carboxamide;
6-chloro-*N-*(6-methoxypyridin-3-yl)-3"-(methylsulfonyl)-1,1':4',1"-terphenyl-3-carboxamide;
6-chloro-*N-*(6-methoxypyridin-3-yl)-4'-pyridin-3-ylbiphenyl-3-carboxamide;
4"-(aminomethyl)-6-chloro-*N-*(6-methoxypyridin-3-yl)-1,1':4',1"-terphenyl-3-carboxamide;
6-chloro-4'-[5-(hydroxymethyl)pyridin-3-yl]-*N-*(6-methoxypyridin-3-yl)biphenyl-3-carboxamide;
6-chloro-*N³-*(6-methoxypyridin-3-yl)-1,1':4',1"-terphenyl-3,3"-dicarboxamide;
6-chlorn-3"-hydroxy-*N-*(6-methoxypyridin-3-yl)-1,1':4',1"-terphenyl-3-carboxamide;
6-chloro-4"-hydroxy-*N-*(6-methoxypyridin-3-yl)-1,1':4',1"-terphenyl-3-carboxamide;
6-chloro-4'-{5-[(dimethylamino)methyl]pyridin-3-yl}-*N-*(6-methoxypyridin-3-yl)biphenyl-3-carboxamide;
5-fluoro-*N-*(6-methoxypyridin-3-yl)-1,1':4',1"-terphenyl-3-carboxamide;
6-chloro-*N-*{3-[(morpholin-4-ylacetyl)amino]phenyl}-1,1':4',1"-terphenyl-3-carboxamide;
6-chloro-*N-*{4-[(morpholin-4-ylacetyl)amino]phenyl}-1,1':4',1"-terphenyl-3-carboxamide;
6-chloro-*N-*{6-[(4-methylpiperazin-1-yl)methyl]pyridin-3-yl}-1,1':4',1"-terphenyl-3-carboxamide;
6-chloro-*N-*[6-(hydroxymethyl)pyridin-3-yl]-1,1':4',1"-terphenyl-3-carboxamide;
2"-chloro-5"-{[(6-methoxypyridin-3-yl)amino]carbonyl}-1,1':4',1"-terphenyl-4-yl 4-methylpiperazine-1-carboxylate;
2-{[(6-chloro-1,1':4',1"-terphenyl-3-yl)carbonyl]amino}indane-2-carboxylic acid;
(4*S*)-3-[(6-chloro-1,1':4',1"-terphenyl-3-yl)carbonyl]-1,3-thiazolidine-4-carboxylic acid;
*N-*[(6-chloro-1,1':4',1"-terphenyl-3-yl)carbonyl]-D-methionine; and
*N-*[(6-chloro-1,1':4',1"-terphenyl-3-yl)carbonyl]-3-(7,7a-dihydro-1*H-*indol-3-yl)-D-alanine;

40. A pharmaceutical composition comprising a compound, according to any one of claims 1-39 and a pharmaceutically acceptable carrier.

41. Use of
a compound of the formula 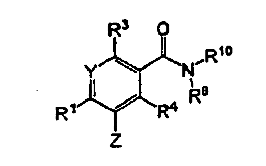 or a pharmaceutically acceptable salt, ester or amide thereof,
wherein
Y is C-R² or N;
R¹ is selected from hydrogen, C₁-C₁₂ alkyl or halo;
R², R³ and R⁴ are independently selected from hydrogen or halo;
R6 is hydrogen, or
R⁶ is C₁-C₁₂ alkyl optionally substituted with one or two groups selected from amino, monoalkylamino or dialkylamino;
R¹⁰ is selected from aryl, arylalkyl, heteroaryl, heteroarylalkyl, cycloalkyl, cycloalkylalkyl, heterocycloalkyl or heterocycloalkylalkyl, each of which is optionally substituted at the ring portion and/or alkyl portion with one to five groups selected from C₁-C₁₂ alkyl, C₂-C₁₂ alkenyl, C₂-C₁₂ alkynyl, trifluoromethyl, C₁-C₁₂ alkoxy, hydroxyl, halo, amino, monoalkylamino, dialkylamino, aminoalkyl, monoalkylaminoalkyl, dialkylaminoalkyl, nitro, -CN, oxo, -C(O)OR, -NH-C(O)-R', -C(O)-NHR', -SR, -SO₂R, -CO₂R, or aryl, heteroaryl, cycloalkyl or heterocycloalkyl, each of which is optionally substituted with one to three groups selected from C₁-C₁₂ alkyl, C₂-C₁₂ alkenyl, C₂-C₁₂ alkynyl, trifluoromethyl, C₁-C₁₂ alkoxy, hydroxyl, halo, amino, monoalkylamino, dialkylamino, aminoalkyl, monoalkylaminoalkyl, dialkylaminoalkyl, nitro, -CN, oxo, -C(O)OR, -NH-C(O)-R', -C(O)-NHR', -SR, -SO₂R, -CO₂R, or
R⁶ and R¹⁰ together with the nitrogen atom to which they are attached form a 5-7 membered heteroaryl or heterocyclic ring optionally substituted with one to three groups independently selected from C₁-C₁₂ alkyl, C₂-C₁₂ alkenyl, C₂-C₁₂ alkynyl, trifluoromethyl, C₁-C₁₂ alkoxy, hydroxyl, halo, amino, monoalkylamino, dialkylamino, aminoalkyl, monoalkylaminoalkyl, dialkylaminoalkyl, nitro, -CN, oxo, -C(O)OR, -NH-C(O)-R', -C(O)-NHR', -SR, -SO₂R, -CO₂R;
Z is selected from 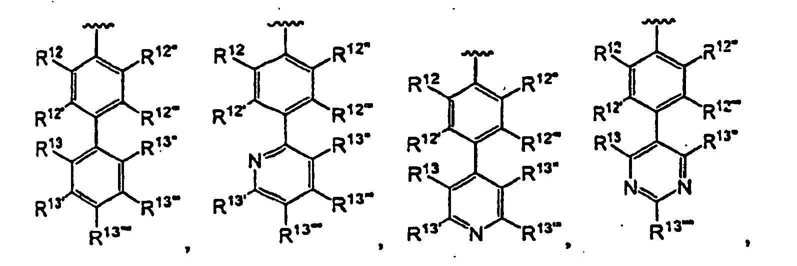 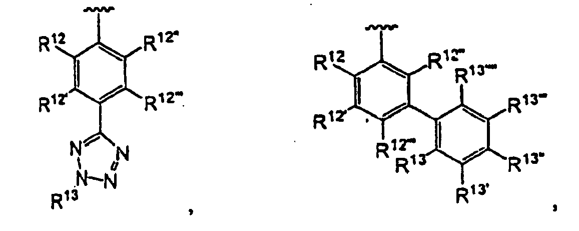 , or 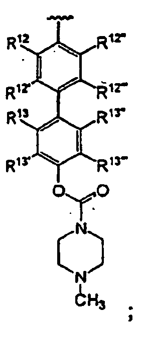
R¹², R^{12'}, R^{12"} and R^{12'''} are independently selected from hydrogen, C₁-C₁₂ alkyl, C₂-C₁₂ alkenyl, C₂-C₁₂ alkynyl, trifluoromethyl, C₁-C₁₂ alkoxy, hydroxyl, halo, amino, monoalkylamino, dialkylamino, aminoalkyl, monoalkylaminoalkyl, dialkylaminoalkyl nitro, -CN, -C(O)OR, -NH- C(O)-R', -C(O)-NHR', -SR, -SO₂R, -CO₂R;
R¹³, R^{13'}, R^{13"}, R^{13'''} and R^{13''''} are independently selected from hydrogen, C₁-C₁₂ alkyl, C₂-C₁₂ alkynyl, C₂-C₁₂ alkynyl, trifluoromethyl, C₁-C₁₂ alkoxy, hydroxyl, halo, amino, monoalkylamino, dialkylamino, aminoalkyl, monoalkylaminoalkyl, dialkylaminoalkyl, nitro, -CN, - C(O)OR, -NH-C(O)-R', -C(O)-NHR', -SR, -SO₂R, -CO₂R;
X is halo;
R is selected from hydrogen and C₁-C₁₂ alkyl; and
R' is independently selected from hydrogen, C₁-C₁₂ alkyl, amino, monoalkylamino, dialkylamino, arylalkyl, heteroaryl, heteroarylalkyl, cycloalkyl, cycloalkylalkyl, heterocycloalkyl or heterocycloalkylalkyl;
for the preparation of a pharmaceutical composition for treating a tumor.

42. The use of claim 41 wherein the tumor is selected from the group consisting of a sarcoma, a melanoma, a neuroblastoma, a carcinoma and a mesothelioma.

43. A compound of the formula: 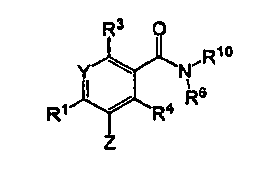 or a pharmaceutically acceptable salt, ester or amide thereof,
wherein
Y is C-R² or N;
R¹ is selected from hydrogen, C₁-C₁₂ alkyl or halo;
R², R³ and R⁴ are independently selected from hydrogen or halo;
R⁶ is hydrogen, or
R⁶ is C₁-C₁₂ alkyl optionally substituted with one or two groups selected from amino, monoalkylamino or dialkylamino;
R¹⁰ is selected from aryl, arylalkyl, heteroaryl, heteroarylalkyl, cycloalkyl, cycloalkylalkyl, heterocycloalkyl or heterocycloalkylalkyl, each of which is optionally substituted at the ring portion and/or alkyl portion with one to five groups selected from C₁-C₁₂ alkyl, C₂-C₁₂ alkenyl, C₂-C₁₂ alkynyl, trifluoromethyl , C₁-C₁₂ alkoxy, hydroxyl, halo, amino, monoalkylamino, dialkylamino, aminoalkyl, monoalkylaminoalkyl, dialkylaminoalkyl, nitro, -CN, oxo, -C(O)OR, -NH-C(O)-R', -C(O)-NHR', -SR, -SO₂R, -CO₂R, or aryl, heteroaryl, cycloalkyl or heterocycloalkyl, each of which is optionally substituted with one to three groups selected from C₁-C₁₂ alkyl, C₂-C₁₂ alkenyl, C₂-C₁₂ alkynyl, trifluoromethyl, C₁-C₁₂ alkoxy, hydroxyl, halo, amino, monoalkylamino, dialkylamino, aminoalkyl, monoalkylaminoalkyl, dialkylaminoalkyl, nitro, -CN, oxo, -C(O)OR, -NH-C(O)-R', -C(O)-NHR', -SR, -SO₂R, -CO₂R, or
R⁶ and R¹⁰ together with the nitrogen atom to which they are attached form a 5-7 membered heteroaryl or heterocyclic ring optionally substituted with one to three groups independently selected from C₁-C₁₂ alkyl, C₂-C₁₂ alkenyl, C₂-C₁₂ alkynyl, trifluoromethyl, C₁-C₁₂ alkoxy, hydroxyl, halo, amino, monoalkylamino, dialkylamino, aminoalkyl, monoalkylaminoalkyl, dialkylaminoalkyl, nitro, -CN, oxo, -C(O)OR, -NH-C(O)-R', -C(O)-NHR', -SR, -SO₂R, -CO₂R;
Z is selected from 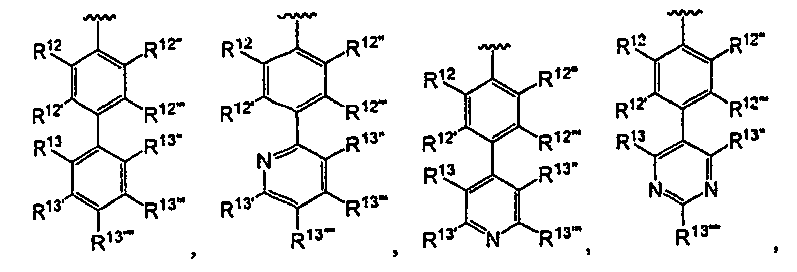 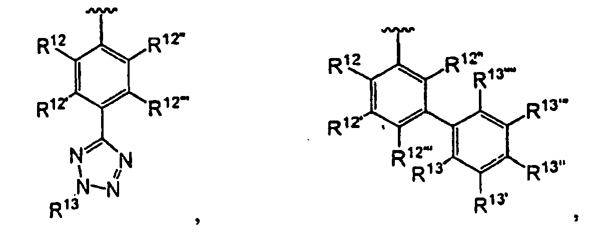 , or 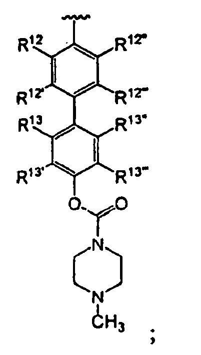
R¹², R^{12'}, R^{12"} and R^{12"'} are independently selected from hydrogen, C₁-C₁₂ alkyl, C₂-C₁₂ alkenyl, C₂-C₁₂ alkynyl, trifluoromethyl, C₁-C₁₂ alkoxy, hydroxyl, halo, amino, monoalkylamino, dialkylamino, aminoalkyl, monoalkylaminoalkyl, dialkylaminoalkyl, nitro, -CN, -C(O)OR, -NH- C(O)-R', -C(O)-NHR', -SR, -SO₂R, -CO₂R;
R¹³, R^{13'}, R^{13"}, R^{13'''} and R^{13""} are independently selected from hydrogen, C₁-C₁₂ alkyl, C₂-C₁₂ alkenyl, C₂-C₁₂ alkynyl, trifluoromethyl, C₁-C₁₂ alkoxy, hydroxyl, halo, amino, monoalkylamino, dialkylamino, aminoalkyl, monoalkylaminoalkyl, dialkylaminoalkyl, nitro, -CN, - C(O)OR, -NH-C(O)-R', -C(O)-NHR', -SR, -SO₂R, -CO₂R;
X is halo;
R is selected from hydrogen and C₁-C₁₂ alkyl; and
R' is independently selected from hydrogen, C₁-C₁₂ alkyl, amino, monoalkylamino, dialkylamino, arylalkyl, heteroaryl, heteroarylalkyl, cycloalkyl, cycloalkylalkyl, heterocycloalkyl or heterocycloalkylalkyl;
for use in the treatment of a tumor.

44. The compound of claim 43, wherein the tumor is selected from the group consisting of a sarcoma, a melanoma, a neuroblastoma, a carcinoma and a mesothelioma.

## Patentansprüche

1. Verbindung der Formel: 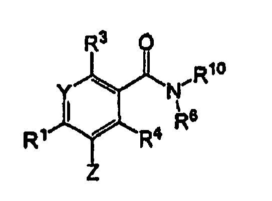 oder ein pharmazeutisch verträgliches Salz, ein pharmazeutisch verträglicher Ester oder ein pharmazeutisch verträgliches Amid derselben,
wobei
Y C-R² oder N darstellt;
R¹ ausgewählt ist aus Wasserstoff, C₁-C₁₂-Alkyl oder Halogen;
R², R³ und R⁴ unabhängig voneinander ausgewählt sind aus Wasserstoff oder Halogen;
R⁶ Wasserstoff ist oder
R⁶ C₁-C₁₂-Alkyl ist, das optional mit einer oder zwei Gruppen substituiert ist, die ausgewählt sind aus Amino, Monoalkylamino oder Dialkylamino;
R¹⁰ ausgewählt ist aus Aryl, Heteroaryl, Heteroarylalkyl, Cycloalkyl, Hete- rocycloalkyl oder Heterocycloalkylalkyl, die jeweils optional am Ringteil und/oder Alkylteil mit ein bis fünf Gruppen substituiert sind, die ausgewählt sind aus C₁-C₁₂-Alkyl, C₂-C₁₂-Alkenyl, C₂-C₁₂- Alkinyl, Trifluormethyl, C₁-C₁₂-Alkoxy, Hydroxyl, Halogen, Amino, Monoalkylamino, Dialkylamino, Aminoalkyl, Monoalkylaminoalkyl, Dialkylaminoalkyl, Nitro, -CN, Oxo, -C(O)OR, -NH-C(O)-R', -C(O)- NHR', -SR, -SO₂R, -CO₂R oder Aryl, Heteroaryl, Cycloalkyl oder Heterocycloalkyl, die jeweils optional mit ein bis drei Gruppen substituiert sind, die ausgewählt sind aus C₁-C₁₂-Alkyl, C₂-C₁₂-Alkenyl, C₂-C₁₂- Alkinyl, Trifluormethyl, C₁-C₁₂-Alkoxy, Hydroxyl, Halogen, Amino, Monoalkylamino, Dialkylamino, Aminoalkyl, Mono- alkylaminoalkyl, Dialkylaminoalkyl, Nitro, -CN, Oxo, - C(O)OR, -NH-C(O)-R', -C(O)-NHR', -SR, -SO₂R, -CO₂R oder
R⁶ und R¹⁰ zusammen mit dem Stickstoffatom, an das sie gebunden sind, einen 5-7-gliedrigen Heteroarylring bilden, der optional mit ein bis drei Gruppen substituiert ist, die unabhängig voneinander ausge- wählt sind aus C₁-C₁₂-Alkyl, C₂-C₁₂-Alkenyl, C₂-C₁₂-Alkinyl, Trifluormethyl, C₁-C₁₂-Alkoxy, Hydroxyl, Halogen, Amino, Monoalkylamino, Dialkylamino, Aminoalkyl, Monoalkylaminoalkyl, Dialkylaminoalkyl, Nitro, -CN, Oxo, -C(O)OR, -NH-C(O)-R', -C(O)- NHR', -SR, -SO₂, -CO₂R;
Z ausgewählt ist aus 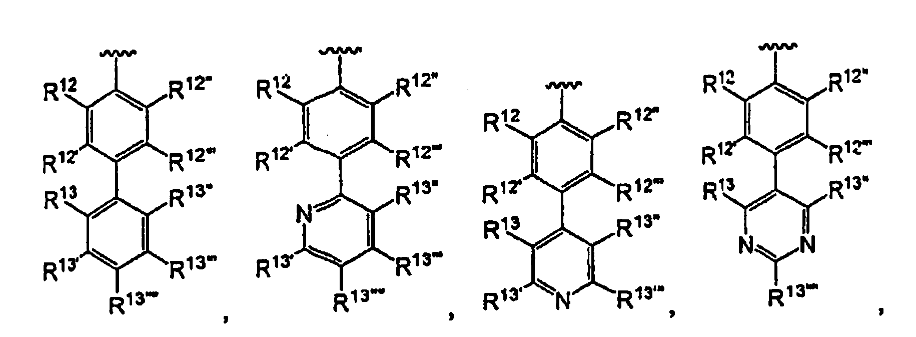 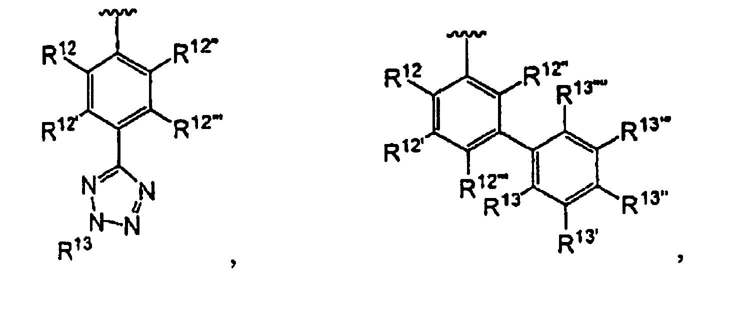 , oder 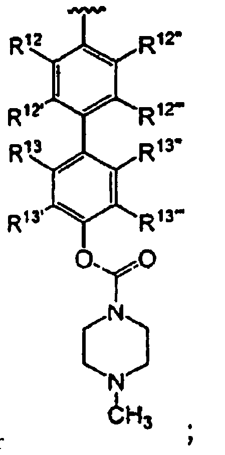
R¹², R^{12'}, R^{12"} und R^{12'"} unabhängig voneinander ausgewählt sind aus Was- serstoff, C₁-C₁₂-Alkyl, C₂-C₁₂-Alkenyl, C₂-C₁₂-Alkinyl, Trifluormethyl, C₁-C₁₂-Alkoxy, Hydroxyl, Halogen, Amino, Monoalkylamino, Dialkylamino, Aminoalkyl, Monoalkylaminoalkyl, Dialkylaminoalkyl, Nitro, -CN, -C(O)OR, -NH-C(O)-R', -C(O)-NHR', -SR, -SO₂R, -CO₂R;
R¹³, R^{13'}, R^{13"}, R^{13"'} und R^{13""} unabhängig voneinander ausgewählt sind aus Wasserstoff, C₁-C₁₂-Alkyl, C₂-C₁₂-Alkenyl, C₂-C₁₂-Alkinyl, Trifluor- methyl, C₁-C₁₂-Alkoxy, Hydroxyl, Halogen, Amino, Monoalkylamino, Dialkylamino, Aminoalkyl, Monoalkylaminoalkyl, Dialkylaminoalkyl, Nitro, -CN, -C(O)OR, -NH-C(O)-R', -C(O)-NHR', -SR, -SO₂R, -CO₂R;
X Halogen ist;
R ausgewählt ist aus Wasserstoff und C₁-C₁₂-Alkyl; und
R' unabhängig ausgewählt ist aus Wasserstoff, C₁-C₁₂-Alkyl, Amino, Mono- alkylamino, Dialkylamino, Arylalkyl, Heteroaryl, Heteroarylalkyl, Cycloalkyl, Cycloalkylalkyl, Heterocycloalkyl oder Heterocyclo- alkylalkyl.

2. Verbindung gemäß Anspruch 1 mit der Formel:

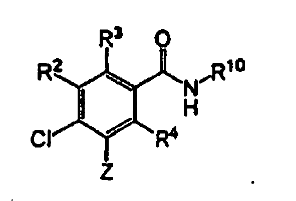

3. Verbindung gemäß Anspruch 2, wobei R², R³ und R⁴ Wasserstoff sind.

4. Verbindung gemäß Anspruch 1 mit der Formel:

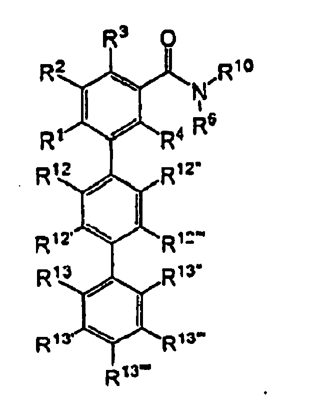

5. Verbindung gemäß Anspruch 4, wobei
R¹ Wasserstoff, Chlor oder Methyl ist und
R², R³ und R⁴ Wasserstoff sind.

6. Verbindung gemäß Anspruch 4, wobei
R^{12'} Wasserstoff oder Halogen ist und
R¹², R^{12"} und R^{12"'} Wasserstoff sind.

7. Verbindung gemäß Anspruch 4, wobei R¹³, R^{13'} und R^{13"} Wasserstoff sind.

8. Verbindung gemäß Anspruch 1 mit der Formel:

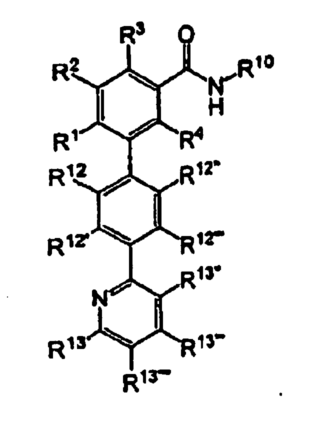

9. Verbindung gemäß Anspruch 8, wobei R¹ Chlor und R², R³ und R⁴ Wasserstoff sind.

10. Verbindung gemäß Anspruch 8, wobei R¹², R^{12'}, R^{12"} und R^{12"'} Wasserstoff sind.

11. Verbindung gemäß Anspruch 8, wobei R^{13'}, R^{13"}, R^{13'"} und R^{13""} Wasserstoff sind.

12. Verbindung gemäß Anspruch 1 mit der Formel:

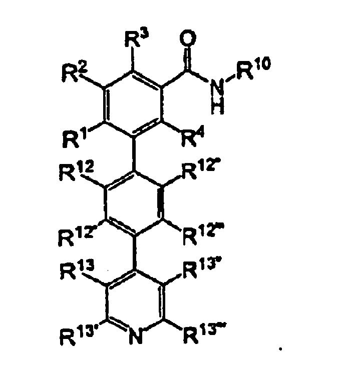

13. Verbindung gemäß Anspruch 12, wobei R¹ Chlor und R², R³ und R⁴ Wasserstoff sind.

14. Verbindung gemäß Anspruch 12, wobei R¹², R^{12'}, R^{12"} und R^{12"'} Wasserstoff sind.

15. Verbindung gemäß Anspruch 12, wobei R¹³, R^{13'}, R^{13"} und R^{13"'} Wasserstoff sind.

16. Verbindung gemäß Anspruch 1 mit der Formel:

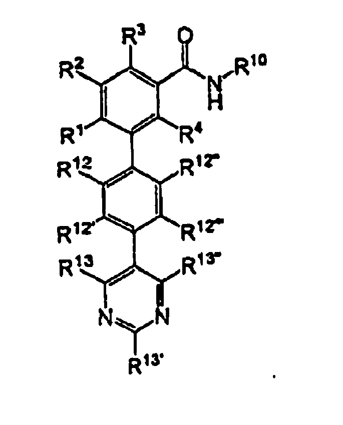

17. Verbindung gemäß Anspruch 16, wobei R¹ Chlor und R², R³ und R⁴ Wasserstoff sind.

18. Verbindung gemäß Anspruch 16, wobei R¹², R^{12'}, R^{12"} und R^{12"'} Wasserstoff sind.

19. Verbindung gemäß Anspruch 16, wobei R¹³, R^{13'} und R^{13"} Wasserstoff sind.

20. Verbindung gemäß Anspruch 1 mit der Formel:

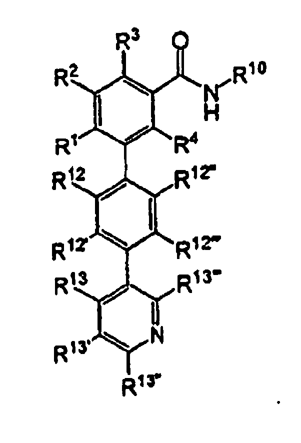

21. Verbindung gemäß Anspruch 20, wobei R¹ Chlor und R², R³ und R⁴ Wasserstoff sind.

22. Verbindung gemäß Anspruch 20, wobei R¹², R^{12'}, R^{12"} und R^{12"'} Wasserstoff sind.

23. Verbindung gemäß Anspruch 20, wobei R¹³, R^{13"} und R^{13'''} Wasserstoff sind und R^{13'} ausgewählt ist aus Wasserstoff, Aminoalkyl, Monoalkylaminoalkyl, Dialkylaminoalkyl oder C₁-C₁₂-Alkoxy.

24. Verbindung gemäß Anspruch 1 mit der Formel: 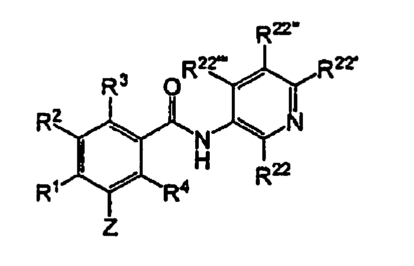 wobei
R22, R^{22'}, R^{22"} und R^{22'''} unabhängig voneinander ausgewählt sind aus Was- serstoff, C₁-C₁₂-Alkyl, C₂-C₁₂-Alkenyl, C₂-C₁₂-Alkinyl, Trifluormethyl, C₁-C₁₂-Alkoxy, Hydroxyl, Halogen, Amino, Monoalkylamino, Dialkyl- amino, Aminoalkyl, Monoalkylaminoalkyl, Dialkylaminoalkyl, Nitro, -CN, Oxo, -C(O)OR, -NH-C(O)-R', -C(O)-NHR', -SR, -SO₂R, -CO₂R; oder Aryl, Heteroaryl, Cycloalkyl oder Heterocycloalkyl, die jeweils optional mit ein bis drei Gruppen substituiert sind, die ausgewählt sind aus C₁-C₁₂-Alkyl, C₂-C₁₂-Alkenyl, C₂-C₁₂-Alkinyl, Trifluor- methyl, C₁-C₁₂-Alkoxy, Hydroxyl, Halogen, Amino, Monoalkylamino, Dialkylamino, Aminoalkyl, Monoalkylaminoalkyl, Dialkylaminoalkyl, Nitro, -CN, Oxo, -C(O)OR, -NH-C(O)-R', -C(O)-NHR', -SR, -SO₂R, -CO₂R.

25. Verbindung gemäß Anspruch 24, wobei R¹ Chlor und R², R³ und R⁴ Wasserstoff sind.

26. Verbindung gemäß Anspruch 24, wobei R²², R^{22'}, R^{22"} und R^{22'''} ausgewählt sind aus Wasserstoff, Halogen, C₁-C₁₂-Alkyl, C₁-C₁₂-Alkoxy oder optional substituiertem Heterocyclyl.

27. Verbindung gemäß Anspruch 1 mit der Formel: 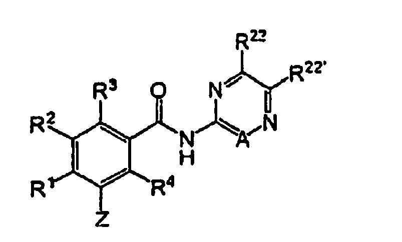 wobei
A -CH oder N ist; und
R²² und R^{22'} unabhängig voneinander ausgewählt sind aus Wasserstoff, C₁-C₁₂-Alkyl, C₂-C₁₂-Alkenyl, C₂-C₁₂-Alkinyl, Trifluormethyl, C₁-C₁₂- Alkoxy, Hydroxyl, Halogen, Amino, Monoalkylamino, Dialkylamino, Aminoalkyl, Monoalkylaminoalkyl, Dialkylaminoalkyl, Nitro, -CN, Oxo, -C(O)OR, -NH-C(O)-R', -C(O)-NHR', -SR, -SO₂R, -CO₂R; oder Aryl, Heteroaryl, Cycloalkyl oder Heterocycloalkyl, die jeweils optional mit ein bis drei Gruppen substituiert sind, die ausgewählt sind aus C₁-C₁₂-Alkyl, C₂-C₁₂-Alkenyl, C₂-C₁₂-Alkinyl, Trifluor- methyl, C₁-C₁₂-Alkoxy, Hydroxyl, Halogen, Amino, Monoalkylamino, Dialkylamino, Aminoalkyl, Monoalkylaminoalkyl, Dialkylaminoalkyl, Nitro, -CN, Oxo, -C(O)OR, -NH-C(O)-R', -C(O)-NHR', -SR, -SO₂R, -CO₂R.

28. Verbindung gemäß Anspruch 27, wobei R¹ Chlor ist und R², R³ und R⁴ Wasserstoff sind.

29. Verbindung gemäß Anspruch 27, wobei R²² und R^{22'} ausgewählt sind aus Wasserstoff, Halogen, C₁-C₁₂-Alkyl und C₁-C₁₂-Alkoxy.

30. Verbindung gemäß Anspruch 1 mit der Formel: 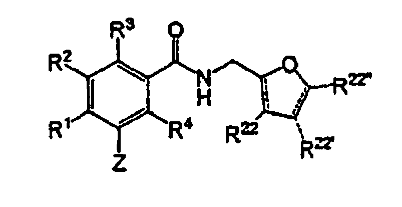 wobei die ---- Bindungen beide vorhanden oder beide abwesend sind, und
R¹, R², R³, R⁴ und Z wie in Anspruch 1 definiert sind;
und
R²², R^{22'} und R^{22"} unabhängig voneinander ausgewählt sind aus Wasser- stoff, C₁-C₁₂-Alkyl, C₂-C₁₂-Alkenyl, C₂-C₁₂-Alkinyl, Trifluormethyl, C₁-C₁₂-Alkoxy, Hydroxyl, Halogen, Amino, Monoalkylamino, Dialkyl- amino, Aminoalkyl, Monoalkylaminoalkyl, Dialkylaminoalkyl, Nitro, -CN, Oxo, -C(O)OR, -NH-C(O)-R', -C(O)-NHR', -SR, -SO₂R, -CO₂R; oder Aryl, Heteroaryl, Cycloalkyl oder Heterocycloalkyl, die jeweils optional mit ein bis drei Gruppen substituiert sind, die ausgewählt sind aus C₁-C₁₂-Alkyl, C₂-C₁₂-Alkenyl, C₂-C₁₂-Alkinyl, Trifluor- methyl, C₁-C₁₂-Alkoxy, Hydroxyl, Halogen, Amino, Monoalkylamino, Dialkylamino, Aminoalkyl, Monoalkylaminoalkyl, Dialkylaminoalkyl, Nitro, -CN, Oxo, -C(O)OR, -NH-C(O)-R', -C(O)-NHR', -SR, -SO₂R, -CO₂R.

31. Verbindung gemäß Anspruch 30, wobei R¹ Chlor ist und R², R³ und R⁴ Wasserstoff sind.

32. Verbindung gemäß Anspruch 30, wobei R²² und R^{22'} ausgewählt sind aus Wasserstoff, Halogen, C₁-C₁₂-Alkyl und C₁-C₁₂-Alkoxy.

33. Verbindung gemäß Anspruch 1 mit der Formel: 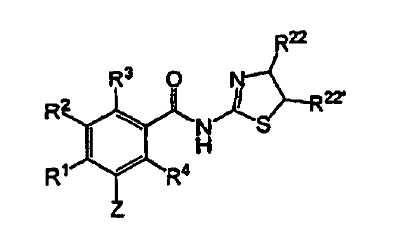 wobei
A -CH oder N ist; und
R²² und R^{22'} unabhängig voneinander ausgewählt sind aus Wasserstoff, C₁-C₁₂-Alkyl, C₂-C₁₂-Alkenyl, C₂-C₁₂-Alkmyl, Trifluormethyl, C₁-C₁₂- Alkoxy, Hydroxyl, Halogen, Amino, Monoalkylamino, Dialkylamino, Aminoalkyl, Monoalkylaminoalkyl, Dialkylaminoalkyl, Nitro, -CN, Oxo, -C(O)OR, -NH-C(O)-R', -C(O)-NHR', -SR, -SO₂R, -CO₂R; oder Aryl, Heteroaryl, Cycloalkyl oder Heterocycloalkyl, die jeweils optio- nal mit ein bis drei Gruppen substituiert sind, die ausgewählt sind aus C₁-C₁₂-Alkyl, C₂-C₁₂-Alkenyl, C₂-C₁₂-Alkinyl, Trifluormethyl, C₁- C₁₂-Alkoxy, Hydroxyl, Halogen, Amino, Monoalkylamino, Dialkyl- amino, Aminoalkyl, Monoalkylaminoalkyl, Dialkylaminoalkyl, Nitro, -CN, Oxo, -C(O)OR, -NH-C(O)-R', -C(O)-NHR', -SR, -SO₂R, -CO₂R.

34. Verbindung gemäß Anspruch 33, wobei R¹ Chlor ist und R², R³ und R⁴ Wasserstoff sind.

35. Verbindung gemäß Anspruch 33, wobei R²² und R^{22'} ausgewählt sind aus Wasserstoff, Halogen, C₁-C₁₂-Alkyl und C₁-C₁₂-Alkoxy.

36. Verbindung gemäß Anspruch 1 mit der Formel: 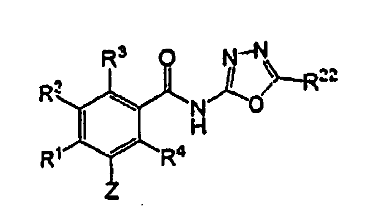 wobei
A -CH oder N ist; und
R²² wie unabhängig ausgewählt ist aus Wasserstoff, C₁-C₁₂-Alkyl, C₂-C₁₂- Alkenyl, C₂-C₁₂-Alkinyl, Trifluormethyl, C₁-C₁₂-Alkoxy, Hydroxyl, Halogen, Amino, Monoalkylamino, Dialkylamino, Aminoalkyl, Monoalkylaminoalkyl, Dialkylaminoalkyl, Nitro, -CN, Oxo, -C(O)OR, -NH-C(O)-R', -C(O)-NHR', -SR, -SO₂R, -CO₂R; oder Aryl, Heteroaryl, Cycloalkyl oder Heterocycloalkyl, die jeweils optional mit ein bis drei Gruppen substituiert sind, die ausgewählt sind aus C₁-C₁₂-Alkyl, C₂-C₁₂-Alkenyl, C₂-C₁₂-Alkinyl, Trifluormethyl, C₁-C₁₂-Alkoxy, Hydroxyl, Halogen, Amino, Monoalkylamino, Dialkylamino, Aminoalkyl, Monoalkylaminoalkyl, Dialkylaminoalkyl, Nitro, -CN, Oxo, -C(O)OR, -NH-C(O)-R', -C(O)-NHR', -SR, -SO₂R, -CO₂R.

37. Verbindung gemäß Anspruch 36, wobei R¹ Chlor ist und R², R³ und R⁴ Wasserstoff sind.

38. Verbindung gemäß Anspruch 36, wobei R²² und R^{22'} ausgewählt sind aus Wasserstoff, Halogen, C₁-C₁₂-Alkyl, C₁-C₁₂-Alkoxy und optional substituiertem Aryl.

39. Verbindung gemäß Anspruch 1, ausgewählt aus:
6-Chlor-*N-*(tetrahydrofuran-2-ylmethyl)-1,1':4',1"-terphenyl-3-carboxamid;
6-Chlor-*N-*(2-furylmethyl)-1,1':4',1 "-terphenyl-3-carboxamid;
6-Chlor-*N-*(6-chlorpyridin-3-yl)-1,1':4',1"-terphenyl-3-carboxamid;
6-Chlor-*N-*(4,5-dihydro-1,3-thiazol-2-yl)-1,1':4',1"-terphenyl-3-carboxamid ;
6-Chlor-*N-*(2-pyrrolidin-1-ylethyl)-1,1':4',1"-terphenyl-3-carboxamid;
6-Chlor-*N-*(5-chlorpyrimidin-2-yl)-1,1':4',1"-terphenyl-3-carboxamid;
6-Chlor-*N-*(3-methylisoxazol-5-yl)-1,1':4',1"-terphenyl-3-carboxamid;
6-Chlor-*N-*[6-(methylsulfonyl)-1,3-benzothiazol-2-yl]-1,1':4',1"-terphenyl-3 - carboxamid;
6-Chlor-*N-*1,2,4-triazin-3-yl-1,1':4',1"-terphenyl-3-carboxamid;
Methyl-3-{[(6-chlor-1,1':4',1"-terphenyl-3-yl)carbonyl]amino}benzoat;
6-Chlor-*N-*(5-phenyl-1,3,4-oxadiazol-2-yl)-1,1':4',1"-terphenyl-3-carboxamid;
6-Chlor-*N-*[3-(2-methyl-1,3-dioxolan-2-yl)phenyl]-1,1':4',1 "-terphenyl-3-carboxamid;
6-Chlor-*N-*(4-methoxyphenyl)-1,1':4',1"-terphenyl-3-carboxamid;
6-Chlor-*N-*chinolin-6-yl-1,1':4',1"-terphenyl-3-carboxamid;
6-Chlor-*N-*pyrazin-2-yl-1,1':4',1"-terphenyl-3-carboxamid;
*N-*(5-brompyridin-2-yl)-6-chlor-1,1':4',1"-terphenyl-3-carboxamid;
6-Chlor-*N-*(6-methoxypyridin-3-yl)-1,1':4',1"-terphenyl-3-carboxamid;
6-Chlor-*N-*(6-fluorpyridin-3-yl)-1,1':4',1"-terphenyl-3-carboxamid;
6-Chlor-*N-*(5-chlorpyridin-2-yl)-1,1':4',1"-terphenyl-3-carboxamid;
6-Chlor-*N-*(6-methoxypyridin-3-yl)-4'-pyridin-2-ylbiphenyl-3-carboxamid;
6-Chlor-3'-fluor-*N-*(6-methoxypyridin-3-yl)-1,1':4',1"-terphenyl-3-carboxamid;
6-Chlor-*N-*(6-methoxypyridin-3-yl)-4'-pyridin-4-ylbiphenyl-3-carboxamid;
6-Chlor-*N-*(4-methylphenyl)-1,1':4',1"-terphenyl-3-carboxamid;
6-Chlor-*N-*(3-fluor-4-methoxyphenyl)-1,1':4',1"-terphenyl-3-carboxamid;
6-Chlor-*N-*(6-methoxypyridin-3-yl)-4'-pyrimidin-5-ylbiphenyl-3-carboxamid;
6-Chlor-*N*-[2-(dimethylamino)ethyl]-*N-*pyridin-3-yl-1,1':4',1"-terphenyl-3-carboxamid;
*N-*(2-Pyrrolidin-1-ylethyl)-1,1':4',1"-terphenyl-3-carboxamid;
6-Chlor-*N-*[6-(4-methylpiperazin-1-yl)pyridin-3-yl]-1,1':4',1"-terphenyl-3-carboxamid;
6-Chlor-*N-*(6-morpholin-4-ylpyridin-3-yl)-1,1':4',1"-terphenyl-3-carboxamid;
4-Chlor-*N-*(6-methoxypyridin-3-yl)-3-(6-phenylpyridin-3-yl)benzamid;
6-Chlor-*N-*[6-(methylthio) pyridin-3-yl]-1,1':4',1"-terphenyl-3-carboxamid;
6-Chlor-*N-*(5-methoxypyrazin-2-yl)-1,1':4',1"-terphenyl-3-carboxamid;
6-Chlor-*N-*[2-(methylthio)pyrimidin-5-yl]-1,1':4',1"-terphenyl-3-carboxamid;
6-Chlor-*N-*(2-methoxypyrimidin-5-yl)-1,1':4',1"-terphenyl-3-carboxamid;
6-Chlor-*N-*(6-methylpyridin-3-yl)-1,1':4',1"-terphenyl-3-carboxamid;
6-Chlor-*N-*(6-oxo-1,6-dihydropyridin-3-yl)-4'-pyridin-2-ylbiphenyl-3-carboxamid;
6-Chlor-*N-*(6-methoxypyridin-3-yl)-4'-(2-methyl-2*H-*tetrazol-5-yl)biphenyl-3-carboxamid;
6-Chlor-*N-*(6-methoxypyridin-3-yl)-1,1':3',1"-terphenyl-3-carboxamid;
*N-*(6-Methoxypyridin-3-yl)-6-methyl-1,1':4',1"-terphenyl-3-carboxamid;
5-Biphenyl-4-yl-6-chlor-*N-*(6-methoxypyridin-3-yl)nicotinamid;
6-Chlor-*N-*(6-methoxypyridin-3-yl)-4'-pyridin-2-ylbiphenyl-3-carboxamid;
6-Chlor-3"-formyl-*N-*(6-methoxypyridin-3-yl)-1,1':4',1"-terphenyl-3-carboxamid;
6-Chlor-3"-[(dimethylamino)methyl]-*N-*(6-methoxypyridin-3-yl)-1,1':4',1"-terphenyl-3-carboxamid;
6-Chlor-3"-(hydroxymethyl)-*N-*(6-methoxypyridin-3-yl)-1,1':4',1"-terphenyl-3-carboxamid;
6-Chlor-4"-[(dimethylamino)methyl]-*N-*(6-methoxypyridin-3-yl)-1,1':4',1"-terphenyl-3-carboxamid;
6-Chlor-4"-(hydroxymethyl)-*N-*(6-methoxypyridin-3-yl)-1,1':4',1"-terphenyl-3-carboxamid;
6-Chlor-*N-*(6-methoxypyridin-3-yl)-3"-(methylsulfonyl)-1,1':4',1"-terphenyl-3-carboxamid;
6-Chlor-*N-*(6-methoxypyridin-3-yl)-4'-pyridin-3-ylbiphenyl-3-carboxamid;
4"-(aminomethyl)-6-chlor-*N-*(6-methoxypyridin-3-yl)-1,1':4',1"-terphenyl-3-carboxamid;
6-Chlor-4'-[5-(hydroxymethyl)pyridin-3-yl]-*N-*(6-methoxypyridin-3-yl)biphenyl-3-carboxamid;
6-Chlor-*N*³-(6-methoxypyridin-3-yl)-1,1':4',1"-terphenyl-3,3"-dicarboxamid;
6-Chlor-3"-hydroxy-*N-*(6-methoxypyridin-3-yl)-1,1':4',1"-terphenyl-3-carboxamid;
6-Chlor-4"-hydroxy-*N-*(6-methoxypyridin-3-yl)-1,1':4',1"-terphenyl-3-carboxamid;
6-Chlor-4'-{5-[(dimethylamino)methyl]pyridin-3-yl}-*N-*(6-methoxypyridin-3-yl)biphenyl-3-carboxamid;
5-Fluor-*N-*(6-methoxypyridin-3-yl)-1,1':4',1"-terphenyl-3-carboxamid;
6-Chlor-*N-*{3-[(morpholin-4-ylacetyl)amino]phenyl}-1,1':4',1"-terphenyl-3-carboxamid;
6-Chlor-*N-*{4-[(morpholin-4-ylacetyl)amino]phenyl}-1,1':4',1"-terphenyl-3-carboxamid;
6-Chlor-*N-*{6-[(4-methylpiperazin-1-yl)methyl]pyridin-3-yl}-1,1':4',1 "-terphenyl-3-carboxamid;
6-Chlor-*N-*[6-(hydroxymethyl)pyridin-3-yl]-1,1':4',1"-terphenyl-3-carboxamid;
2"-Chlor-5"-{[(6-methoxypyridin-3-yl)amino]carbonyl}-1,1':4',1"-terphenyl-4-yl 4-methylpiperazin-1-carboxylat;
2-{[(6-Chlor-1,1':4',1"-terphenyl-3-yl)carbonyl]amino}indan-2-carbonsäure;
(4S)-3-[(6-Chlor-1,1':4',1"-terphenyl-3-yl)carbonyl]-1,3-thiazolidin-4-carbonsäure;
*N-*[(6-Chlor-1,1':4',1"-terphenyl-3-yl)carbonyl]-D-methionin; und
N-[(6-Chlor-1,1':4',1"-terphenyl-3-yl)carbonyl]-3-(7,7a-dihydro-1*H-*indol-3-yl)-D-alanin.

40. Pharmazeutische Zusammensetzung, umfassend eine Verbindung gemäß einem der Ansprüche 1-39 und einen pharmazeutisch verträglichen Träger.

41. Verwendung einer Verbindung der Formel: 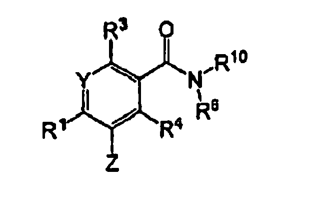 oder eines pharmazeutisch verträglichen Salzes, Esters oder Amids derselben zur Herstellung einer pharmazeutischen Zusammensetzung zur Behandlung eines Tumors, wobei
Y C-R² oder N darstellt;
R¹ ausgewählt ist aus Wasserstoff, C₁-C₁₂-Alkyl oder Halogen;
R², R³ und R⁴ unabhängig voneinander ausgewählt sind aus Wasserstoff oder Halogen;
R⁶ Wasserstoff ist oder
R⁶ C₁-C₁₂-Alkyl ist, das optional mit einer oder zwei Gruppen substituiert ist, die ausgewählt sind aus Amino, Monoalkylamino oder Dialkylamino;
R¹⁰ ausgewählt ist aus Aryl, Arylalkyl, Heteroaryl, Heteroarylalkyl, Cyclo- alkyl, Cycloalkylalkyl, Heterocycloalkyl oder Heterocycloalkylalkyl, die jeweils optional am Ringteil und/oder Alkylteil mit ein bis fünf Gruppen substituiert sind, die ausgewählt sind aus C₁-C₁₂-Alkyl, C₂-C₁₂-Alkenyl, C₂-C₁₂-Alkinyl, Trifluormethyl, C₁-C₁₂-Alkoxy, Hydroxyl, Halogen, Amino, Monoalkylamino, Dialkylamino, Aminoalkyl, Monoalkylaminoalkyl, Dialkylaminoalkyl, Nitro, -CN, Oxo, -C(O)OR, -NH-C(O)-R', -C(O)-NHR', -SR, -SO₂R, -CO₂R oder Aryl, Heteroaryl, Cycloalkyl oder Heterocycloalkyl, die jeweils optional mit ein bis drei Gruppen substituiert sind, die ausgewählt sind aus C₁-C₁₂-Alkyl, C₂-C₁₂-Alkenyl, C₂-C₁₂- Alkinyl, Trifluormethyl, C₁-C₁₂-Alkoxy, Hydroxyl, Halogen, Amino, Monoalkylamino, Dialkylamino, Aminoalkyl, Mono- alkylaminoalkyl, Dialkylaminoalkyl, Nitro, -CN, Oxo, - C(O)OR, -NH-C(O)-R', -C(O)-NHR', -SR, -SO₂R, -CO₂R oder
R⁶ und R¹⁰ zusammen mit dem Stickstoffatom, an das sie gebunden sind, einen 5-7-gliedrigen Heteroaryl- oder heterocyclischen Ring bilden, der optional mit ein bis drei Gruppen substituiert ist, die unabhän- gig voneinander ausgewählt sind aus C₁-C₁₂-Alkyl, C₂-C₁₂-Alkenyl, C₂-C₁₂-Alkinyl, Trifluormethyl, C₁-C₁₂-Alkoxy, Hydroxyl, Halogen, Amino, Monoalkylamino, Dialkylamino, Aminoalkyl, Monoalkyl- aminoalkyl, Dialkylaminoalkyl, Nitro, -CN, Oxo, -C(O)OR, -NH-C(O)- R', -C(O)-NHR', -SR, -SO₂R, -CO₂R;
Z ausgewählt ist aus 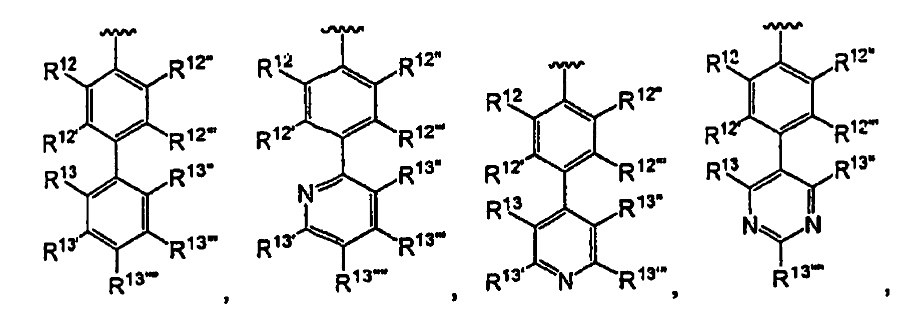 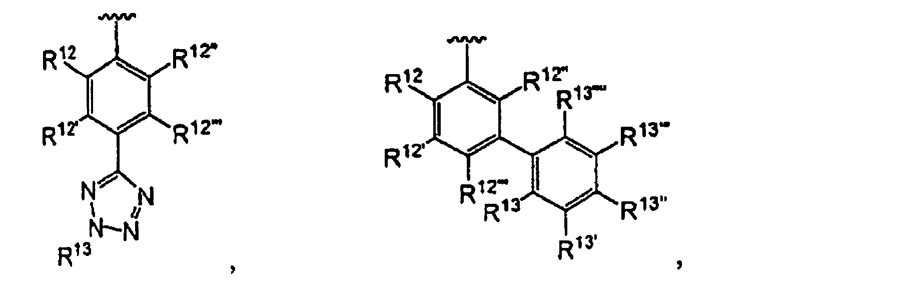 , oder 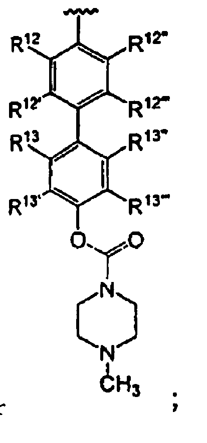
R¹², R^{12'}, R^{12"} und R^{12'''} unabhängig voneinander ausgewählt sind aus Was- serstoff, C₁-C₁₂-Alkyl, C₂-C₁₂-Alkenyl, C₂-C₁₂-Alkinyl, Trifluormethyl, C₁-C₁₂-Alkoxy, Hydroxyl, Halogen, Amino, Monoalkylamino, Dialkyl- amino, Aminoalkyl, Monoalkylaminoalkyl, Dialkylaminoalkyl, Nitro, -CN, -C(O)OR, -NH-C(O)-R', -C(O)-NHR', -SR, -SO₂R, -CO₂R;
R13, R^{13'}, R^{13"}, R^{13"} und R^{13''''} unabhängig voneinander ausgewählt sind aus Wasserstoff, C₁-C₁₂-Alkyl, C₂-C₁₂-Alkenyl, C₂-C₁₂-Alkinyl, Trifluor- methyl, C₁-C₁₂-Alkoxy, Hydroxyl, Halogen, Amino, Monoalkylamino, Dialkylamino, Aminoalkyl, Monoalkylaminoalkyl, Dialkylaminoalkyl, Nitro, -CN, -C(O)OR, -NH-C(O)-R', -C(O)-NHR', -SR, -SO₂R, -CO₂R;
X Halogen ist;
R ausgewählt ist aus Wasserstoff und C₁-C₁₂-Alkyl; und
R' unabhängig ausgewählt ist aus Wasserstoff, C₁-C₁₂-Alkyl, Amino, Mono- alkylamino, Dialkylamino, Arylalkyl, Heteroaryl, Heteroarylalkyl, Cycloalkyl, Cycloalkylalkyl, Heterocycloalkyl oder Heterocyclo- alkylalkyl.

42. Verwendung nach Anspruch 41, wobei der Tumor ausgewählt ist aus der Gruppe bestehend aus einem Sarkom, einem Melanom, einem Neuroblastom, einem Karzinom und einem Mesotheliom.

43. Verbindung der Formel: 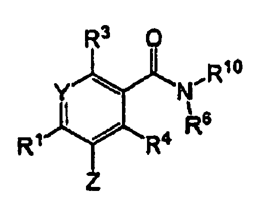 oder ein pharmazeutisch verträgliches Salz, ein pharmazeutisch verträglicher Ester oder ein pharmazeutisch verträgliches Amid derselben zur Verwendung in der Behandlung eines Tumors,
wobei
Y C-R² oder N darstellt;
R¹ ausgewählt ist aus Wasserstoff, C₁-C₁₂-Alkyl oder Halogen;
R², R³ und R⁴ unabhängig voneinander ausgewählt sind aus Wasserstoff oder Halogen;
R⁶ Wasserstoff ist oder
R⁶ C₁-C₁₂-Alkyl ist, das optional mit einer oder zwei Gruppen substituiert ist, die ausgewählt sind aus Amino, Monoalkylamino oder Dialkyl- amino;
R¹⁰ ausgewählt ist aus Aryl, Arylalkyl, Heteroaryl, Heteroarylalkyl, Cycloal- kyl, Cycloalkylalkyl, Heterocycloalkyl oder Heterocycloalkylalkyl, die jeweils optional am Ringteil und/oder Alkylteil mit ein bis fünf Gruppen substituiert sind, die ausgewählt sind aus C₁-C₁₂-Alkyl, C₂-C₁₂-Alkenyl, C₂-C₁₂-Alkinyl, Trifluormethyl, C₁-C₁₂-Alkoxy, Hydroxyl, Halogen, Amino, Monoalkylamino, Dialkylamino, Amino- alkyl, Monoalkylaminoalkyl, Dialkylaminoalkyl, Nitro, -CN, Oxo, -C(O)OR, -NH-C(O)-R', -C(O)-NHR', -SR, -SO₂R, -CO₂R oder Aryl, Heteroaryl, Cycloalkyl oder Heterocycloalkyl, die jeweils optio- nal mit ein bis drei Gruppen substituiert sind, die ausge- wählt sind aus C₁-C₁₂-Alkyl, C₂-C₁₂-Alkenyl, C₂-C₁₂-Alkinyl, Trifluormethyl, C₁-C₁₂-Alkoxy, Hydroxyl, Halogen, Amino, Monoalkylamino, Dialkylamino, Aminoalkyl, Monoalkyl- aminoalkyl, Dialkylaminoalkyl, Nitro, -CN, Oxo, -C(O)OR, -NH-C(O)-R', -C(O)-NHR', -SR, -SO₂R, -CO₂R oder
R⁶ und R¹⁰ zusammen mit dem Stickstoffatom, an das sie gebunden sind, einen 5-7-gliedrigen Heteroaryl- oder heterocyclischen Ring bilden, der optional mit ein bis drei Gruppen substituiert ist, die unabhän- gig voneinander ausgewählt sind aus C₁-C₁₂-Alkyl, C₂-C₁₂-Alkenyl, C₂-C₁₂-Alkinyl, Trifluormethyl, C₁-C₁₂-Alkoxy, Hydroxyl, Halogen, Amino, Monoalkylamino, Dialkylamino, Aminoalkyl, Monoalkyl- aminoalkyl, Dialkylaminoalkyl, Nitro, -CN, Oxo, -C(O)OR, -NH-C(O)- R', -C(O)-NHR', -SR, -SO₂R, -CO₂R;
Z ausgewählt ist aus 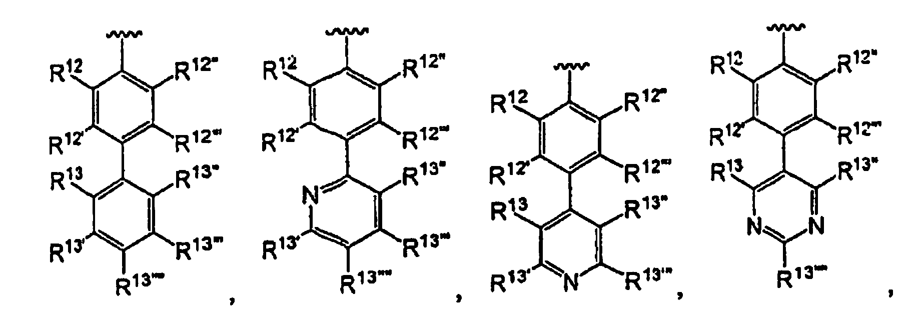 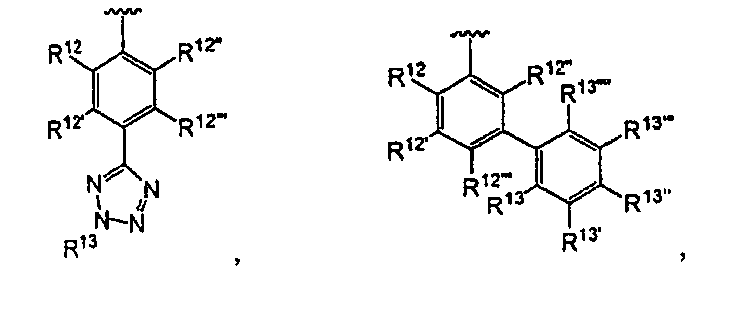 , oder 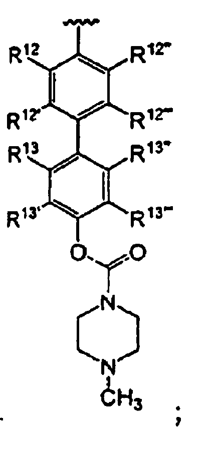
R¹², R^{12'}, R^{12"} und R^{12'''} unabhängig voneinander ausgewählt sind aus Was- serstoff, C₁-C₁₂-Alkyl, C₂-C₁₂-Alkenyl, C₂-C₁₂-Alkinyl, Trifluormethyl, C₁-C₁₂-Alkoxy, Hydroxyl, Halogen, Amino, Monoalkylamino, Dialkyl- amino, Aminoalkyl, Monoalkylaminoalkyl, Dialkylaminoalkyl, Nitro, -CN, -C(O)OR, -NH-C(O)-R', -C(O)-NHR', -SR, -SO₂R, -CO₂R;
R13, R^{13'}, R^{13"}, R^{13"'} und R^{13""} unabhängig voneinander ausgewählt sind aus Wasserstoff, C₁-C₁₂-Alkyl, C₂-C₁₂-Alkenyl, C₂-C₁₂-Alkinyl, Trifluor- methyl, C₁-C₁₂-Alkoxy, Hydroxyl, Halogen, Amino, Monoalkylamino, Dialkylamino, Aminoalkyl, Monoalkylaminoalkyl, Dialkylaminoalkyl, Nitro, -CN, -C(O)OR, -NH-C(O)-R', -C(O)-NHR', -SR, -SO₂R, -CO₂R;
X Halogen ist;
R ausgewählt ist aus Wasserstoff und C₁-C₁₂-Alkyl; und
R' unabhängig ausgewählt ist aus Wasserstoff, C₁-C₁₂-Alkyl, Amino, Mono- alkylamino, Dialkylamino, Arylalkyl, Heteroaryl, Heteroarylalkyl, Cycloalkyl, Cycloalkylalkyl, Heterocycloalkyl oder Heterocyclo- alkylalkyl.

44. Verbindung nach Anspruch 43, wobei der Tumor ausgewählt ist aus der Gruppe bestehend aus einem Sarkom, einem Melanom, einem Neuroblastom, einem Karzinom und einem Mesotheliom.

## Revendications

1. Composé de formule: 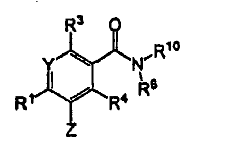 ou un sel, ester ou amide pharmaceutiquement acceptable de celui-ci, dans lequel
Y est C-R² ou N;
R¹ est choisi parmi l'hydrogène, alkyle en C₁-C₁₂ ou halogéno;
R², R³ et R⁴ sont indépendamment choisis parmi l'hydrogène ou halogéno;
R⁶ est l'hydrogène, ou
R⁶ est un alkyle en C₁-C₁₂ éventuellement substitué par un ou deux groupes choisis parmi amino, monoalkylamino ou dialkylamino;
R¹⁰ est choisi parmi aryle, hétéroaryle, hétéroarylalkyle, cycloalkyle, hétérocycloalkyle ou hétérocycloalkylalkyle, dont chacun est éventuellement substitué sur la portion cyclique et/ou la portion alkyle par un à cinq groupes choisis parmi alkyle en C₁-C₁₂, alcényle en C₂-C₁₂, alcynyle en C₂-C₁₂, trifluorométhyle, alcoxy en C₁-C₁₂, hydroxyle, halogéno, amino, monoalkylamino, dialkylamino, aminoalkyle, monoalkylaminoalkyle, dialkylaminoalkyle, nitro, -CN, oxo, -C(O)OR, -NH-C(O)-R', -C(O)-NHR', -SR, -SO₂R, -CO₂R, ou aryle, hétéroaryle, cycloalkyle ou hétérocycloalkyle, dont chacun est éventuellement substitué par un à trois groupes choisis parmi alkyle en C₁-C₁₂, alcényle en C₂-C₁₂, alcynyle en C₂-C₁₂, trifluorométhyle, alcoxy en C₁-C₁₂, hydroxyle, halogéno, amino, monoalkylamino, dialkylamino, aminoalkyle, monoalkylaminoalkyle, dialkylaminoalkyle, nitro, -CN, oxo, -C(O)OR, -NH-C(O)-R', -C(O)-NHR', -SR, -SO₂R, -CO₂R, ou
R⁶ et R¹⁰ conjointement avec l'atome d'azote auquel ils sont liés forment un cycle hétéroaryle à 5-7 chaînons éventuellement substitué par un à trois groupes indépendamment choisis parmi alkyle en C₁-C₁₂, alcényle en C₂-C₁₂, alcynyle en C₂-C₁₂, trifluorométhyle, alcoxy en C₁-C₁₂, hydroxyle, halogéno, amino, monoalkylamino, dialkylamino, aminoalkyle, monoalkylaminoalkyle, dialkylaminoalkyle, nitro, -CN, oxo, -C(O)OR, -NH-C(O)-R', -C(O)-NHR', -SR, -SO₂R, -CO₂R;
Z est choisi parmi 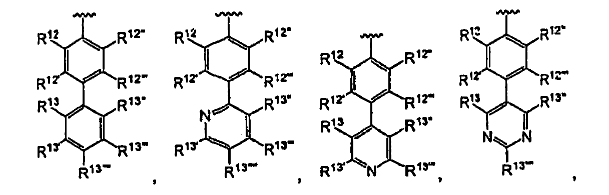 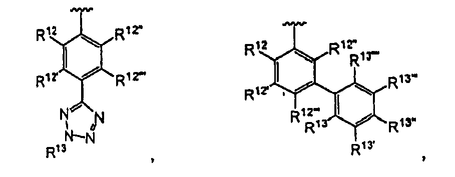 , ou 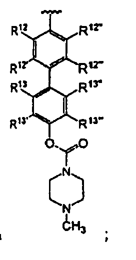
R¹², R^{12'}, R^{12"}, R^{12'''} sont indépendamment choisis parmi l'hydrogène, alkyle en C₁-C₁₂, alcényle en C₂-C₁₂, alcynyle en C₂-C₁₂, trifluorométhyle, alcoxy en C₁-C₁₂, hydroxyle, halogéno, amino, monoalkylamino, dialkylamino, aminoalkyle, monoalkylaminoalkyle, dialkylaminoalkyle, nitro, -CN, -C(O)OR, -NH-C(O)-R', -C(O)-NHR', -SR, -SO₂R, -CO₂R;
R¹³, R^{13'}, R^{13"}, R^{13'''} et R^{13''''} sont indépendamment choisis parmi l'hydrogène, alkyle en C₁-C₁₂, alcényle en C₂-C₁₂, alcynyle en C₂-C₁₂, trifluorométhyle, alcoxy en C₁-C₁₂, hydroxyle, halogéno, amino, monoalkylamino, dialkylamino, aminoalkyle, monoalkylaminoalkyle, dialkylaminoalkyle, nitro, -CN, -C(O)OR, -NH-C(O)-R', -C(O)-NHR', -SR, -SO₂R, -CO₂R;
X est halogéno;
R est choisi parmi l'hydrogène et alkyle en C₁-C₁₂; et
R' est indépendamment choisi parmi l'hydrogène, alkyle en C₁-C₁₂, amino, monoalkylamino, dialkylamino, arylalkyle, hétéroaryle, hétéroarylalkyle, cycloalkyle, cycloalkylalkyle, hétérocycloalkyle ou hétérocycloalkylalkyle.

2. Composé selon la revendication 1, ayant la formule:

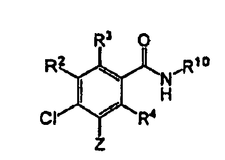

3. Composé selon la revendication 2, dans lequel R², R³ et R⁴ sont l'hydrogène.

4. Composé selon la revendication 1, ayant la formule:

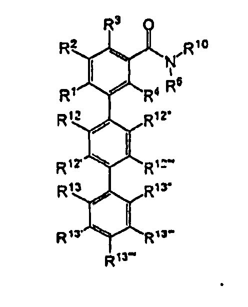

5. Composé selon la revendication 4, dans lequel
R¹ est hydrogène, chloro ou méthyle, et
R², R³ et R⁴ sont l'hydrogène.

6. Composé selon la revendication 4, dans lequel
R^{12'} est hydrogène ou halogéno, et
R¹², R^{12'} et R^{12''} sont l'hydrogène.

7. Composé selon la revendication 4, dans lequel
R¹³, R^{13'} et R^{13"} sont l'hydrogène.

8. Composé selon la revendication 1, ayant la formule:

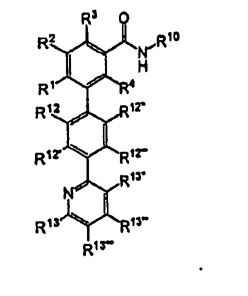

9. Composé selon la revendication 8, dans lequel R¹ est chloro, et R², R³ et R⁴ sont l'hydrogène.

10. Composé selon la revendication 8, dans lequel R¹², R^{12'}, R^{12"} et R^{12"'} sont l'hydrogène.

11. Composé selon la revendication 8, dans lequel R¹³, R^{13'}, R^{13"} et R^{13"'} sont l'hydrogène.

12. Composé selon la revendication 1, ayant la formule:

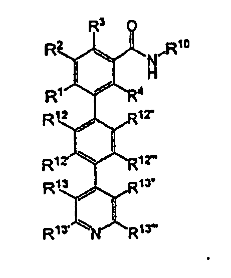

13. Composé selon la revendication 12, dans lequel R¹ est chloro, et R², R3 et R⁴ sont l'hydrogène.

14. Composé selon la revendication 12, dans lequel R¹², R^{12'}, R^{12"} et R^{12"'} sont l'hydrogène.

15. Composé selon la revendication 12, dans lequel R¹³, R^{13'}, R^{13"} et R^{13"'} sont l'hydrogène.

16. Composé selon la revendication 1, ayant la formule: -

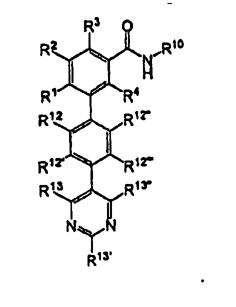

17. Composé selon la revendication 16, dans lequel R¹ est chloro, et R², R³ et R⁴ sont l'hydrogène.

18. Composé selon la revendication 16, dans lequel R¹², R^{12'}, R^{12"} et R^{12"'} sont l'hydrogène.

19. Composé selon la revendication 16, dans lequel R¹³, R^{13'}, et R^{13"} sont l'hydrogène.

20. Composé selon la revendication 1, ayant la formule:

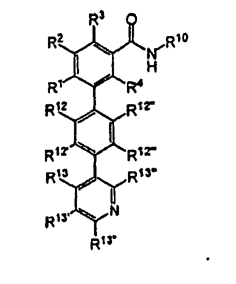

21. Composé selon la revendication 20, dans lequel R¹ est chloro, et R², R³ et R⁴ sont l'hydrogène.

22. Composé selon la revendication 20, dans lequel R¹², R^{12'}, R^{12"} et R^{12"'} sont l'hydrogène.

23. Composé selon la revendication 20, dans lequel R¹³, R^{13"} et R^{13"'} sont l'hydrogène, et R¹³' est choisi parmi l'hydrogène, aminoalkyle, monoalkylaminoalkyle, dialkylaminoalkyle ou alcoxy en C₁-C₁₂.

24. Composé selon la revendication 1, ayant la formule: 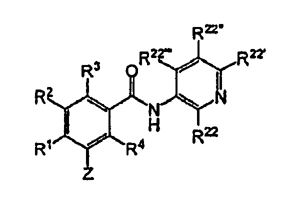 où
R²², R^{22'}, R^{22"} et R^{22"'} sont indépendamment choisis parmi l'hydrogène, alkyle en C₁-C₁₂, alcényle en C₂-C₁₂, alcynyle en C₂-C₁₂, trifluorométhyle, alcoxy en C₁-C₁₂, hydroxyle, halogéno, amino, monoalkylamino, dialkylamino, aminoalkyle, monoalkylaminoalkyle, dialkylaminoalkyle, nitro, -CN, oxo, -C(O)OR, -NH-C(O)-R', -C(O)-NHR', -SR, -SO₂R, -CO₂R; ou aryle, hétéroaryle, cycloalkyle ou hétérocycloalkyle, dont chacun est éventuellement substitué par un à trois groupes choisis parmi alkyle en C₁-C₁₂, alcényle en C₂-C₁₂, alcynyle en C₂-C₁₂, trifluorométhyle, alcoxy en C₁-C₁₂, hydroxyle, halogéno, amino, monoalkylamino, dialkylamino, aminoalkyle, monoalkylaminoalkyle, dialkylaminoalkyle, nitro, -CN, oxo, -C(O)OR, -NH-C(O)-R', -C(O)-NHR', -SR, -SO₂R, -CO₂R.

25. Composé selon la revendication 24, dans lequel R¹ est chloro, et R², R³ et R⁴ sont l'hydrogène.

26. Composé selon la revendication 24, dans lequel R²², R^{22'}, R^{22"} et R^{22'"} sont choisis parmi l'hydrogène, halogéno, alkyle en C₁-C₁₂, alcoxy en C₁-C₁₂ ou hétérocyclyle éventuellement substitué.

27. Composé selon la revendication 1, ayant la formule: 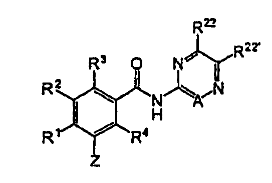 où
A est -CH ou N; et
R²² et R^{22'} sont indépendamment choisis parmi l'hydrogène, alkyle en C₁-C₁₂, alcényle en C₂-C₁₂, alcynyle en C₂-C₁₂, trifluorométhyle, alcoxy en C₁-C₁₂, hydroxyle, halogéno, amino, monoalkylamino, dialkylamino, aminoalkyle, monoalkylaminoalkyle, dialkylaminoalkyle, nitro, -CN, oxo, -C(O)OR, -NH-C(O)-R', -C(O)-NHR', -SR, -SO₂R, -CO₂R; ou aryle, hétéroaryle, cycloalkyle ou hétérocycloalkyle, dont chacun est éventuellement substitué par un à trois groupes choisis parmi alkyle en C₁-C₁₂, alcényle en C₂-C₁₂, alcynyle en C₂-C₁₂, trifluorométhyle, alcoxy en C₁-C₁₂, hydroxyle, halogéno, amino, monoalkylamino, dialkylamino, aminoalkyle, monoalkylaminoalkyle, dialkylaminoalkyle, nitro, -CN, oxo, -C(O)OR, -NH-C(O)-R', -C(O)-NHR', -SR, -SO₂R, -CO₂R.

28. Composé selon la revendication 27, dans lequel R¹ est chloro, et R², R³ et R⁴ sont l'hydrogène.

29. Composé selon la revendication 27, dans lequel R²² et R^{22'} sont choisis parmi l'hydrogène, halogéno, alkyle en C₁-C₁₂ et alcoxy en C₁-C₁₂.

30. Composé selon la revendication 1, ayant la formule: 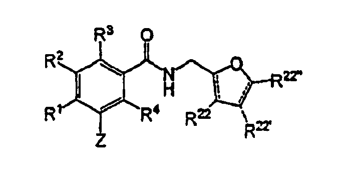 où les liaisons - - - - sont toutes présentes ou toutes absentes, et R¹, R², R³, R⁴, et Z sont tels que définis dans la revendication 1,
et
R²², R²²' et R^{22"} sont indépendamment choisis parmi l'hydrogène, alkyle en C₁-C₁₂, alcényle en C₂-C₁₂, alcynyle en C₂-C₁₂, trifluorométhyle, alcoxy en C₁-C₁₂, hydroxyle, halogéno, amino, monoalkylamino, dialkylamino, aminoalkyle, monoalkylaminoalkyle, dialkylaminoalkyle, nitro, -CN, oxo, -C(O)OR, -NH-C(O)-R', -C(O)-NHR', -SR, -SO₂R, -CO₂R; ou aryle, hétéroaryle, cycloalkyle ou hétérocycloalkyle, dont chacun est éventuellement substitué par un à trois groupes choisis parmi alkyle en C₁-C₁₂, alcényle en C₂-C₁₂, alcynyle en C₂-C₁₂, trifluorométhyle, alcoxy en C₁-C₁₂, hydroxyle, halogéno, amino, monoalkylamino, dialkylamino, aminoalkyle, monoalkylaminoalkyle, dialkylaminoalkyle, nitro, -CN, oxo, -C(O)OR, -NH-C(O)-R', -C(O)-NHR', -SR, -SO₂R, -CO₂R.

31. Composé selon la revendication 30, dans lequel R¹ est chloro, et R², R³ et R⁴ sont l'hydrogène.

32. Composé selon la revendication 30, dans lequel R²² et R²²' sont choisis parmi l'hydrogène, halogéno, alkyle en C₁-C₁₂ et alcoxy en C₁-C₁₂.

33. Composé selon la revendication 1, ayant la formule: 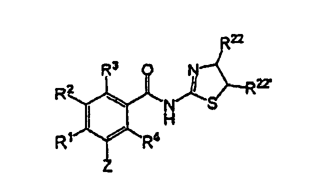 où
A est -CH ou N; et
R²² et R^{22'} sont indépendamment choisis parmi l'hydrogène, alkyle en C₁-C₁₂, alcényle en C₂-C₁₂, alcynyle en C₂-C₁₂, trifluorométhyle, alcoxy en C₁-C₁₂, hydroxyle, halogéno, amino, monoalkylamino, dialkylamino, aminoalkyle, monoalkylaminoalkyle, dialkylaminoalkyle, nitro, -CN, oxo, -C(O)OR, -NH-C(O)-R', -C(O)-NHR', -SR, -SO₂R, -CO₂R, ou aryle, hétéroaryle, cycloalkyle ou hétérocycloalkyle, dont chacun est éventuellement substitué par un à trois groupes choisis parmi alkyle en C₁-C₁₂, alcényle en C₂-C₁₂, alcynyle en C₂-C₁₂, trifluorométhyle, alcoxy en C₁-C₁₂, hydroxyle, halogéno, amino, monoalkylamino, dialkylamino, aminoalkyle, monoalkylaminoalkyle, dialkylaminoalkyle, nitro, -CN, oxo, -C(O)OR, -NH-C(O)-R', -C(O)-NHR', -SR, -SO₂R, -CO₂R.

34. Composé selon la revendication 33, dans lequel R¹ est chloro, et R², R³ et R⁴ sont l'hydrogène.

35. Composé selon la revendication 33, dans lequel R²² et R^{22'} sont choisis parmi l'hydrogène, halogéno, alkyle en C₁-C₁₂ et alcoxy en C₁-C₁₂.

36. Composé selon la revendication 1, ayant la formule: 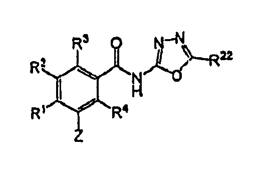 où
A est -CH ou N; et
R²² est indépendamment choisi parmi l'hydrogène, alkyle en C₁-C₁₂, alcényle en C₂-C₁₂, alcynyle en C₂-C₁₂, trifluorométhyle, alcoxy en C₁-C₁₂, hydroxyle, halogéno, amino, monoalkylamino, dialkylamino, aminoalkyle, monoalkylaminoalkyle, dialkylaminoalkyle, nitro, -CN, oxo, -C(O)OR, -NH-C(O)-R', -C(O)-NHR', -SR, -SO₂R, -CO₂R, ou aryle, hétéroaryle, cycloalkyle ou hétérocycloalkyle, dont chacun est éventuellement substitué par un à trois groupes choisis parmi alkyle en C₁-C₁₂, alcényle en C₂-C₁₂, alcynyle en C₂-C₁₂, trifluorométhyle, alcoxy en C₁-C₁₂, hydroxyle, halogéno, amino, monoalkylamino, dialkylamino, aminoalkyle, monoalkylaminoalkyle, dialkylaminoalkyle, nitro, -CN, oxo, -C(O)OR, -NH-C(O)-R', -C(O)-NHR', -SR, -SO₂R, -CO₂R.

37. Composé selon la revendication 36, dans lequel R¹ est chloro, et R², R³ et R⁴ sont l'hydrogène.

38. Composé selon la revendication 36, dans lequel R²² et R^{22'} sont choisis parmi l'hydrogène, halogéno, alkyle en C₁-C₁₂, alcoxy en C₁-C₁₂ et aryle éventuellement substitué.

39. Composé selon la revendication 1, choisi parmi:
6-chloro-*N-*(tétrahydrofuranne-2-ylméthyl)-1,1':4',1"-terphényl-3-carboxamide;
6-chloro-*N-*(2-furylméthyl)-1,1':4',1"-terphényl-3-carboxamide;
6-chloro-*N-*(6-chloropyridine-3-yl)-1,1':4',1"-terphényl-3-carboxamide;
6-chloro-*N-*(4,5-dihydro-1,3-thiazole-2-yl)-1,1':4',1 "-terphényl-3-carboxamide;
6-chloro-*N-*(2-pyrrolidine-1-yléthyl)-1,1':4',1"-terphényl-3-carboxamide;
6-chloro-*N-*(5-chloropyrimidine-2-yl)-1,1':4',1"-terphényl-3-carboxamide;
6-chloro-*N-*(3-méthylisoxazole-5-yl)-1,1':4',1"-terphényl-3-carboxamide;
6-chloro-*N-*[6-(méthylsulfonyl)-1,3-benzothiazole-2-yl]-1,1':4',1"-terphényl-3-carboxamide;
6-chloro-*N-*1,2,4-triazine-3-yl-1,1':4',1"-terphényl-3-carboxamide;
3-1[(6-Chloro-1,1':4',l"-terphényl-3-yl)carbonyl]aminolbenzoate de méthyle;
6-chloro-*N-*(5-phényl-1,3,4-oxadiazole-2-yl)-1,1':4',1"-terphényl-3-carboxamide;
6-chloro-*N-*[3-(2-méthyl-1,3-dioxolanne-2-yl)phényl]-1,1':4',1 "-terphényl-3-carboxamide;
6-chloro-*N-*(4-méthoxyphényl)-1,1':4',1"-terphényl-3-carboxamide;
6-chloro-*N-*quinoline-6-yl-1,1':4',1"-terphényl-3-carboxamide;
6-chloro-*N-*pyrazine-2-yl-1,1':4',1"-terphényl-3-carboxamide;
*N-*(5-bromopyridine-2-yl)-6-chloro-1,1':4',1"-terphényl-3-carboxamide;
6-chloro-*N-*(6-méthoxypyridine-3-yl)-1,1':4',1"-terphényl-3-carboxamide;
6-chloro-*N-*(6-fluoropyridine-3-yl)-1,1':4',1 "-terphényl-3-carboxamide;
6-chloro-*N-*(5-chloropyridine-2-yl)-1,1':4',1"-terphényl-3-carboxamide;
6-chloro-*N-*(6-méthoxypyridine-3-yl)-4'-pyridine-2-ylbiphényl-3-carboxamide;
6-chloro-3'-fluoro-*N-*(6-méthoxypyridine-3-yl)-1,1':4',1 "-terphényl-3-carboxamide;
6-chloro-*N-*(6-méthoxypyridine-3-yl)-4'-pyridine-4-ylbiphényl-3-carboxamide;
6-chloro-*N-*(4-méthylphényl)-1,1':4',1"-terphényl-3-carboxamide;
6-chloro-*N-*(3-fluoro-4-méthoxyphényl)-1,1':4',1"-terphényl-3-carboxamide;
6-chloro-*N-*(6-méthoxypyridine-3-yl)-4'-pyrimidine-5-ylbiphényl-3-carboxamide;
6-chloro-*N-*[2-(diméthylamino)éthyl]-*N-*pyridine-3-yl-1,1':4',1"-terphényl-3-carboxamide;
*N-*(2-pyrrolidine-1-yléthyl)-1,1':4',1"-terphényl-3-carboxamide;
6-chloro-*N-*[6-(4-méthylpipérazine-1-yl)pyridine-3yl]-1,1':4',1"-terphényl-3-carboxamide;
6-chloro-*N-*(6-morpholine-4-ylpyridine-3-yl)-1,1':4',1"-terphényl-3-carboxamide;
4-chloro-*N-*(6-méthoxypyridine-3-yl)-3-(6-phénylpyridine-3-yl)benzamide;
6-chloro-*N-*[6-(méthylthio)pyridine-3-yl]-1,1':4',1"-terphényl-3-carboxamide;
6-chloro-*N-*(5-méthoxypyrazine-2-yl)-1,1':4',1"-terphényl-3-carboxamide;
6-chloro-*N-*[2-(méthylthio)pyrimidine-5-yl]-1,1':4',1"-terphényl-3-carboxamide;
6-chloro-*N*-(2-méthoxypyrimidine-5-yl)-1,1':4',1"-terphényl-3-carboxamide;
6-chloro-*N-*(6-méthylpyridine-3-yl)-1,1':4',1"-terphényl-3-carboxamide;
6-chloro-*N-*(6-oxo-1,6-dihydropyridine-3-yl)-4'-pyridine-2-ylbiphényl-3-carboxamide;
6-chloro-*N-*(6-méthoxypyridine-3-yl)-4'-(2-méthyl-2*H-*tétrazole-5-yl)biphényl-3-carboxamide;
6-chloro-*N-*(6-méthoxypyridine-3-yl)-1,1':3',1"-terphényl-3-carboxamide;
*N-*(6-méthoxypyridine-3-yl)-6-méthyl-1,1':4',1"-terphényl-3-carboxamide;
5-biphényl-4-yl-6-chloro-*N-*(6-méthoxypyridine-3-yl)nicotinamide;
6-chloro-*N-*(6-méthoxypyridine-3-yl)-4'-pyridine-2-ylbiphényl-3-carboxamide;
6-chloro-3"-formyl-*N-*(6-méthoxypyridine-3-yl)-1,1':4',1"-terphényl-3-carboxamide;
6-chloro-3"-[(diméthylamino)méthyl]-*N-*(6-méthoxypyridine-3-yl)-1,1':4',1"-terphényl-3-carboxamide;
6-chloro-3"-(hydroxyméthyl)-*N-*(6-méthoxypyridine-3-yl)-1,1':4',1 "-terphényl-3-carboxamide;
6-chloro-4"-[(diméthylamino)méthyl]-*N-*(6-méthoxypyridine-3-yl)-1,1':4',1 "-terphényl-3-carboxamide;
6-chloro-4"-(hydroxyméthyl)-*N-*(6-méthoxypyridine-3-yl)-1,1':4',1 "-terphényl-3-carboxamide;
6-chloro-*N-*(6-méthoxypyridine-3-yl)-3"-(méthylsulfonyl)-1,1':4',1"-terphényl-3-carboxamide;
6-chloro-N-(6-méthoxypyridine-3-yl)-4'-pyridine-3-ylbiphényl-3-carboxamide;
4"-(aminométhyl)-6-chloro-*N-*(6-méthoxypyridine-3-yl)-1,1':4',1"-terphényl-3-carboxamide;
6-chloro-4'-[5-(hydroxyméthyl)pyridine-3-yl]-*N-*(6-méthoxypyridine-3-yl)biphényl-3-carboxamide;
6-chloro-N³-(6-méthoxypyridine-3-yl)-1,1':4',1"-terphényl-3,3"-dicarboxamide;
6-chloro-3"-hydroxy-*N-*(6-méthoxypyridine-3-yl)-1,1':4',1 "-terphényl-3-carboxamide;
6-chloro-4"-hydroxy-*N-*(6-méthoxypyridine-3-yl)-1,1':4',1"-terphényl-3-carboxamide;
6-chloro-4'-{5-[(diméthylamino)méthyl]pyridine-3-yl}-*N-*(6-méthoxypyridine-3-yl)biphényl-3-carboxamide;
5-fluoro-*N-*(6-méthoxypyridine-3-yl)-1,1':4',1"-terphényl-3-carboxamide;
6-chloro-*N-*{3-[(morpholine-4-ylacétyl)amino]phényl})-1,1':4',1"-terphényl-3-carboxamide;
6-chloro-*N-*{4-[(morpholine-4-ylacétyl)amino]phényl})-1,1':4',1"-terphényl-3-carboxamide;
6-chloro-*N-*{6-[(4-méthylpipérazine-1-yl)méthyl]pyridine-3-yl}-1,1':4',1"-terphényl-3-carboxamide;
6-chloro-*N-*[6-(hydroxyméthyl)pyridine-3-yl]-1,1':4',1"-terphényl-3-carboxamide;
4-Méthylpipérazine-1-carboxylate de 2"-chloro-5"-{[(6-méthoxy-pyridine-3-yl)amino]carbonyl}-1,1':4',1"-terphényl-4-yle;
acide 2-{[(6-chloro-1,1':4',1"-terphényl-3-yl)carbonyl]amino}indane-2-carboxylique;
acide (4S)-3-[(6-chloro-1,1':4',1"-terphényl-3-yl)carbonyl]-1,3-thiazolidine-4-carboxylique;
*N-*[(6-chloro-1,1':4',1"-terphényl-3-yl)carbonyl]-D-méthionine; et
*N-*[(6-chloro-1,1':4',1"-terphényl-3-yl)carbonyl]-3-(7,7a-dihydro-1*H-*indole-3-yl)-D-alanine.

40. Composition pharmaceutique comprenant un composé selon l'une quelconque des revendications 1-39 et un support pharmaceutiquement acceptable.

41. Utilisation d'un composé de formule: 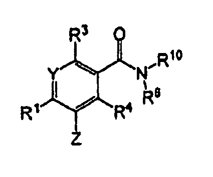 ou d'un sel, ester ou amide pharmaceutiquement acceptable de celui-ci,
où
Y est C-R² ou N;
R¹ est choisi parmi l'hydrogène, alkyle en C₁-C₁₂ ou halogéno;
R², R³ et R⁴ sont indépendamment choisis parmi l'hydrogène ou halogéno;
R⁶ est l'hydrogène, ou
R⁶ est un alkyle en C₁-C₁₂ éventuellement substitué par un ou deux groupes choisis parmi amino, monoalkylamino ou dialkylamino;
R¹⁰ est choisi parmi aryle, arylalkyle, hétéroaryle, hétéroarylalkyle, cycloalkyle, cycloalkylalkyle, hétérocycloalkyle ou hétérocycloalkylalkyle, dont chacun est éventuellement substitué sur la portion cyclique et/ ou la portion alkyle par un à cinq groupes choisis parmi alkyle en C₁-C₁₂, alcényle en C₂- C₁₂, alcynyle en C₂-C₁₂, trifluorométhyle, alcoxy en C₁-C₁₂, hydroxyle, halogéno, amino, monoalkylamino, dialkylamino, aminoalkyle, monoalkylaminoalkyle, dialkylaminoalkyle, nitro, -CN, oxo, -C(O)OR, -NH-C(O)-R', -C(O)-NHR', -SR, -SO₂R, - CO₂R, ou aryle, hétéroaryle, cycloalkyle ou hétérocycloalkyle, dont chacun est éventuellement substitué par un à trois groupes choisis parmi alkyle en C₁-C₁₂, alcényle en C₂-C₁₂, alcynyle en C₂-C₁₂, trifluorométhyle, alcoxy en C₁-C₁₂, hydroxyle, halogéno, amino, monoalkylamino, dialkylamino, aminoalkyle, monoalkylaminoalkyle, dialkylaminoalkyle, nitro, -CN, oxo, -C(O)OR, -NH- C(O)-R', -C(O)-NHR', -SR, -SO₂R, -CO₂R, ou
R⁶ et R¹⁰ conjointement avec l'atome d'azote auquel ils sont liés formes un cycle hétéroarylique ou hétérocyclique à 5-7 chaînons éventuellement substitué par un à trois groupes indépendamment choisis parmi alkyle en C₁-C₁₂, alcényle en C₂-C₁₂, alcynyle en C₂-C₁₂, trifluorométhyle, alcoxy en C₁- C₁₂, hydroxyle, halogéno, amino, monoalkylamino, dialkylamino, aminoalkyle, monoalkylaminoalkyle, dialkylaminoalkyle, nitro, -CN, oxo, -C(O)OR, -NH-C(O)-R', - C(O)-NHR', -SR, -SO₂R, -CO₂R;
Z est choisi parmi 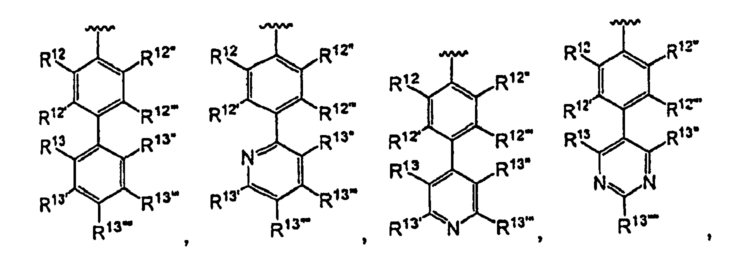 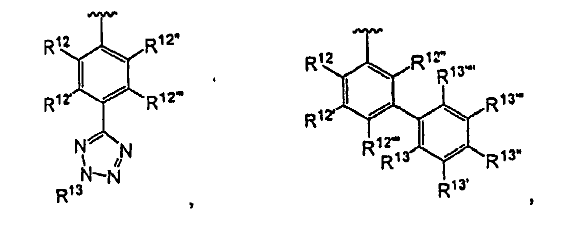 , ou 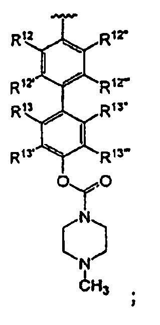
R¹², R^{12'}, R^{12"}, R^{12"'} sont indépendamment choisis parmi hydrogène, alkyle en C₁-C₁₂, alcényle en C₂-C₁₂, alcynyle en C₂-C₁₂, trifluorométhyle, alcoxy en C₁-C₁₂, hydroxyle, halogéno, amino, monoalkylamino, dialkylamino, aminoalkyle, monoalkylaminoalkyle, dialkylaminoalkyle, nitro, -CN, -C(O)OR, -NH-C(O)-R', -C(O)-NHR', -SR, -SO₂R, -CO₂R;
R¹³, R^{13'}, R^{13"}, R^{13"'} et R^{13""} sont indépendamment choisis parmi hydrogène, alkyle en C₁-C₁₂, alcényle en C₂-C₁₂, alcynyle en C₂-C₁₂, trifluorométhyle, alcoxy en C₁-C₁₂, hydroxyle, halogéno, amino, monoalkylamino, dialkylamino, aminoalkyle, monoalkylaminoalkyle, dialkylaminoalkyle, nitro, -CN, -C(O)OR, -NH-C(O)-R', -C(O)-NHR', -SR, -SO₂R, -CO₂R;
X est halogéno;
R est choisi parmi l'hydrogène et alkyle en C₁-C₁₂; et
R' est indépendamment choisi parmi l'hydrogène, alkyle en C₁-C₁₂, amino, monoalkylamino, dialkylamino, arylalkyle, hétéroaryle, hétéroarylalkyle, cycloalkyle, cycloalkylalkyle, hétérocycloalkyle ou hétérocycloalkylalkyle;
pour la préparation d'une composition pharmaceutique pour le traitement d'une tumeur.

42. Utilisation selon la revendication 41, dans laquelle la tumeur est choisie dans le groupe consistant en un sarcome, un mélanome, un neuroblastome, un carcinome et un mésothéliome.

43. Composé de formule: 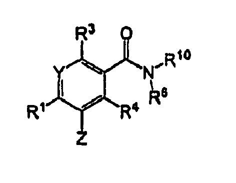 ou un sel, ester ou amide pharmaceutiquement acceptable de celui-ci, où
Y est C-R² ou N;
R¹ est choisi parmi l'hydrogène, alkyle en C₁-C₁₂ ou halogéno;
R², R³ et R⁴ sont indépendamment choisis parmi l'hydrogène ou halogéno;
R⁶ est l'hydrogène, ou
R⁶ est un alkyle en C₁-C₁₂ éventuellement substitué par un ou deux groupes choisis parmi amino, monoalkylamino ou dialkylamino;
R¹⁰ est choisi parmi aryle, arylalkyle, hétéroaryle, hétéroarylalkyle, cycloalkyle, cycloalkylalkyle, hétérocycloalkyle ou hétérocycloalkylalkyle, dont chacun est éventuellement substitué sur la portion cyclique et/ ou la portion alkyle par un à cinq groupes choisis parmi alkyle en C₁-C₁₂, alcényle en C₂- C₁₂, alcynyle en C₂-C₁₂, trifluorométhyle, alcoxy en C₁-C₁₂, hydroxyle, halogéno, amino, monoalkylamino, dialkylamino, aminoalkyle, monoalkylaminoalkyle, dialkylaminoalkyle, nitro, -CN, oxo, -C(O)OR, -NH-C(O)-R', -C(O)-NHR', -SR, -SO₂R, - CO₂R, ou aryle, hétéroaryle, cycloalkyle ou hétérocycloalkyle, dont chacun est éventuellement substitué par un à trois groupes choisis parmi alkyle en C₁-C₁₂, alcényle en C₂-C₁₂, alcynyle en C₂-C₁₂, trifluorométhyle, alcoxy en C₁-C₁₂, hydroxyle, halogéno, amino, monoalkylamino, dialkylamino, aminoalkyle, monoalkylaminoalkyle, dialkylaminoalkyle, nitro, -CN, oxo, -C(O)OR, -NH- C(O)-R', -C(O)-NHR', -SR, -SO₂R, -CO₂R, ou
R⁶ et R¹⁰ conjointement avec l'atome d'azote auquel ils sont liés formes un cycle hétéroarylique ou hétérocyclique à 5-7 chaînons éventuellement substitué par un à trois groupes indépendamment choisis parmi alkyle en C₁-C₁₂, alcényle en C₂-C₁₂, alcynyle en C₂-C₁₂, trifluorométhyle, alcoxy en C₁- C₁₂, hydroxyle, halogéno, amino, monoalkylamino, dialkylamino, aminoalkyle, monoalkylaminoalkyle, dialkylaminoalkyle, nitro, -CN, oxo, -C(O)OR, -NH-C(O)-R', - C(O)-NHR', -SR, -SO₂R, -CO₂R;
Z est choisi parmi 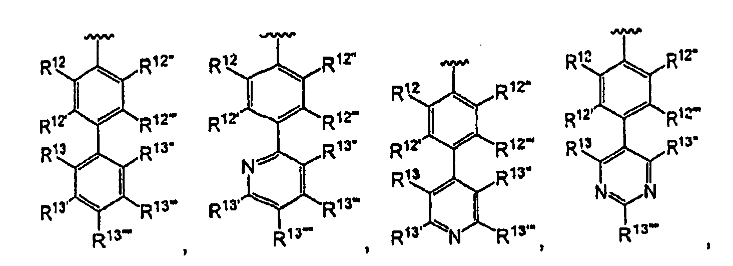 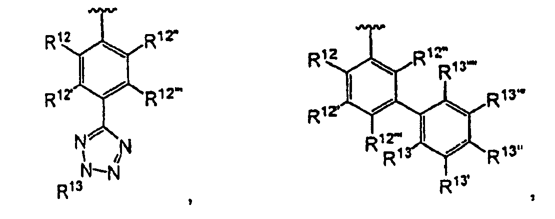 ou 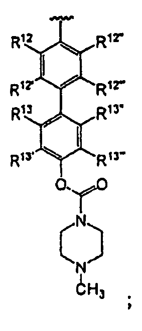
R¹², R^{12'}, R^{12"}, R12"' sont indépendamment choisis parmi l'hydrogène, alkyle en C₁-C₁₂, alcényle en C₂-C₁₂, alcynyle en C₂-C₁₂, trifluorométhyle, alcoxy en C₁-C₁₂, hydroxyle, halogéno, amino, monoalkylamino, dialkylamino, aminoalkyle, monoalkylaminoalkyle, dialkylaminoalkyle, nitro, -CN, -C(O)OR, -NH-C(O)-R', -C(O)-NHR', -SR, -SO₂R, -CO₂R;
R¹³, R^{13'}, R^{13"}, R^{13"'} et R^{13""} sont indépendamment choisis parmi l'hydrogène, alkyle en C₁-C₁₂, alcényle en C₂-C₁₂, alcynyle en C₂-C₁₂, trifluorométhyle, alcoxy en C₁-C₁₂, hydroxyle, halogéno, amino, monoalkylamino, dialkylamino, aminoalkyle, monoalkylaminoalkyle, dialkylaminoalkyle, nitro, -CN, -C(O)OR, -NH-C(O)-R', -C(O)-NHR', -SR, -SO₂R, -CO₂R;
X est halogéno;
R est choisi parmi l'hydrogène et alkyle en C₁-C₁₂; et
R' est indépendamment choisi parmi l'hydrogène, alkyle en C₁-C₁₂, amino, monoalkylamino, dialkylamino, arylalkyle, hétéroaryle, hétéroarylalkyle, cycloalkyle, cycloalkylalkyle, hétérocycloalkyle ou hétérocycloalkylalkyle;
pour utilisation dans le traitement d'une tumeur.

44. Composé selon la revendication 43, dans lequel la tumeur est choisie dans le groupe consistant en un sarcome, un mélanome, un neuroblastome, un carcinome et un mésothéliome.
